(19) Europäisches Patentamt / European Patent Office / Office européen des brevets

(11) EP 4 748 834 A1

(12) EUROPEAN PATENT APPLICATION
published in accordance with Art. 153(4) EPC

(43) Date of publication:
**27.05.2026 Bulletin 2026/22**

(21) Application number: **24843178.5**

(22) Date of filing: **18.07.2024**

(51) International Patent Classification (IPC):
*C07D 231/12* (2006.01) *A01N 43/48* (2006.01)
*A01N 43/50* (2006.01) *A01N 43/653* (2006.01)
*A01P 5/00* (2006.01) *A01P 7/00* (2006.01)
*C07D 233/64* (2006.01) *C07D 249/08* (2006.01)

(52) Cooperative Patent Classification (CPC):
**A01N 43/48; A01N 43/50; A01N 43/653; A01P 5/00; A01P 7/00; C07D 231/12; C07D 233/64; C07D 249/08**

(86) International application number:
**PCT/JP2024/025816**

(87) International publication number:
**WO 2025/018390 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.07.2023 JP 2023116792**

(71) Applicant: **Agro-Kanesho Co., Ltd.**
**Tokyo 100-0005 (JP)**

(72) Inventors:
- **USUI, Shuichi**
**Tokorozawa-shi, Saitama 359-0024 (JP)**
- **ARAKI, Koichi**
**Tokorozawa-shi, Saitama 359-0024 (JP)**
- **HANAGATA, Satoshi**
**Tokorozawa-shi, Saitama 359-0024 (JP)**
- **NAKAJIMA, Asuka**
**Tokorozawa-shi, Saitama 359-0024 (JP)**
- **MIYAMA, Atsushi**
**Yuki-shi, Ibaraki 307-0001 (JP)**

(74) Representative: **Gulde & Partner**
**Patent- und Rechtsanwaltskanzlei mbB**
**Berliner Freiheit 2**
**10785 Berlin (DE)**

# (54) NOVEL HYDRAZONE DERIVATIVE AND AGENT FOR AGRICULTURAL AND HORTICULTURAL USE CONTAINING SAME AS ACTIVE INGREDIENT

(57) The present invention provides a compound which exhibits an excellent effect as an agricultural and horticultural chemical agent, especially as an agricultural and horticultural bactericide. The present invention provides, as an active ingredient, a hydrazone derivative represented by formula (1) or a salt thereof. In the formula, $R_1$ represents a hydrogen atom, a $C_{1-6}$ alkyl group, or the like; $R_2$ represents a formyl group, or may form a group represented by formula (2) together with $R_1$; and $R_5$ represents $OR_6$ or $NR_7R_8$, wherein $R_6$ represents a $C_{1-6}$ alkyl group, $R_7$ represents a hydrogen atom or a $C_{1-6}$ alkyl group, and $R_8$ represents a $C_{1-6}$ alkyl group, or $R_7$ and $R_8$ may bond to each other and, together with the nitrogen atom to which $R_7$ and $R_8$ are bonded, form a saturated 4- to 7-membered ring.

EP 4 748 834 A1

## Description

### Technical Field

The present invention relates to a novel hydrazone derivative and an agricultural and horticultural chemical agent having the compound as an active ingredient, particularly an agricultural and horticultural pest control agent or a nematode control agent, and a method for using the same.

### Background Art

In the field of agriculture and horticulture, plant pest and disease control agents have been developed and put into practical use for the purpose of controlling various pests and diseases. However, the emergence of pests or diseases that have acquired drug resistance has become a problem, and the development of novel agents is eagerly desired.

Patent Literature 1 describes that a semicarbazone derivative is useful as an insecticide for agricultural use. However, although Patent Literature 1 discloses a semicarbazone compound, it does not disclose a hydrazone compound like that of the present invention. Furthermore, regarding the compound described in Patent Literature 1, no effect against nematodes is described at all.

Patent Literatures 2 and 3 describe compounds similar to the compound of the present invention as agents for agricultural nematodes, but they do not describe a compound like that of the present invention in which an azole is bonded to the β-carbon of phenylhydrazone.

Patent Literatures 4 and 5 disclose hydrazone compounds similar to the compound of the present invention. However, these patent documents relate to a compound in which a phenyl group and the β-carbon of phenylhydrazone are bonded to form a ring structure in the phenylhydrazone skeleton, and do not disclose a compound that does not have such a ring structure.

Patent Literature 6 discloses, for example, a cyclic hydrazone-based compound having 2,3,4,5-tetrahydro-1,2,4-triazine, 3,6-dihydro-2H-1,3,4-oxadiazine, or the like. However, in a compound having these structures, the β-carbon of the phenylhydrazone forms a ring structure with the nitrogen atom of the hydrazone group, and a compound not forming such a ring is not disclosed.

### Citation List

#### Patent Literatures

Patent Literature 1: Japanese Unexamined Patent Application Publication No. Hei 08-073436
Patent Literature 2: International Publication No. WO 2020/179910
Patent Literature 3: International Publication No. WO 2023/282110
Patent Literature 4: International Publication No. WO 01/36381
Patent Literature 5: Japanese Unexamined Patent Application Publication No. 2002-155044
Patent Literature 6: International Publication No. WO 93/19045

### Summary of Invention

#### Problems to be solved by the invention

In crop production such as agriculture and horticulture, damage due to pests and diseases is still significant, and development of a novel agricultural and horticultural chemical agent is desired due to factors such as an emergence of pests and diseases resistant to existing drugs.

#### Means for solution of the problems

As a result of diligent research to solve the above problem, the present inventors found that a hydrazone derivative represented by the following formula (1), which is a novel compound not described in the literature, is useful as an agricultural and horticultural chemical agent, particularly as an agricultural and horticultural pest control agent or a nematode control agent, and have completed the present invention.

That is, the present invention relates to a hydrazone derivative represented by the following formula (1),

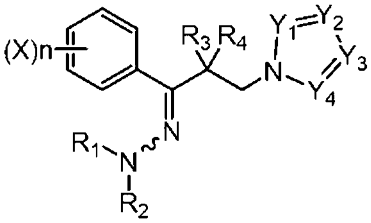

(1)

[wherein in the formula, $R_1$ represents a hydrogen atom, a $C_{1-6}$ alkyl group, a $C_{2-6}$ alkenyl group, a $C_{2-6}$ alkynyl group, or a benzyl group,

$R_2$ represents a formyl group, or

$R_1$ and $R_2$ may, together, form a group represented by the following formula,

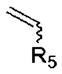

,

$R_5$ represents $OR_6$ or $NR_7R_8$,

$R_6$ represents a $C_{1-6}$ alkyl group,

$R_7$ represents a hydrogen atom or a $C_{1-6}$ alkyl group,

$R_8$ represents a $C_{1-6}$ alkyl group, or

$R_7$ and $R_8$ may bond to each other and, together with the nitrogen atom to which $R_7$ and $R_8$ are bonded, form a saturated 4- to 7-membered ring,

$R_3$ represents a $C_{1-6}$ alkyl group,

$R_4$ represents a hydrogen atom or a $C_{1-6}$ alkyl group, or

$R_3$ and $R_4$ may, together, form a ($C_3$-$C_6$)-carbocycle,

X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,

n represents an integer of 1 to 5,

$Y_1$, $Y_2$, $Y_3$, and $Y_4$ each independently represent a nitrogen atom or a CR group,

R represents a hydrogen atom, a halogen atom, a $C_{1-6}$ alkyl group, or a $C_{1-6}$ haloalkyl group, and when a plurality of Rs are present, they may be the same as or different from each other.]

or a salt thereof, and further relates to an agricultural and horticultural chemical agent having the compound as an active ingredient, particularly an agricultural and horticultural pest control agent or a nematode control agent, a method for using the same, and a method for producing the compound and the like.

Specifically, aspects of the present invention are as follows.

1. A hydrazone derivative represented by the following formula (1),

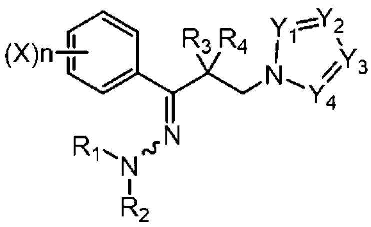

(1)

or a salt thereof,

wherein in the formula, $R_1$ represents a hydrogen atom, a $C_{1-6}$ alkyl group, a $C_{2-6}$ alkenyl group, a $C_{2-6}$ alkynyl group, or a benzyl group,

$R_2$ represents a formyl group, or

$R_1$ and $R_2$ may, together, form a group represented by the following formula,

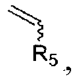

$R_5$ represents $OR_6$ or $NR_7R_8$,
$R_6$ represents a $C_{1-6}$ alkyl group,
$R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other.

2. The hydrazone derivative or a salt thereof according to 1 above, wherein $Y_2$ represents CR, and R represents a $C_{1-6}$ haloalkyl group.
3. A hydrazone derivative represented by the following formula (2).

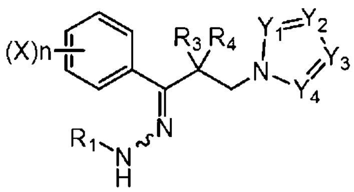
(2)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other.

4. A ketone derivative represented by the following formula (3).

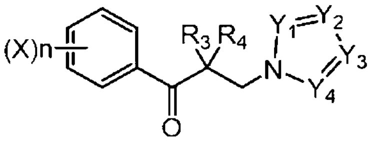
(3)

wherein in the formula, $R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other

same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other.

5. A method for producing a hydrazone derivative represented by the following formula (4),

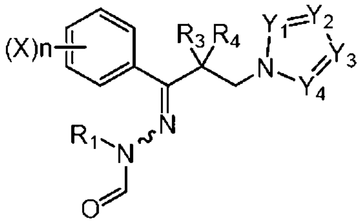
(4)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (2),

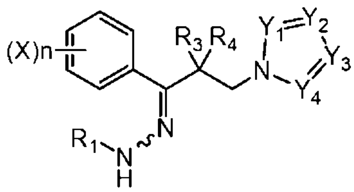
(2)

wherein in the formula, $R_1$, $R_3$, $R_4$, X, $Y_1$, $Y_2$, $Y_3$, $Y_4$, and n are as defined in the above formula (4),
with formic acid and/or a formate ester represented by the following formula (5), $HCOOR_9$ (5)
wherein in the formula, $R_9$ represents a $C_{1-6}$ alkyl group.

6. A method for producing a hydrazone derivative represented by the following formula (6),

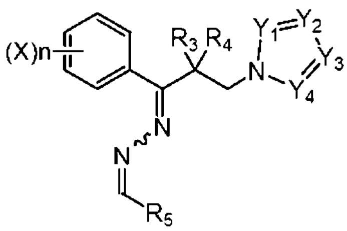
(6)

wherein in the formula, $R_5$ represents $OR_6$ or $NR_7R_8$,
$R_6$ represents a $C_{1-6}$ alkyl group,
$R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a

saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (7),

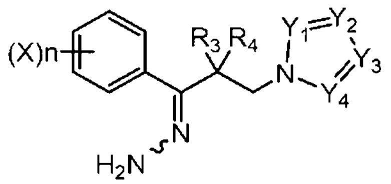 (7)

wherein in the formula, $R_3$, $R_4$, X, $Y_1$, $Y_2$, $Y_3$, $Y_4$, and n are as defined in the above formula (6),
with an acetal derivative represented by the following formula (8),

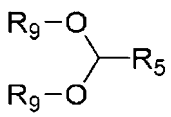 (8)

wherein in the formula, $R_5$ is as defined in the above formula (6), and $R_9$s represent a $C_{1-6}$ alkyl group and may be the same as or different from each other.

7. A method for producing a hydrazone derivative represented by the following formula (9),

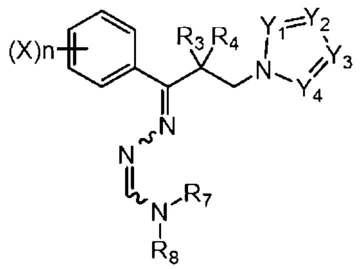 (9)

wherein in the formula, $R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (10),

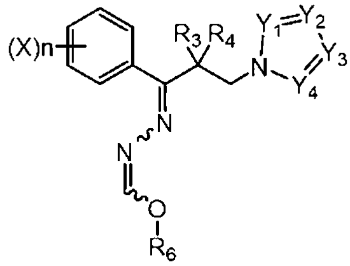

(10)

wherein in the formula, $R_3$, $R_4$, X, $Y_1$, $Y_2$, $Y_3$, $Y_4$, and n are as defined in the above formula (9), and $R_6$ represents a $C_{1-6}$ alkyl group,
with an amine derivative represented by the following formula (11),

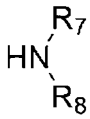

(11)

wherein in the formula, $R_7$ and $R_8$ are as defined in the above formula (9).

8. A method for producing a hydrazone derivative represented by the following formula (4),

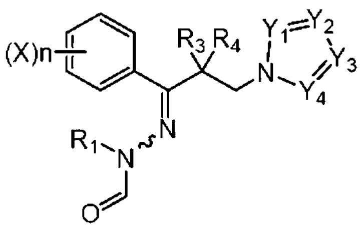

(4)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (12)

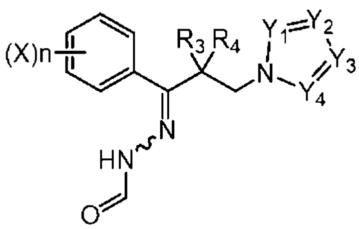

(12)

wherein in the formula, $R_3$, $R_4$, X, $Y_1$, $Y_2$, $Y_3$, $Y_4$, and n are as defined in the above formula (4),
with an alkylating agent represented by the following formula (13),

Z-$R_1$ (13)

wherein in the formula, $R_1$ is as defined in the above formula (4), and Z represents a chlorine atom, a bromine atom, an iodine atom, a methanesulfonyloxy group, a p-toluenesulfonyloxy group, or a trifluoromethanesulfonyloxy group.

9. A method for producing a hydrazone derivative represented by the following formula (2),

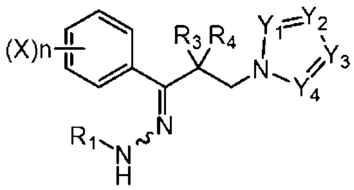

(2)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a ketone derivative represented by the following formula (3),

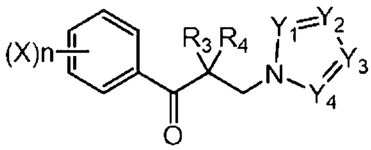

(3)

wherein in the formula, $R_3$, $R_4$, X, $Y_1$, $Y_2$, $Y_3$, $Y_4$, and n are as defined in the above formula (2),
with a hydrazine derivative represented by the following formula (14),

$NH_2NHR_1$ (14)

wherein in the formula, $R_1$ is as defined in the above formula (2).

10. An agricultural and horticultural chemical agent comprising the hydrazone derivative or a salt thereof according to 1 or 2 above.
11. An agricultural and horticultural nematode control agent comprising the hydrazone derivative or a salt thereof according to 1 or 2 above.
12. An agricultural and horticultural pest control agent comprising the hydrazone derivative or a salt thereof according to 1 or 2 above.
13. A method for controlling a plant pest, comprising a step of treating the pest and/or a habitat thereof and/or a seed and/or a plant propagation material with the agricultural and horticultural chemical agent according to 10 above.
14. A method for preventing a plant pest, comprising a step of treating a place where a useful crop is to be grown or is being grown, or a growing crop, with the agricultural and horticultural chemical agent according to 10 above.
15. A method for preparing an agrochemical composition, the method comprising a step of mixing the hydrazone derivative or a salt thereof according to claim 1 or 2 with any one of a carrier, an emulsifier, a wetting agent, a dispersing agent, and a disintegrant, or a combination of any of these.

Advantageous Effects of Invention

The compound of the present invention exhibits an excellent effect as an agricultural and horticultural chemical agent, particularly as an agricultural and horticultural pest control agent or a nematode control agent.

Description of Embodiments

In the present specification, the "halogen atom" represents a chlorine atom, a bromine atom, an iodine atom, or a fluorine atom.

"$C_{1-6}$ alkyl group" is a linear or branched alkyl group, and examples of such an alkyl group preferably include a methyl group, an ethyl group, an n-propyl group, an i-propyl group, an n-butyl group, an i-butyl group, an s-butyl group, a t-butyl group, an n-pentyl group, an isopentyl group, a neopentyl group, a tert-pentyl group, an n-hexyl group, an isohexyl group, and the like. The number of carbon atoms of the alkyl group is 1 to 6, and is preferably 1 to 3.

"$C_{2-6}$ alkenyl group" is a linear or branched alkyl group containing one or more unsaturated double bonds between carbon-carbon bonds, and examples of such an alkenyl group preferably include a vinyl group, an allyl group, an isopropenyl group, a 2-butenyl group, a 3-butenyl group, a 1-methyl-2-propenyl group, a 2-pentenyl group, a 3-pentenyl group, a 4-pentenyl group, a 1,1-dimethyl-2-propenyl group, a 1-ethyl-2-propenyl group, a 1-methyl-2-butenyl group, a 1-methyl-3-butenyl group, a 2-hexenyl group, a 3-hexenyl group, a 4-hexenyl group, a 5-hexenyl group, a 1,1-dimethyl-2-butenyl group, a 1,1-dimethyl-3-butenyl group, and the like. The unsaturated double bond can exist at any position between carbon-carbon bonds, and the number of unsaturated double bonds may be one or more. The number of carbon atoms of the alkenyl group is 2 to 6, and is preferably 3 to 4.

"$C_{2-6}$ alkynyl group" is a linear or branched alkyl group containing one or more unsaturated triple bonds between carbon-carbon bonds, and examples of such an alkynyl group preferably include an ethynyl group, a 2-propynyl group, a 1-methyl-2-propynyl group, a 1,1-dimethyl-2-propynyl group, a 1-butynyl group, a 2-butynyl group, a 3-butynyl group, a 1-pentynyl group, a 2-pentynyl group, a 3-pentynyl group, a 4-pentynyl group, a 1-methyl-2-butynyl group, a 1-methyl-3-butynyl group, a 1,1-dimethyl-2-butynyl group, a 1,1-dimethyl-3-butynyl group, a 1-methyl-3-pentynyl group, a 1-methyl-4-pentynyl group, and the like. The triple bond can exist at any position between carbon-carbon bonds, and its number may be one or more. The number of carbon atoms of the alkynyl group is 2 to 6, and is preferably 3 to 4.

"$C_{1-6}$ haloalkyl group" is a linear or branched alkyl group in which any carbon atom of the above "$C_{1-6}$ alkyl group" is substituted with one or more of the above halogen atoms, and examples of such a haloalkyl group preferably include a fluoromethyl group, a chloromethyl group, a difluoromethyl group, a trifluoromethyl group, a chlorodifluoromethyl group, a bromodifluoromethyl group, a dichlorofluoromethyl group, a 1-fluoroethyl group, a 2-fluoroethyl group, a 2-chloroethyl group, a 2-bromoethyl group, a 2-iodoethyl group, a 2,2,2-trifluoroethyl group, a 2,2,2-trichloroethyl group, a pentafluoroethyl group, a 2,2-difluoroethyl group, a 1-fluoroisopropyl group, a 3-fluoropropyl group, a 3-chloropropyl group, a 3-bromopropyl group, a heptafluoropropyl group, a heptafluoroisopropyl group, a 4-fluorobutyl group, a 4-chlorobutyl, a nonafluorobutyl group, and the like. The number of carbon atoms of the haloalkyl group is 1 to 6, and is preferably 1 to 3.

"$C_{1-6}$ alkoxy group" is a linear or branched alkyl group bonded to an oxygen atom, and examples of such an alkoxy group preferably include a methoxy group, an ethoxy group, an n-propoxy group, an isopropoxy group, a cyclopropyloxy group, an n-butoxy group, a sec-butoxy group, a tert-butoxy group, an n-pentoxy group, an n-hexyloxy group, and the like. The number of carbons of the alkoxy group is 1 to 6, and is preferably 1 to 4.

"$C_{1-6}$ haloalkoxy group" is an alkoxy group in which a halogen atom is substituted at any carbon atom of the above alkoxy group, and examples of such a haloalkoxy group preferably include a fluoromethoxy group, a dichloromethoxy group, a trichloromethoxy group, a difluoromethoxy group, a trifluoromethoxy group, a chlorodifluoromethoxy group, a bromodifluoromethoxy group, a dichlorofluoromethoxy group, a 1-fluoroethoxy group, a 2-fluoroethoxy group, a 2-chloroethoxy group, a 2-bromoethoxy group, a 2-iodoethoxy group, a 2,2,2-trifluoroethoxy group, a 2,2,2-trichloroethoxy group, a pentafluoroethoxy group, a 1-fluoroisopropoxy group, a 3-fluoropropoxy group, a 3-chloropropoxy group, a 3-bromopropoxy group, a 4-fluorobutoxy group, a 4-chlorobutoxy group, and the like. The number of carbon atoms of the haloalkoxy group is 1 to 6, and is preferably 1 to 3. Alternatively, in the haloalkoxy group, it may be substituted with 1 to 9 halogen atoms.

"$C_{3-6}$ cycloalkyl group" is a cyclic alkyl group, and examples of such a $C_{3-6}$ cycloalkyl group preferably include a cyclopropyl group, a cyclobutyl group, a cyclopentyl group, a cyclohexyl group, and the like. The number of carbon atoms of such a cycloalkyl group is preferably 3 to 6.

The benzyl group may be substituted with, for example, a $C_{1-6}$ alkyl group, a halogen atom, a $C_{1-6}$ alkoxy group, a $C_{1-6}$ haloalkyl group, or a $C_{1-6}$ haloalkoxy group.

In the compound described in the present specification, $R_7$ and $R_8$ may bond to each other and, together with the nitrogen atom to which $R_7$ and $R_8$ are bonded, form a saturated 4- to 7-membered ring. Examples of such a saturated 4- to 7-membered ring preferably include a pyrrolidine ring, a piperidine ring, and the like.

Examples of the salt of the hydrazone derivative represented by the formula (1) of the present invention include inorganic acid salts such as a hydrochloride, a sulfate, a nitrate, and a phosphate, and organic acid salts such as an acetate, a fumarate, a maleate, an oxalate, a methanesulfonate, a benzenesulfonate, and a p-toluenesulfonate.

The compound of the present invention may contain one or more asymmetric centers in its structural formula, and two or more types of optical isomers and diastereomers may exist; the present invention also encompasses all of each optical isomer and mixtures containing them in any proportion. The compound of the present invention may also have two

or more types of geometric isomers derived from a carbon-carbon double bond or a carbon-nitrogen double bond in its structural formula, but the present invention also encompasses all of each geometric isomer and mixtures containing them in any proportion.

For example, in the compounds described in the present specification, a wavy line in a structural formula means that any of two or more types of geometric isomers derived from a carbon-carbon double bond or a carbon-nitrogen double bond is included.

In the hydrazone derivative represented by the formula (1) of the present invention, it is preferable that $Y_2$ represents CR.

In the hydrazone derivative represented by the formula (1) of the present invention, it is preferable that R represents a $C_{1-6}$ haloalkyl group.

In the hydrazone derivative represented by the formula (1) of the present invention, it is desirable that $R_3$ represents a methyl group and $R_4$ represents a hydrogen atom. It is also preferable that $R_3$ and $R_4$ each represent a methyl group.

In the hydrazone derivative represented by the formula (1) of the present invention, it is more preferable that $Y_2$ represents CR, R represents a $C_{1-6}$ haloalkyl group, $R_3$ represents a methyl group, and $R_4$ represents a hydrogen atom. Alternatively, it is more preferable that $Y_2$ represents CR, R represents a $C_{1-6}$ haloalkyl group, and $R_3$ and $R_4$ each represent a methyl group.

Hereinafter, representative production methods for the novel hydrazone derivative represented by the formula (1) of the present invention and its intermediates, the hydrazone derivative represented by the formula (2) and the ketone derivative represented by the formula (3), are shown, but in the present invention, the production methods for the above compounds are not limited to these.

Reaction Scheme 1

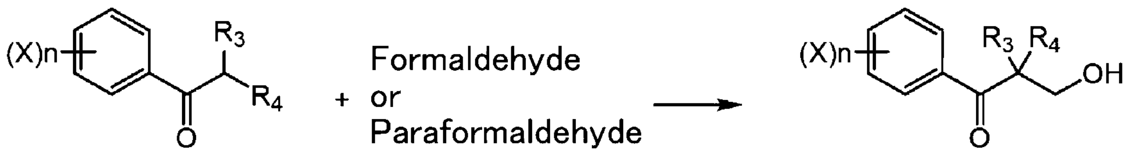


(15) (16)

[wherein in the formula, X, $R_3$, $R_4$, and n are as defined in the above formula (1).]

In the method shown in Reaction Scheme 1, the alcohol derivative represented by the above formula (16) can be produced, when $R_4$ is a hydrogen atom, by reacting the ketone compound represented by the above formula (15) with an aqueous formaldehyde solution in an inert solvent in the presence or absence of a base, according to the method described in the Journal of Organic Chemistry, Vol. 86, p. 6943 (2021), and when $R_4$ is a $C_{1-6}$ alkyl group, by reacting the ketone compound represented by the above formula (15) with paraformaldehyde according to the method described in Organic Preparations and Procedures International, Vol. 21, p. 91 (1989).

Reaction Scheme 2

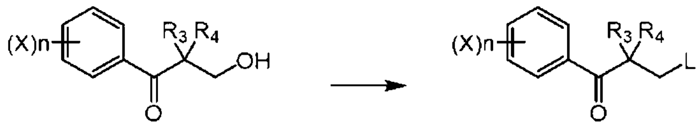

(16) (17)

[wherein in the formula, L represents a chlorine atom, a bromine atom, a p-toluenesulfonyloxy group, or a methanesulfonyloxy group, and X, $R_3$, $R_4$, and n are as defined in the above formula (1).]

In the method shown in Reaction Scheme 2, the ketone derivative represented by the above formula (17) can be produced by, for example, reacting the ketone compound represented by the above formula (16) with a halogenating agent, p-toluenesulfonyl chloride, methanesulfonyl chloride, or the like, in the presence or absence of a base.

As the solvent used in the halogenation reaction or sulfonic acid esterification reaction of the compound of the

formula (16), a known solvent can be widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; ether solvents such as diethyl ether, tetrahydrofuran, and 1,4-dioxane; ester solvents such as methyl acetate and ethyl acetate; ketone solvents such as acetone and methyl ethyl ketone; amide solvents such as N,N-dimethylformamide; nitrile solvents such as acetonitrile and propionitrile; and aprotic polar solvents such as N-methylpyrrolidone and N,N'-dimethylimidazolinone. These solvents can be used alone as one type or as a mixture of two or more types as needed.

As the base used in the sulfonic acid esterification reaction of the compound of the formula (16), known inorganic bases and organic bases can be used. Examples of the inorganic base include alkali metal carbonates such as sodium carbonate, potassium carbonate, and sodium bicarbonate; alkali metal hydroxides such as sodium hydroxide and potassium hydroxide; and alkali metal hydrides such as sodium hydride and potassium hydride. Examples of the organic base include alkali metal alkoxides such as sodium methoxide, sodium ethoxide, and potassium tert-butoxide; and amines such as triethylamine and pyridine. These bases may be used alone as one type or as a mixture of two or more types.

Such a base can be usually used in an amount of 1 to 100 equivalents with respect to the ketone compound represented by the formula (16).

Examples of the halogenating agent used in the halogenation reaction of the ketone compound represented by the formula (16) include chlorine, bromine, N-chlorosuccinimide, N-bromosuccinimide, thionyl chloride, sulfuryl chloride, phosphorus oxychloride, phosphorus tribromide, and phosphorus pentabromide.

The reaction can usually be carried out within a range from -78°C to the boiling point of the solvent to be used, and it is preferable to carry out the reaction at room temperature to 100°C.

The reaction time varies depending on the reaction temperature and the like and cannot be unconditionally stated, but the reaction is usually completed in about 0.5 to 24 hours.

The target compound obtained in each of the above reactions can be easily isolated from the reaction mixture by commonly used isolation means, for example, an organic solvent extraction method, a chromatography method, a recrystallization method, a distillation method, or the like. It is also possible to proceed to the next reaction step without isolating the target product from the reaction system.

Reaction Scheme 3

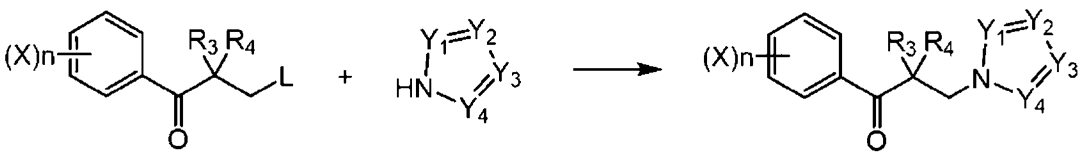

(17) (18) (3)

[wherein in the formula, X, $Y_1$, $Y_2$, $Y_3$, $Y_4$, L, $R_3$, $R_4$, and n are as defined in the above formulas (1) and (17).]

In the method shown in Reaction Scheme 3, the ketone derivative represented by the formula (3) can be produced by reacting the ketone compound represented by the formula (17) with the azole compound of the formula (18) in an inert solvent in the presence or absence of a base.

As the solvent used in the reaction between the compound of the formula (17) and the compound of the formula (18), a known solvent can be widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; ether solvents such as diethyl ether, tetrahydrofuran, and 1,4-dioxane; ester solvents such as methyl acetate and ethyl acetate; ketone solvents such as acetone and methyl ethyl ketone; amide solvents such as N,N-dimethylformamide; nitrile solvents such as acetonitrile and propionitrile; and aprotic polar solvents such as N-methylpyrrolidone and N,N'-dimethylimidazolinone. These solvents can be used alone as one type or as a mixture of two or more types as needed.

As the base used in the reaction between the compound of the formula (17) and the compound of the formula (18), known inorganic bases and organic bases can be used. Examples of the inorganic base include alkali metal carbonates such as sodium carbonate, potassium carbonate, and sodium bicarbonate; alkali metal hydroxides such as sodium hydroxide and potassium hydroxide; and alkali metal hydrides such as sodium hydride and potassium hydride. Examples of the organic base include alkali metal alkoxides such as sodium methoxide, sodium ethoxide, and potassium tert-butoxide; and amines such as triethylamine and pyridine. These bases may be used alone as one type or as a mixture of two or more types.

Such a base can be used in an amount that is usually 1 to 100 equivalents, and preferably 1 to 2 equivalents, with respect to the ketone compound represented by the formula (17).

The use ratio of the ketone compound represented by the formula (17) and the azole compound represented by the formula (18) is more preferably 1 to 2 equivalents of compound (18) to compound (17).

The reaction can usually be carried out within a range from -78°C to the boiling point of the solvent to be used, and it is preferable to carry out the reaction at room temperature to 100°C.

The reaction time varies depending on the reaction temperature and the like and cannot be unconditionally stated, but the reaction is usually completed in about 0.5 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Reaction Scheme 4

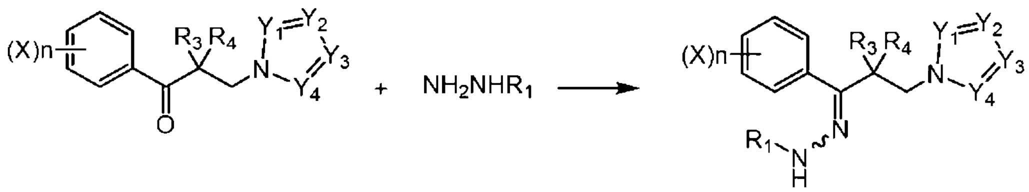

(3) (14) (2)

[wherein in the formula, X, $Y_1$, $Y_2$, $Y_3$, $Y_4$, $R_1$, $R_3$, $R_4$, and n are as defined in the above formula (1).]

In the method shown in Reaction Scheme 4, the hydrazone derivative represented by the formula (2) can be produced by reacting the ketone compound represented by the formula (3) with the hydrazine compound of the formula (14) in an inert solvent in the presence or absence of an acid.

The solvent used in the reaction between the ketone derivative of the formula (3) and the hydrazine compound of the formula (14) can be a known solvent widely used as long as it is inert to the reaction. Examples of such a solvent include alcohols such as methanol, ethanol, n-propanol, and isopropanol; aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; ether solvents such as diethyl ether, tetrahydrofuran, and 1,4-dioxane; ester solvents such as methyl acetate and ethyl acetate; amide solvents such as N,N-dimethylformamide; and solvents such as N-methylpyrrolidone, N,N'-dimethylimidazolinone, and acetonitrile. These solvents can be used alone as one type or as a mixture of two or more types as needed.

As the acid used in the reaction between the compound of the formula (3) and the compound of the formula (14), known inorganic acids and organic acids can be used. Examples of the inorganic acid include hydrogen chloride, hydrogen bromide, sulfuric acid, and nitric acid. Examples of the organic acid include acetic acid, propionic acid, citric acid, methanesulfonic acid, ethanesulfonic acid, and p-toluenesulfonic acid. These acids may be used alone as one type or as a mixture of two or more types.

The acid used in the reaction between the compound of the formula (3) and the compound of the formula (14) can be used in an amount that is usually 0.001 to 2 equivalents, and preferably 0.01 to 1 equivalent, with respect to the ketone compound represented by the formula (3).

The use ratio of the ketone derivative of the formula (3) and the hydrazine compound represented by the formula (14) can be appropriately selected from a wide range, but it is preferable to use 1 to 10 mol or more of the latter per 1 mol of the former, and more preferably 2.0 to 5.0 mol.

The reaction is preferably carried out at 0°C to the boiling point of the solvent to be used, and more preferably at room temperature to under heating at reflux.

The reaction time varies depending on the reaction temperature and the like and cannot be unconditionally stated, but the reaction is usually completed in about 0.1 to 24 hours.

After completion of the reaction, the target product can be isolated from the reaction system containing the target product by a conventional method, and if necessary, the target product can be produced by purification by recrystallization, column chromatography, or the like. It is also possible to proceed to the next reaction step without isolating the target product from the reaction system.

Reaction Scheme 5

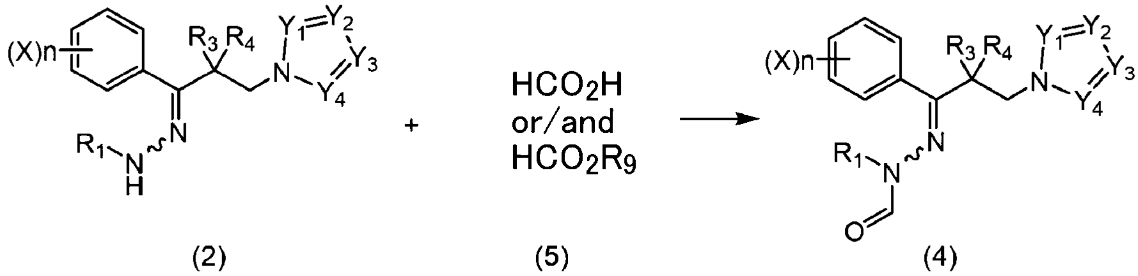


(2) (5) (4)

[wherein in the formula, $R_9$ represents a $C_{1-6}$ alkyl group, and X, $Y_1$, $Y_2$, $Y_3$, $Y_4$, $R_1$, $R_3$, $R_4$, and n are as defined in the above formula (1).]

In the method shown in Reaction Scheme 5, the hydrazone derivative represented by the formula (4) can be produced by a formylation reaction of the hydrazone derivative represented by the formula (2) with formic acid and/or the formic acid derivative represented by the formula (5).

The solvent used in the formylation reaction of the hydrazone compound represented by the formula (2) can be a known solvent widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; alcohol-based solvents such as methanol and ethanol; ester-based solvents such as methyl formate, ethyl formate, methyl acetate, and ethyl acetate; ketone-based solvents such as acetone and methyl ethyl ketone; amide-based solvents such as N,N-dimethylformamide; nitrile-based solvents such as acetonitrile and propionitrile; aprotic polar solvents such as N-methylpyrrolidone and N,N'-dimethylimidazolinone; and acetic acid. These solvents can be used alone as one type or as a mixture of two or more types as needed.

Examples of the formylating agent used in the formylation reaction of the hydrazone compound represented by the formula (2) include formic acid, methyl formate, and ethyl formate.

Such a formylating agent can be used in an amount that is usually 1 to 100 equivalents, and preferably 1 to 10 equivalents, with respect to the hydrazone derivative represented by the formula (2).

The reaction can usually be carried out within a range from -78°C to the boiling point of the solvent to be used, and it is preferable to carry out the reaction at 0°C to the boiling point.

The reaction time varies depending on the reaction temperature and the like and cannot be unconditionally stated, but the reaction is usually completed in about 0.1 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Reaction Scheme 6

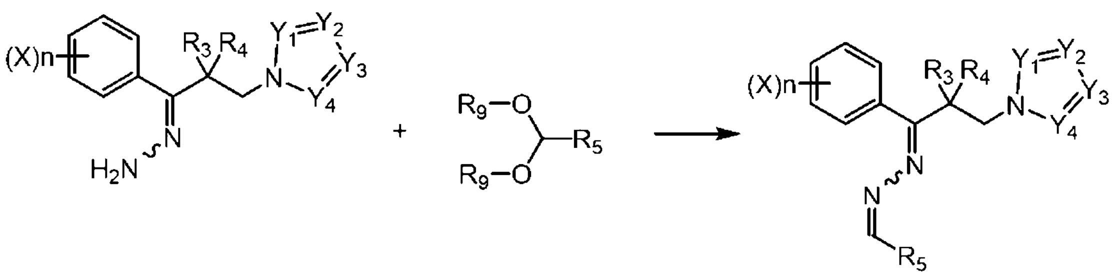

(7) (8) (6)

[wherein in the formula, X, $Y_1$, $Y_2$, $Y_3$, $Y_4$, $R_3$, $R_4$, $R_5$, $R_9$, and n are as defined in the above formulas (1) and (5).]

Regarding this Reaction Scheme 6, specific examples of its embodiments include those shown in Reaction Schemes 7, 8, and 9 described below. The compound defined by formula (7) is a compound in which $R_1$ is a hydrogen atom in formula (2), which has already been described.

Reaction Scheme 7

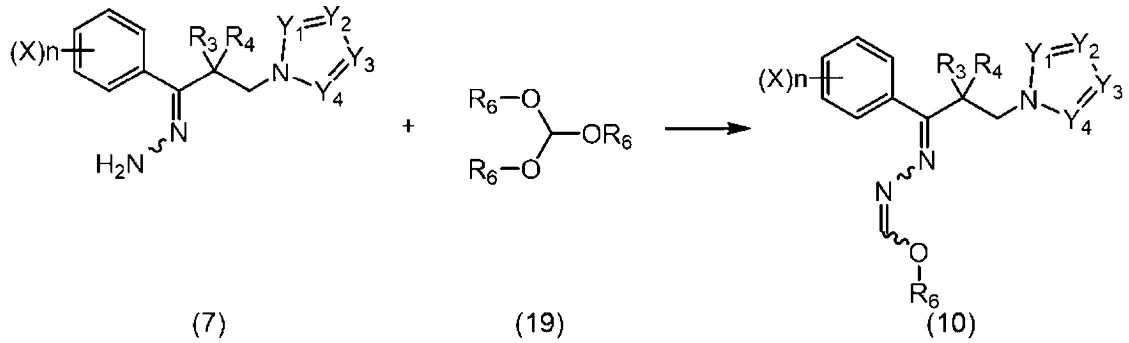

(7) (19) (10)

[wherein in the formula, X, $Y_1$, $Y_2$, $Y_3$, $Y_4$, $R_3$, $R_4$, $R_6$, and n are as defined in the above formula (1).]

In the method shown in Reaction Scheme 7, the hydrazone derivative represented by the formula (10) can be produced by the reaction of the hydrazone derivative represented by the formula (7) with an orthoformate ester (19).

The solvent used in the reaction of the hydrazone compound represented by the formula (7) and the orthoformate ester represented by the formula (19) can be a known solvent widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; alcohol-based solvents such as methanol and ethanol; ester-based solvents such as methyl formate, ethyl formate, methyl acetate, and ethyl acetate; ketone-based solvents such as acetone and methyl ethyl ketone; amide-based solvents such as N,N-dimethylformamide; nitrile-based solvents such as acetonitrile and propionitrile; aprotic polar solvents such as N-methylpyrrolidone and N,N'-dimethylimidazolinone; and acetic acid. These solvents can be used alone as one type or as a mixture of two or more types as needed.

Examples of the orthoformate ester (19) used in the imination reaction of the hydrazone compound represented by the formula (7) include methyl orthoformate, ethyl orthoformate, propyl orthoformate, and butyl orthoformate.

Such an orthoformate ester (19) can be used in an amount that is usually 1 to 20 equivalents, and preferably 2 to 10 equivalents, with respect to the hydrazone derivative represented by the formula (7).

The reaction can usually be carried out within a range from -78°C to the boiling point of the solvent to be used, and it is preferable to carry out the reaction at 0°C to the boiling point.

The reaction time varies depending on the reaction temperature and the like and cannot be unconditionally stated, but the reaction is usually completed in about 0.1 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Reaction Scheme 8

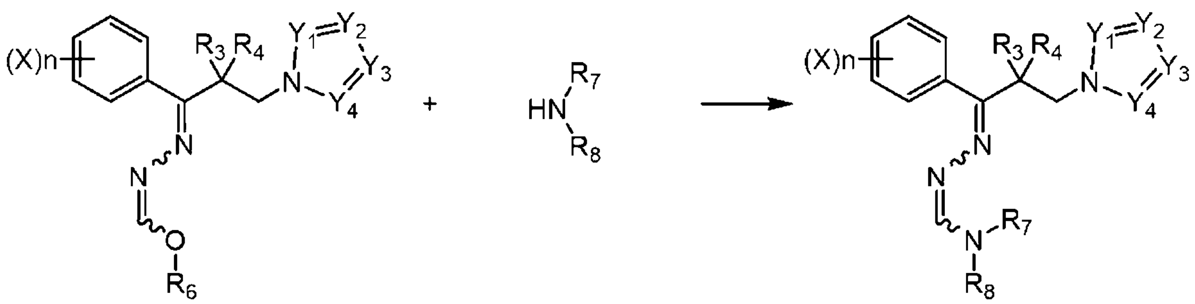

(10) (11) (9)

[wherein in the formula, X, $Y_1$, $Y_2$, $Y_3$, $Y_4$, $R_3$, $R_4$, $R_6$, $R_7$, $R_8$, and n are as defined in the above formula (1).]

In the method shown in Reaction Scheme 8, the hydrazone derivative represented by the formula (9) can be produced by the reaction of the hydrazone derivative represented by the formula (10) with the amine derivative represented by the formula (11).

The solvent used in the amination reaction of the hydrazone compound represented by the formula (10) can be a known solvent widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; alcohol-based solvents such as methanol and ethanol; ester-based solvents such as methyl formate, ethyl formate, methyl acetate, and ethyl acetate; ketone-based solvents such as acetone and methyl ethyl ketone; amide-based solvents such as N,N-dimethylformamide; nitrile-based solvents such as acetonitrile and propionitrile; aprotic polar solvents such as N-methylpyrrolidone and N,N'-dimethylimidazolinone; and acetic acid. These solvents can be used alone as one type or as a mixture of two or more types as needed.

The amine derivative (11) can be used in an amount that is usually 1 to 5 equivalents, and preferably 1 to 2.5 equivalents, with respect to the hydrazone derivative represented by the formula (10).

The reaction can usually be carried out within a range from -78°C to the boiling point of the solvent to be used, and it is preferable to carry out the reaction at 0°C to the boiling point.

The reaction time varies depending on the reaction temperature and the like and cannot be unconditionally stated, but the reaction is usually completed in about 0.1 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Reaction Scheme 9

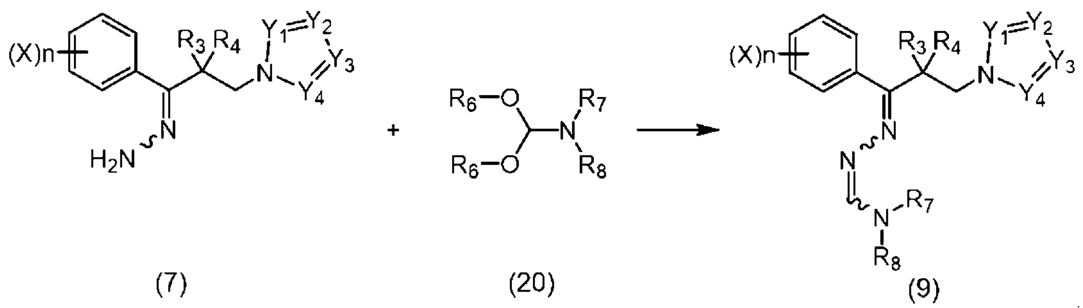

(7) (20) (9)

[wherein in the formula, X, $Y_1$, $Y_2$, $Y_3$, $Y_4$, $R_3$, $R_4$, $R_6$, $R_7$, $R_8$, and n are as defined in the above formula (1).]

In the method shown in Reaction Scheme 9, the hydrazone derivative represented by the formula (9) can be produced by the reaction of the hydrazone derivative represented by the formula (7) with an acetal derivative (20).

The solvent used in the reaction of the hydrazone compound represented by the formula (7) and the acetal derivative represented by the formula (20) can be a known solvent widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; alcohol-based solvents such as methanol and ethanol; ester-based solvents such as methyl formate, ethyl formate, methyl acetate, and ethyl acetate; ketone-based solvents such as acetone and methyl ethyl ketone; amide-based solvents such as N,N-dimethylformamide; nitrile-based solvents such as acetonitrile and propionitrile; aprotic polar solvents such as N-methylpyrrolidone and N,N'-dimethylimidazolinone; and acetic acid. These solvents can be used alone as one type or as a mixture of two or more types as needed.

Examples of the acetal derivative (20) used in the imination reaction of the hydrazone compound represented by the formula (7) include N,N-dimethylformamide dimethyl acetal.

The acetal derivative (20) can be used in an amount that is usually 1 to 10 equivalents, and preferably 1 to 5 equivalents, with respect to the hydrazone derivative represented by the formula (7).

The reaction can usually be carried out within a range from -78°C to the boiling point of the solvent to be used, and it is preferable to carry out the reaction at 0°C to the boiling point.

The reaction time varies depending on the reaction temperature and the like and cannot be unconditionally stated, but the reaction is usually completed in about 0.1 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Reaction Scheme 10

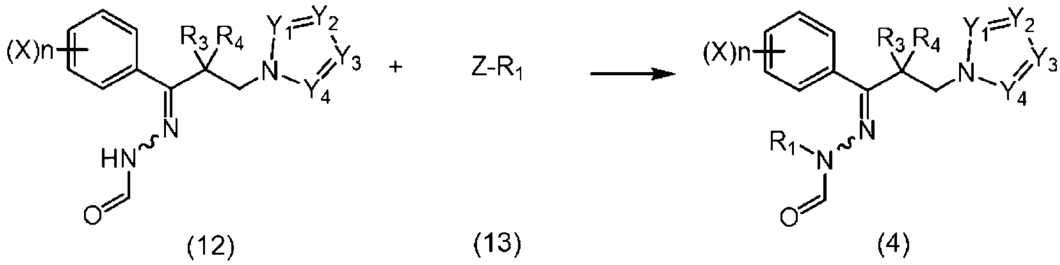

(12) (13) (4)

[wherein in the formula, Z represents a chlorine atom, a bromine atom, an iodine atom, a methanesulfonyloxy group, a p-toluenesulfonyloxy group, or a trifluoromethanesulfonyloxy group, X, $Y_1$, $Y_2$, $Y_3$, $Y_4$, $R_3$, $R_4$, and n are as defined in the above formula (1), and $R_1$ means a substituent other than a hydrogen atom.]

The compound defined by formula (12) is a compound in which $R_1$ is a hydrogen atom in formula (4), for which a production method has already been described.

In the method shown in Reaction Scheme 10, the hydrazone derivative represented by the formula (4) can be produced by reacting the hydrazone derivative represented by the formula (12) with the alkylating agent represented by the formula (13) in an inert solvent in the presence or absence of a base.

The solvent used in the alkylation reaction of the hydrazone derivative represented by the formula (12) can be a known solvent widely used as long as it is inert to the reaction. Examples of such a solvent include aliphatic or alicyclic hydrocarbon solvents such as hexane, cyclohexane, and heptane; aromatic hydrocarbon solvents such as benzene, chlorobenzene, toluene, and xylene; halogenated hydrocarbon solvents such as methylene chloride, 1,2-dichloroethane, and chloroform; alcohol-based solvents such as methanol and ethanol; ester-based solvents such as methyl formate, ethyl formate, methyl acetate, and ethyl acetate; ketone-based solvents such as acetone and methyl ethyl ketone; amide-based solvents such as N,N-dimethylformamide; nitrile-based solvents such as acetonitrile and propionitrile; aprotic polar solvents such as N-methylpyrrolidone and N,N'-dimethylimidazolinone; and acetic acid. These solvents can be used alone as one type or as a mixture of two or more types as needed.

Examples of the alkylating agent (13) used in the alkylation reaction of the hydrazone compound represented by the formula (12) include methyl iodide, ethyl iodide, and dimethyl sulfate.

Such an alkylating agent can be used in an amount that is usually 1 to 10 equivalents, and preferably 1 to 3 equivalents, with respect to the hydrazone derivative represented by the formula (12).

The reaction can usually be carried out within a range from -78°C to the boiling point of the solvent to be used, and it is preferable to carry out the reaction at 0°C to the boiling point.

The reaction time varies depending on the reaction temperature and the like and cannot be unconditionally stated, but the reaction is usually completed in about 0.1 to 24 hours.

The target compound obtained in each of the above reactions can be easily isolated from the reaction mixture by commonly used isolation means, for example, an organic solvent extraction method, a chromatography method, a recrystallization method, a distillation method, or the like.

Representative compounds of the hydrazone derivative represented by the formula (1) are shown in Table 1, representative compounds of the hydrazone derivative represented by the formula (2) are shown in Table 2, representative compounds of the ketone derivative represented by the formula (3) are shown in Table 3, representative compounds of the ketone derivative represented by the formula (16) are shown in Table 4, and representative compounds of the ketone derivative represented by the formula (17) are shown in Table 5, but the scope of the present invention is not limited to these compounds.

In Tables 1 to 5, the physical properties are shown as the state or melting point (°C), "Me" represents a methyl group, "Et" represents an ethyl group, "i-Pr" represents an isopropyl group, and "Bn" represents a benzyl group.

General Formula (1)

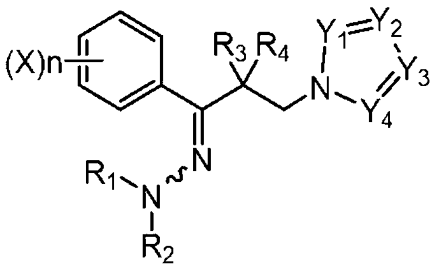

(1)

Table 1-1

| Compound No. | Ri | $R_2$ | R3 | $R_4$ | X | $Y_1$ | $Y_2$ | $Y_3$ | $Y_4$ | State, Melting Point (°C) |
|---|---|---|---|---|---|---|---|---|---|---|
| A-1 | H | CHO | Me | H | H | N | $CCF_3$ | N | CH | amorphous |
| A-2 | Me | CHO | Me | H | H | N | $CCF_3$ | N | CH | |
| A-3 | Et | CHO | Me | H | H | N | $CCF_3$ | N | CH | |
| A-4 | $CH_2CCH$ | CHO | Me | H | H | N | $CCF_3$ | N | CH | |
| A-5 | $CHN(Me)_2$ | | Me | H | H | N | $CCF_3$ | N | CH | oil |
| A-6 | H | CHO | Me | H | H | N | $CCF_3$ | CH | CH | |
| A-7 | Me | CHO | Me | H | H | N | $CCF_3$ | CH | CH | |
| A-8 | Et | CHO | Me | H | H | N | $CCF_3$ | CH | CH | |
| A-9 | $CH_2CCH$ | CHO | Me | H | H | N | $CCF_3$ | CH | CH | |
| A-10 | $CHN(Me)_2$ | | Me | H | H | N | $CCF_3$ | CH | CH | |
| A-11 | $CH_2CCH$ | CHO | Me | H | H | CH | $CCF_3$ | N | CH | |
| A-12 | $CHN(Me)_2$ | | Me | H | H | CH | $CCF_3$ | N | CH | |
| A-13 | H | CHO | Me | H | H | CH | $CCF_3$ | CH | N | |
| A-14 | $CHN(Me)_2$ | | Me | H | H | CH | $CCF_3$ | CH | N | |
| A-15 | H | CHO | Me | H | H | N | $CCF_3$ | N | N | |
| A-16 | $CHN(Me)_2$ | | Me | H | H | N | $CCF_3$ | N | N | |
| A-17 | H | CHO | Me | H | 4-F | N | $CCF_3$ | N | CH | oil |
| A-18 | Me | CHO | Me | H | 4-F | N | $CCF_3$ | N | CH | |
| A-19 | Et | CHO | Me | H | 4-F | N | $CCF_3$ | N | CH | |
| A-20 | $CH_2CCH$ | CHO | Me | H | 4-F | N | $CCF_3$ | N | CH | |

Table 1-2

| | | | | | | | | | | |
|---|---|---|---|---|---|---|---|---|---|---|
| A-21 | $CHN(Me)_2$ | | Me | H | 4-F | N | $CCF_3$ | N | CH | oil |
| A-22 | H | CHO | Me | H | 4-F | N | $CCF_3$ | CH | CH | oil |
| A-23 | Me | CHO | Me | H | 4-F | N | $CCF_3$ | CH | CH | |
| A-24 | Et | CHO | Me | H | 4-F | N | $CCF_3$ | CH | CH | |
| A-25 | $CH_2CCH$ | CHO | Me | H | 4-F | N | $CCF_3$ | CH | CH | |
| A-26 | $CHN(Me)_2$ | | Me | H | 4-F | N | $CCF_3$ | CH | CH | oil |
| A-27 | H | CH0 | Me | H | 4-Cl | N | $CCF_3$ | N | CH | solid |

(continued)

| | | | | | | | | | | |
|---|---|---|---|---|---|---|---|---|---|---|
| A-28 | Me | CHO | Me | H | 4-Cl | N | $CCF_3$ | N | CH | oil |
| A-29 | Et | CHO | Me | H | 4-Cl | N | $CCF_3$ | N | CH | |
| A-30 | $CH_2CH=CH_2$ | CHO | Me | H | 4-Cl | N | $CCF_3$ | N | CH | oil |
| A-31 | $CH_2CCH$ | CHO | Me | H | 4-Cl | N | $CCF_3$ | N | CH | |
| A-32 | Bn | CHO | Me | H | 4-Cl | N | $CCF_3$ | N | CH | |
| A-33 | $CHN(Me)_2$ | | Me | H | 4-Cl | N | $CCF_3$ | N | CH | oil |
| A-34 | CHN(Me) Et | | Me | H | 4-Cl | N | $CCF_3$ | N | CH | |
| A-35 | $CHN(Et)_2$ | | Me | H | 4-Cl | N | $CCF_3$ | N | CH | |
| A-36 | CHNHMe | | Me | H | 4-Cl | N | $CCF_3$ | N | CH | |
| A-37 | CHNHEt | | Me | H | 4-Cl | N | $CCF_3$ | N | CH | |
| A-38 | 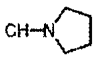 | | Me | H | 4-Cl | N | $CCF_3$ | N | CH | |
| A-39 | 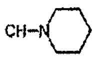 | | Me | H | 4-Cl | N | $CCF_3$ | N | CH | |
| A-40 | CHOMe | | Me | H | 4-Cl | N | $CCF_3$ | N | CH | |

Table 1-3

| | | | | | | | | | | |
|---|---|---|---|---|---|---|---|---|---|---|
| A-41 | CHOEt | | Me | H | 4-Cl | N | $CCF_3$ | N | CH | |
| A-42 | H | CHO | Me | H | 4-Cl | N | $CCF_3$ | CH | CH | oil |
| A-43 | Me | CHO | Me | H | 4-Cl | N | $CCF_3$ | CH | CH | |
| A-44 | Et | CHO | Me | H | 4-Cl | N | $CCF_3$ | CH | CH | |
| A-45 | $CH_2CCH$ | CHO | Me | H | 4-Cl | N | $CCF_3$ | CH | CH | |
| A-46 | $CHN(Me)_2$ | | Me | H | 4-Cl | N | $CCF_3$ | CH | CH | oil |
| A-47 | H | CHO | Me | H | 4-Br | N | $CCF_3$ | N | CH | oil |
| A-48 | Me | CHO | Me | H | 4-Br | N | $CCF_3$ | N | CH | |
| A-49 | Et | CHO | Me | H | 4-Br | N | $CCF_3$ | N | CH | |
| A-50 | $CH_2CCH$ | CHO | Me | H | 4-Br | N | $CCF_3$ | N | CH | |
| A-51 | $CHN(Me)_2$ | | Me | H | 4-Br | N | $CCF_3$ | N | CH | oil |
| A-52 | H | CHO | Me | H | 4-Br | N | $CCF_3$ | CH | CH | oil |
| A-53 | Me | CHO | Me | H | 4-Br | N | $CCF_3$ | CH | CH | |
| A-54 | Et | CHO | Me | H | 4-Br | N | $CCF_3$ | CH | CH | |
| A-55 | $CH_2CCH$ | CHO | Me | H | 4-Br | N | $CCF_3$ | CH | CH | |
| A-56 | $CHN(Me)_2$ | | Me | H | 4-Br | N | $CCF_3$ | CH | CH | oil |
| A-57 | H | CHO | Me | H | 4-$CF_3$ | N | $CCF_3$ | N | CH | |
| A-58 | Me | CHO | Me | H | 4-$CF_3$ | N | $CCF_3$ | N | CH | |
| A-59 | Et | CHO | Me | H | 4-$CF_3$ | N | $CCF_3$ | N | CH | |
| A-60 | $CH_2CCH$ | CHO | Me | H | 4-$CF_3$ | N | $CCF_3$ | N | CH | |

Table 1-4

| | | | | | | | | | | |
|---|---|---|---|---|---|---|---|---|---|---|
| A-61 | $CHN(Me)_2$ | | Me | H | 4-$CF_3$ | N | $CCF_3$ | N | CH | |
| A-62 | H | CHO | Me | H | 4-$CF_3$ | N | $CCF_3$ | CH | CH | |

(continued)

| | | | | | | | | | | |
|---|---|---|---|---|---|---|---|---|---|---|
| A-63 | Me | CHO | Me | H | 4-$CF_3$ | N | $CCF_3$ | CH | CH | |
| A-64 | Et | CHO | Me | H | 4-$CF_3$ | N | $CCF_3$ | CH | CH | |
| A-65 | $CH_2CCH$ | CHO | Me | H | 4-$CF_3$ | N | $CCF_3$ | CH | CH | |
| A-66 | $CHN(Me)_2$ | | Me | H | 4-$CF_3$ | N | $CCF_3$ | CH | CH | |
| A-67 | H | CHO | Me | H | 4-I | N | $CCF_3$ | N | CH | |
| A-68 | $CHN(Me)_2$ | | Me | H | 4-I | N | $CCF_3$ | N | CH | |
| A-69 | H | CHO | Me | H | 4-I | N | $CCF_3$ | CH | CH | |
| A-70 | $CHN(Me)_2$ | | Me | H | 4-I | N | $CCF_3$ | CH | CH | |
| A-71 | H | CHO | Me | H | 4-Me | N | $CCF_3$ | N | CH | oil |
| A-72 | $CHN(Me)_2$ | | Me | H | 4-Me | N | $CCF_3$ | N | CH | |
| A-73 | H | CHO | Me | H | 4-Me | N | $CCF_3$ | CH | CH | |
| A-74 | $CHN(Me)_2$ | | Me | H | 4-Me | N | $CCF_3$ | CH | CH | |
| A-75 | H | CHO | Me | H | 4-CN | N | $CCF_3$ | N | CH | oil |
| A-76 | $CHN(Me)_2$ | | Me | H | 4-CN | N | $CCF_3$ | N | CH | |
| A-77 | H | CHO | Me | H | 4-CN | N | $CCF_3$ | CH | CH | |
| A-78 | $CHN(Me)_2$ | | Me | H | 4-CN | N | $CCF_3$ | CH | CH | |
| A-79 | H | CHO | Me | H | 4-$NO_2$ | N | $CCF_3$ | N | CH | |
| A-80 | $CHN(Me)_2$ | | Me | H | 4-$NO_2$ | N | $CCF_3$ | N | CH | |

Table 1-5

| | | | | | | | | | | |
|---|---|---|---|---|---|---|---|---|---|---|
| A-81 | H | CHO | Me | H | 4-$NO_2$ | N | $CCF_3$ | CH | CH | |
| A-82 | $CHN (Me)_2$ | | Me | H | 4-$NO_2$ | N | $CCF_3$ | CH | CH | |
| A-83 | H | CHO | Me | H | 4-MeO | N | $CCF_3$ | N | CH | oil |
| A-84 | $CHN(Me)_2$ | | Me | H | 4-MeO | N | $CCF_3$ | N | CH | |
| A-85 | H | CHO | Me | H | 4-MeO | N | $CCF_3$ | CH | CH | |
| A-86 | $CHN(Me)_2$ | | Me | H | 4-MeO | N | $CCF_3$ | CH | CH | |
| A-87 | H | CHO | Me | H | 4-$CF_3O$ | N | $CCF_3$ | N | CH | oil |
| A-88 | $CHN(Me)_2$ | | Me | H | 4-$CF_3O$ | N | $CCF_3$ | N | CH | |
| A-89 | H | CHO | Me | H | 4-$CF_3O$ | N | $CCF_3$ | CH | CH | |
| A-90 | $CHN(Me)_2$ | | Me | H | 4-$CF_3O$ | N | $CCF_3$ | CH | CH | |
| A-91 | H | CHO | Me | H | 2-F | N | $CCF_3$ | N | CH | amorphou s |
| A-92 | Me | CHO | Me | H | 2-F | N | $CCF_3$ | N | CH | |
| A-93 | Et | CHO | Me | H | 2-F | N | $CCF_3$ | N | CH | |
| A-94 | $CH_2CCH$ | CHO | Me | H | 2-F | N | $CCF_3$ | N | CH | |
| A-95 | $CHN(Me)_2$ | | Me | H | 2-F | N | $CCF_3$ | N | CH | oil |
| A-96 | H | CHO | Me | H | 2-F | N | $CCF_3$ | CH | CH | |
| A-97 | Me | CHO | Me | H | 2-F | N | $CCF_3$ | CH | CH | |
| A-98 | Et | CHO | Me | H | 2-F | N | $CCF_3$ | CH | CH | |
| A-99 | CH2CCH | CHO | Me | H | 2-F | N | $CCF_3$ | CH | CH | |
| A-100 | $CHN(Me)_2$ | | Me | H | 2-F | N | $CCF_3$ | CH | CH | |

Table 1-6

| | | | | | | | | | | |
|---|---|---|---|---|---|---|---|---|---|---|
| A-101 | H | CHO | Me | H | 2-Cl | N | $CCF_3$ | N | CH | |
| A-102 | Me | CHO | Me | H | 2-Cl | N | $CCF_3$ | N | CH | |
| A-103 | Et | CHO | Me | H | 2-Cl | N | $CCF_3$ | N | CH | |
| A-104 | $CH_2CCH$ | CHO | Me | H | 2-Cl | N | $CCF_3$ | N | CH | |
| A-105 | $CHN(Me)_2$ | | Me | H | 2-Cl | N | $CCF_3$ | N | CH | |
| A-106 | H | CHO | Me | H | 2-Cl | N | $CCF_3$ | CH | CH | |
| A-107 | Me | CHO | Me | H | 2-Cl | N | $CCF_3$ | CH | CH | |
| A-108 | Et | CHO | Me | H | 2-Cl | N | $CCF_3$ | CH | CH | |
| A-109 | $CH_2CCH$ | CHO | Me | H | 2-Cl | N | $CCF_3$ | CH | CH | |
| A-110 | $CHN(Me)_2$ | | Me | H | 2-Cl | N | $CCF_3$ | CH | CH | |
| A-111 | H | CHO | Me | H | 2-Br | N | $CCF_3$ | N | CH | |
| A-112 | Me | CHO | Me | H | 2-Br | N | $CCF_3$ | N | CH | |
| A-113 | Et | CHO | Me | H | 2-Br | N | $CCF_3$ | N | CH | |
| A-114 | $CH_2CCH$ | CHO | Me | H | 2-Br | N | $CCF_3$ | N | CH | |
| A-115 | $CHN(Me)_2$ | | Me | H | 2-Br | N | $CCF_3$ | N | CH | |
| A-116 | H | CHO | Me | H | 2-Br | N | $CCF_3$ | CH | CH | |
| A-117 | Me | CHO | Me | H | 2-Br | N | $CCF_3$ | CH | CH | |
| A-118 | Et | CHO | Me | H | 2-Br | N | $CCF_3$ | CH | CH | |
| A-119 | $CH_2CCH$ | CHO | Me | H | 2-Br | N | $CCF_3$ | CH | CH | |
| A-120 | $CHN(Me)_2$ | | Me | H | 2-Br | N | $CCF_3$ | CH | CH | |

Table 1-7

| | | | | | | | | | | |
|---|---|---|---|---|---|---|---|---|---|---|
| A-121 | H | CHO | Me | H | 3-F | N | $CCF_3$ | N | CH | oil |
| A-122 | Me | CHO | Me | H | 3-F | N | $CCF_3$ | N | CH | |
| A-123 | Et | CHO | Me | H | 3-F | N | $CCF_3$ | N | CH | |
| A-124 | $CH_2CCH$ | CHO | Me | H | 3-F | N | $CCF_3$ | N | CH | |
| A-125 | $CHN(Me)_2$ | | Me | H | 3-F | N | $CCF_3$ | N | CH | oil |
| A-126 | H | CHO | Me | H | 3-F | N | $CCF_3$ | CH | CH | |
| A-127 | Me | CHO | Me | H | 3-F | N | $CCF_3$ | CH | CH | |
| A-128 | Et | CHO | Me | H | 3-F | N | $CCF_3$ | CH | CH | |
| A-129 | $CH_2CCH$ | CHO | Me | H | 3-F | N | $CCF_3$ | CH | CH | |
| A-130 | $CHN(Me)_2$ | | Me | H | 3-F | N | $CCF_3$ | CH | CH | |
| A-131 | H | CHO | Me | H | 3-Cl | N | $CCF_3$ | N | CH | 75-79 |
| A-132 | Me | CHO | Me | H | 3-Cl | N | $CCF_3$ | N | CH | oil |
| A-133 | Et | CHO | Me | H | 3-Cl | N | $CCF_3$ | N | CH | oil |
| A-134 | $CH_2CCH$ | CHO | Me | H | 3-Cl | N | $CCF_3$ | N | CH | oil |
| A-135 | $CHN(Me)_2$ | | Me | H | 3-Cl | N | $CCF_3$ | N | CH | oil |
| A-136 | H | CHO | Me | H | 3-Cl | N | $CCF_3$ | CH | CH | oil |
| A-137 | Me | CHO | Me | H | 3-Cl | N | $CCF_3$ | CH | CH | oil |
| A-138 | Et | CHO | Me | H | 3-Cl | N | $CCF_3$ | CH | CH | oil |

(continued)

| | | | | | | | | | | |
|---|---|---|---|---|---|---|---|---|---|---|
| A-139 | $CH_2CCH$ | CHO | Me | H | 3-Cl | N | $CCF_3$ | CH | CH | oil |
| A-140 | $CHN(Me)_2$ | | Me | H | 3-Cl | N | $CCF_3$ | CH | CH | oil |

Table 1-8

| | | | | | | | | | | |
|---|---|---|---|---|---|---|---|---|---|---|
| A-141 | H | CHO | Me | H | 3-Br | N | $CCF_3$ | N | CH | oil |
| A-142 | Me | CHO | Me | H | 3-Br | N | $CCF_3$ | N | CH | oil |
| A-143 | Et | CHO | Me | H | 3-Br | N | $CCF_3$ | N | CH | oil |
| A-144 | $CH_2CCH$ | CHO | Me | H | 3-Br | N | $CCF_3$ | N | CH | oil |
| A-145 | $CHN(Me)_2$ | | Me | H | 3-Br | N | $CCF_3$ | N | CH | oil |
| A-146 | H | CHO | Me | H | 3-Br | N | $CCF_3$ | CH | CH | oil |
| A-147 | Me | CHO | Me | H | 3-Br | N | $CCF_3$ | CH | CH | oil |
| A-148 | Et | CHO | Me | H | 3-Br | N | $CCF_3$ | CH | CH | oil |
| A-149 | $CH_2CCH$ | CHO | Me | H | 3-Br | N | $CCF_3$ | CH | CH | oil |
| A-150 | $CHN(Me)_2$ | | Me | H | 3-Br | N | $CCF_3$ | CH | CH | oil |
| A-151 | H | CHO | Me | H | 2, 3-$F_2$ | N | $CCF_3$ | N | CH | oil |
| A-152 | Me | CHO | Me | H | 2, 3-$F_2$ | N | $CCF_3$ | N | CH | |
| A-153 | Et | CHO | Me | H | 2, 3-$F_2$ | N | $CCF_3$ | N | CH | |
| A-154 | $CH_2CCH$ | CHO | Me | H | 2, 3-$F_2$ | N | $CCF_3$ | N | CH | |
| A-155 | $CHN(Me)_2$ | | Me | H | 2, 3-$F_2$ | N | $CCF_3$ | N | CH | |
| A-156 | H | CHO | Me | H | 2, 3-$F_2$ | N | $CCF_3$ | CH | CH | |
| A-157 | Me | CHO | Me | H | 2, 3-$F_2$ | N | $CCF_3$ | CH | CH | |
| A-158 | Et | CHO | Me | H | 2, 3-$F_2$ | N | $CCF_3$ | CH | CH | |
| A-159 | $CH_2CCH$ | CHO | Me | H | 2, 3-$F_2$ | N | $CCF_3$ | CH | CH | |
| A-160 | $CHN(Me)_2$ | | Me | H | 2, 3-$F_2$ | N | $CCF_3$ | CH | CH | |

Table 1-9

| | | | | | | | | | | |
|---|---|---|---|---|---|---|---|---|---|---|
| A-161 | H | CHO | Me | H | 2, 4-$F_2$ | N | $CCF_3$ | N | CH | oil |
| A-162 | Me | CHO | Me | H | 2, 4-$F_2$ | N | $CCF_3$ | N | CH | |
| A-163 | Et | CHO | Me | H | 2, 4-$F_2$ | N | $CCF_3$ | N | CH | |
| A-164 | $CH_2CCH$ | CHO | Me | H | 2, 4-$F_2$ | N | $CCF_3$ | N | CH | |
| A-165 | $CHN(Me)_2$ | | Me | H | 2, 4-$F_2$ | N | $CCF_3$ | N | CH | |
| A-166 | H | CHO | Me | H | 2, 4-$F_2$ | N | $CCF_3$ | CH | CH | |
| A-167 | Me | CHO | Me | H | 2, 4-$F_2$ | N | $CCF_3$ | CH | CH | |
| A-168 | Et | CHO | Me | H | 2, 4-$F_2$ | N | $CCF_3$ | CH | CH | |
| A-169 | $CH_2CCH$ | CHO | Me | H | 2, 4-$F_2$ | N | $CCF_3$ | CH | CH | |
| A-170 | $CHN(Me)_2$ | | Me | H | 2, 4-$F_2$ | N | $CCF_3$ | CH | CH | |
| A-171 | H | CHO | Me | H | 2, 5-$F_2$ | N | $CCF_3$ | N | CH | oil |
| A-172 | Me | CHO | Me | H | 2, 5-$F_2$ | N | $CCF_3$ | N | CH | |
| A-173 | Et | CHO | Me | H | 2, 5-$F_2$ | N | $CCF_3$ | N | CH | |
| A-174 | $CH_2CCH$ | CHO | Me | H | 2, 5-$F_2$ | N | $CCF_3$ | N | CH | |
| A-175 | $CHN(Me)_2$ | | Me | H | 2, 5-$F_2$ | N | $CCF_3$ | N | CH | |

(continued)

| | | | | | | | | | | |
|---|---|---|---|---|---|---|---|---|---|---|
| A-176 | H | CHO | Me | H | 2, 5-$F_2$ | N | $CCF_3$ | CH | CH | oil |
| A-177 | Me | CHO | Me | H | 2, 5-$F_2$ | N | $CCF_3$ | CH | CH | oil |
| A-178 | Et | CHO | Me | H | 2, 5-$F_2$ | N | $CCF_3$ | CH | CH | oil |
| A-179 | $CH_2CCH$ | CHO | Me | H | 2, 5-$F_2$ | N | $CCF_3$ | CH | CH | oil |
| A-180 | $CHN(Me)_2$ | | Me | H | 2, 5-$F_2$ | N | $CCF_3$ | CH | CH | |

Table 1-10

| | | | | | | | | | | |
|---|---|---|---|---|---|---|---|---|---|---|
| A-181 | H | CHO | Me | H | 3, 4-$F_2$ | N | $CCF_3$ | N | CH | oil |
| A-182 | Me | CHO | Me | H | 3, 4-$F_2$ | N | $CCF_3$ | N | CH | oil |
| A-183 | Et | CHO | Me | H | 3, 4-$F_2$ | N | $CCF_3$ | N | CH | |
| A-184 | $CH_2CCH$ | CHO | Me | H | 3, 4-$F_2$ | N | $CCF_3$ | N | CH | oil |
| A-185 | $CH_2CH=CH_2$ | CHO | Me | H | 3, 4-$F_2$ | N | $CCF_3$ | N | CH | oil |
| A-186 | Bn | CHO | Me | H | 3, 4-$F_2$ | N | $CCF_3$ | N | CH | oil |
| A-187 | 4-Cl-Bn | CHO | Me | H | 3, 4-$F_2$ | N | $CCF_3$ | N | CH | oil |
| A-188 | $CHN(Me)_2$ | | Me | H | 3, 4-$F_2$ | N | $CCF_3$ | N | CH | |
| A-189 | H | CHO | Me | H | 3, 4-$F_2$ | N | $CCF_3$ | CH | CH | |
| A-190 | Me | CHO | Me | H | 3, 4-$F_2$ | N | $CCF_3$ | CH | CH | |
| A-191 | Et | CHO | Me | H | 3, 4-$F_2$ | N | $CCF_3$ | CH | CH | |
| A-192 | $CH_2CCH$ | CHO | Me | H | 3, 4-$F_2$ | N | $CCF_3$ | CH | CH | |
| A-193 | $CHN(Me)_2$ | | Me | H | 3, 4-$F_2$ | N | $CCF_3$ | CH | CH | |
| A-194 | H | CHO | Me | H | 3, 5-$F_2$ | N | $CCF_3$ | N | CH | oil |
| A-195 | Me | CHO | Me | H | 3,5-$F_2$ | N | $CCF_3$ | N | CH | oil |
| A-196 | Et | CHO | Me | H | 3, 5-$F_2$ | N | $CCF_3$ | N | CH | oil |
| A-197 | $CH_2CCH$ | CHO | Me | H | 3, 5-$F_2$ | N | $CCF_3$ | N | CH | oil |
| A-198 | $CHN(Me)_2$ | | Me | H | 3, 5-$F_2$ | N | $CCF_3$ | N | CH | |
| A-199 | H | CHO | Me | H | 3, 5-$F_2$ | N | $CCF_3$ | CH | CH | |
| A-200 | Me | CHO | Me | H | 3, 5-$F_2$ | N | $CCF_3$ | CH | CH | |

Table 1-11

| | | | | | | | | | | |
|---|---|---|---|---|---|---|---|---|---|---|
| A-201 | Et | CHO | Me | H | 3, 5-$F_2$ | N | $CCF_3$ | CH | CH | |
| A-202 | $CH_2CCH$ | CHO | Me | H | 3, 5-$F_2$ | N | $CCF_3$ | CH | CH | |
| A-203 | $CHN (Me)_2$ | | Me | H | 3, 5-$F_2$ | N | $CCF_3$ | CH | CH | |
| A-204 | H | CHO | Me | H | 2, 3-$Cl_2$ | N | $CCF_3$ | N | CH | |
| A-205 | Me | CHO | Me | H | 2, 3-$Cl_2$ | N | $CCF_3$ | N | CH | |
| A-206 | Et | CHO | Me | H | 2, 3-$Cl_2$ | N | $CCF_3$ | N | CH | |
| A-207 | $CH_2CCH$ | CHO | Me | H | 2, 3-$Cl_2$ | N | $CCF_3$ | N | CH | |
| A-208 | $CHN (Me)_2$ | | Me | H | 2, 3-$Cl_2$ | N | $CCF_3$ | N | CH | |
| A-209 | H | CHO | Me | H | 2, 3-$Cl_2$ | N | $CCF_3$ | CH | CH | |
| A-210 | Me | CHO | Me | H | 2, 3-$Cl_2$ | N | $CCF_3$ | CH | CH | |
| A-211 | Et | CHO | Me | H | 2, 3-$Cl_2$ | N | $CCF_3$ | CH | CH | |
| A-212 | $CH_2CCH$ | CHO | Me | H | 2, 3-$Cl_2$ | N | $CCF_3$ | CH | CH | |

(continued)

| | | | | | | | | | | |
|---|---|---|---|---|---|---|---|---|---|---|
| A-213 | CHN (Me)$_2$ | | Me | H | 2, 3-Cl$_2$ | N | CCF$_3$ | CH | CH | |
| A-214 | H | CHO | Me | H | 2, 4-Cl$_2$ | N | CCF$_3$ | N | CH | |
| A-215 | Me | CHO | Me | H | 2, 4-Cl$_2$ | N | CCF$_3$ | N | CH | |
| A-216 | Et | CHO | Me | H | 2, 4-Cl$_2$ | N | CCF$_3$ | N | CH | |
| A-217 | CH$_2$CCH | CHO | Me | H | 2, 4-Cl$_2$ | N | CCF$_3$ | N | CH | |
| A-218 | CHN(Me)$_2$ | | Me | H | 2, 4-Cl$_2$ | N | CCF$_3$ | N | CH | |
| A-219 | H | CHO | Me | H | 2, 4-Cl$_2$ | N | CCF$_3$ | CH | CH | |
| A-220 | Me | CHO | Me | H | 2, 4-Cl$_2$ | N | CCF$_3$ | CH | CH | |

Table 1-12

| | | | | | | | | | | |
|---|---|---|---|---|---|---|---|---|---|---|
| A-221 | Et | CHO | Me | H | 2, 4-Cl$_2$ | N | CCF$_3$ | CH | CH | |
| A-222 | CH$_2$CCH | CHO | Me | H | 2, 4-Cl$_2$ | N | CCF$_3$ | CH | CH | |
| A-223 | CHN (Me)$_2$ | | Me | H | 2, 4-Cl$_2$ | N | CCF$_3$ | CH | CH | |
| A-224 | H | CHO | Me | H | 2, 5-Cl$_2$ | N | CCF$_3$ | N | CH | |
| A-225 | Me | CHO | Me | H | 2, 5-Cl$_2$ | N | CCF$_3$ | N | CH | |
| A-226 | Et | CHO | Me | H | 2, 5-Cl$_2$ | N | CCF$_3$ | N | CH | |
| A-227 | CH$_2$CCH | CHO | Me | H | 2, 5-Cl$_2$ | N | CCF$_3$ | N | CH | |
| A-228 | CHN (Me) $_2$ | | Me | H | 2, 5-Cl$_2$ | N | CCF$_3$ | N | CH | |
| A-229 | H | CHO | Me | H | 2, 5-Cl$_2$ | N | CCF$_3$ | CH | CH | |
| A-230 | Me | CHO | Me | H | 2, 5-Cl2 | N | CCF$_3$ | CH | CH | |
| A-231 | Et | CHO | Me | H | 2, 5-Cl$_2$ | N | CCF$_3$ | CH | CH | |
| A-232 | CH$_2$CCH | CHO | Me | H | 2, 5-Cl$_2$ | N | CCF$_3$ | CH | CH | |
| A-233 | CHN(Me)$_2$ | | Me | H | 2, 5-Cl$_2$ | N | CCF$_3$ | CH | CH | |
| A-234 | H | CHO | Me | H | 3, 4-Cl$_2$ | N | CCF$_3$ | N | CH | oil |
| A-235 | Me | CHO | Me | H | 3, 4-Cl$_2$ | N | CCF$_3$ | N | CH | |
| A-236 | Et | CHO | Me | H | 3, 4-Cl$_2$ | N | CCF$_3$ | N | CH | |
| A-237 | CH$_2$CCH | CHO | Me | H | 3, 4-Cl$_2$ | N | CCF$_3$ | N | CH | oil |
| A-238 | CHN (Me)$_2$ | | Me | H | 3, 4-Cl$_2$ | N | CCF$_3$ | N | CH | oil |
| A-239 | H | CHO | Me | H | 3, 4-Cl$_2$ | N | CCF$_3$ | CH | CH | |
| A-240 | Me | CHO | Me | H | 3, 4-Cl$_2$ | N | CCF$_3$ | CH | CH | |

Table 1-13

| | | | | | | | | | | |
|---|---|---|---|---|---|---|---|---|---|---|
| A-241 | Et | CHO | Me | H | 3, 4-Cl$_2$ | N | CCF$_3$ | CH | CH | |
| A-242 | CH$_2$CCH | CHO | Me | H | 3, 4-Cl$_2$ | N | CCF$_3$ | CH | CH | |
| A-243 | CHN (Me)$_2$ | | Me | H | 3, 4-Cl$_2$ | N | CCF$_3$ | CH | CH | |
| A-244 | H | CHO | Me | H | 3, 5-Cl$_2$ | N | CCF$_3$ | N | CH | oil |
| A-245 | Me | CHO | Me | H | 3, 5-Cl$_2$ | N | CCF$_3$ | N | CH | |
| A-246 | Et | CHO | Me | H | 3, 5-Cl$_2$, | N | CCF$_3$ | N | CH | |
| A-247 | CH$_2$CCH | CHO | Me | H | 3, 5-Cl$_2$ | N | CCF$_3$ | N | CH | |
| A-248 | CHN (Me)$_2$ | | Me | H | 3, 5-Cl$_2$ | N | CCF$_3$ | N | CH | |
| A-249 | H | CHO | Me | H | 3, 5-Cl$_2$ | N | CCF$_3$ | CH | CH | |

(continued)

| | | | | | | | | | | |
|---|---|---|---|---|---|---|---|---|---|---|
| A-250 | Me | CHO | Me | H | 3, 5-$Cl_2$ | N | $CCF_3$ | CH | CH | |
| A-251 | Et | CHO | Me | H | 3, 5-$Cl_2$ | N | $CCF_3$ | CH | CH | |
| A-252 | $CH_2CCH$ | CHO | Me | H | 3, 5-$Cl_2$ | N | $CCF_3$ | CH | CH | |
| A-253 | CHN $(Me)_2$ | | Me | H | 3, 5-$Cl_2$ | N | $CCF_3$ | CH | CH | |
| A-254 | H | CHO | Me | H | 2, 3, 5-F 3 | N | $CCF_3$ | N | CH | oil |
| A-255 | Me | CHO | Me | H | 2, 3, 5-F 3 | N | $CCF_3$ | N | CH | |
| A-256 | Et | CHO | Me | H | 2, 3, 5-F 3 | N | $CCF_3$ | N | CH | |
| A-257 | $CH_2CCH$ | CHO | Me | H | 2, 3, 5-F 3 | N | $CCF_3$ | N | CH | |
| A-258 | CHN $(Me)_2$ | | Me | H | 2, 3, 5-F 3 | N | $CCF_3$ | N | CH | |
| A-259 | H | CHO | Me | H | 2, 3, 5-F 3 | N | $CCF_3$ | CH | CH | |
| A-260 | Me | CHO | Me | H | 2, 3, 5-F 3 | N | $CCF_3$ | CH | CH | |

Table 1-14

| | | | | | | | | | | |
|---|---|---|---|---|---|---|---|---|---|---|
| A-261 | Et | CHO | Me | H | 2, 3, 5-F 3 | N | $CCF_3$ | CH | CH | |
| A-262 | $CH_2CCH$ | CHO | Me | H | 2, 3, 5-F 3 | N | $CCF_3$ | CH | CH | |
| A-263 | CHN $(Me)_2$ | | Me | H | 2, 3, 5-F 3 | N | $CCF_3$ | CH | CH | |
| A-264 | H | CHO | Et | H | H | N | $CCF_3$ | CH | CH | oil |
| A-265 | Me | CHO | Et | H | H | N | $CCF_3$ | CH | CH | |
| A-266 | Et | CHO | Et | H | H | N | $CCF_3$ | CH | CH | |
| A-267 | $CH_2CCH$ | CHO | Et | H | H | N | $CCF_3$ | CH | CH | |
| A-268 | $CHNMe_2$ | | Et | H | H | N | $CCF_3$ | CH | CH | oil |
| A-269 | H | CHO | Me | Me | H | N | $CCF_3$ | N | CH | 107-109 |
| A-270 | Me | CHO | Me | Me | H | N | $CCF_3$ | N | CH | oil |
| A-271 | Et | CHO | Me | Me | H | N | $CCF_3$ | N | CH | oil |
| A-272 | $CH_2CCH$ | CHO | Me | Me | H | N | $CCF_3$ | N | CH | oil |
| A-273 | CHN $(Me)_2$ | | Me | Me | H | N | $CCF_3$ | N | CH | 116-118 |
| A-274 | H | CHO | Me | Me | H | N | $CCF_3$ | CH | CH | 99-100 |
| A-275 | Me | CHO | Me | Me | H | N | $CCF_3$ | CH | CH | oil |
| A-276 | Et | CHO | Me | Me | H | N | $CCF_3$ | CH | CH | oil |
| A-277 | $CH_2CCH$ | CHO | Me | Me | H | N | $CCF_3$ | CH | CH | oil |
| A-278 | CHN $(Me)_2$ | | Me | Me | H | N | $CCF_3$ | CH | CH | 100-102 |
| A-279 | $CH_2CCH$ | CHO | Me | Me | H | CH | $CCF_3$ | N | CH | |
| A-280 | CHN $(Me)_2$ | | Me | Me | H | CH | $CCF_3$ | N | CH | |

Table 1-15

| | | | | | | | | | | |
|---|---|---|---|---|---|---|---|---|---|---|
| A-281 | H | CHO | Me | Me | H | CH | $CCF_3$ | CH | N | |
| A-282 | CHN $(Me)_2$ | | Me | Me | H | CH | $CCF_3$ | CH | N | |
| A-283 | H | CHO | Me | Me | H | N | $CCF_3$ | N | N | |
| A-284 | CHN $(Me)_2$ | | Me | Me | H | N | $CCF_3$ | N | N | |
| A-285 | H | CHO | Me | Me | 4-F | N | $CCF_3$ | N | CH | 126-127 |
| A-286 | Me | CHO | Me | Me | 4-F | N | $CCF_3$ | N | CH | oil |

(continued)

| | | | | | | | | | | |
|---|---|---|---|---|---|---|---|---|---|---|
| A-287 | Et | CHO | Me | Me | 4-F | N | $CCF_3$ | N | CH | oil |
| A-288 | $CH_2CCH$ | CHO | Me | Me | 4-F | N | $CCF_3$ | N | CH | oil |
| A-289 | CHN $(Me)_2$ | | Me | Me | 4-F | N | $CCF_3$ | N | CH | 54-55 |
| A-290 | H | CHO | Me | Me | 4-F | N | $CCF_3$ | CH | CH | |
| A-291 | Me | CHO | Me | Me | 4-F | N | $CCF_3$ | CH | CH | |
| A-292 | Et | CHO | Me | Me | 4-F | N | $CCF_3$ | CH | CH | |
| A-293 | $CH_2CCH$ | CHO | Me | Me | 4-F | N | $CCF_3$ | CH | CH | |
| A-294 | CHN $(Me)_2$ | | Me | Me | 4-F | N | $CCF_3$ | CH | CH | |
| A-295 | H | CHO | Me | Me | 4-Cl | N | $CCF_3$ | N | CH | 120-121 |
| A-296 | Me | CHO | Me | Me | 4-Cl | N | $CCF_3$ | N | CH | oil |
| A-297 | Et | CHO | Me | Me | 4-Cl | N | $CCF_3$ | N | CH | oil |
| A-298 | $CH_2CCH$ | CHO | Me | Me | 4-Cl | N | $CCF_3$ | N | CH | oil |
| A-299 | CHN $(Me)_2$ | | Me | Me | 4-Cl | N | $CCF_3$ | N | CH | oil |
| A-300 | H | CHO | Me | Me | 4-Cl | N | $CCF_3$ | CH | CH | 92-94 |

Table 1-16

| | | | | | | | | | | |
|---|---|---|---|---|---|---|---|---|---|---|
| A-301 | Me | CHO | Me | Me | 4-Cl | N | $CCF_3$ | CH | CH | oil |
| A-302 | Et | CHO | Me | Me | 4-Cl | N | $CCF_3$ | CH | CH | oil |
| A-303 | $CH_2CCH$ | CHO | Me | Me | 4-Cl | N | $CCF_3$ | CH | CH | oil |
| A-304 | CHN $(Me)_2$ | | Me | Me | 4-Cl | N | $CCF_3$ | CH | CH | 73-75 |
| A-305 | H | CHO | Me | Me | 4-Br | N | $CCF_3$ | N | CH | 148-149 |
| A-306 | Me | CHO | Me | Me | 4-Br | N | $CCF_3$ | N | CH | |
| A-307 | Et | CHO | Me | Me | 4-Br | N | $CCF_3$ | N | CH | oil |
| A-308 | $CH_2CCH$ | CHO | Me | Me | 4-Br | N | $CCF_3$ | N | CH | oil |
| A-309 | CHN $(Me)_2$ | | Me | Me | 4-Br | N | $CCF_3$ | N | CH | oil |
| A-310 | H | CHO | Me | Me | 4-Br | N | $CCF_3$ | CH | CH | |
| A-311 | Me | CHO | Me | Me | 4-Br | N | $CCF_3$ | CH | CH | |
| A-312 | Et | CHO | Me | Me | 4-Br | N | $CCF_3$ | CH | CH | |
| A-313 | $CH_2CCH$ | CHO | Me | Me | 4-Br | N | $CCF_3$ | CH | CH | |
| A-314 | CHN $(Me)_2$ | | Me | Me | 4-Br | N | $CCF_3$ | CH | CH | |
| A-315 | H | CHO | Me | Me | 4-$CF_3$ | N | $CCF_3$ | N | CH | |
| A-316 | Me | CHO | Me | Me | 4-$CF_3$ | N | $CCF_3$ | N | CH | |
| A-317 | Et | CHO | Me | Me | 4-$CF_3$ | N | $CCF_3$ | N | CH | |
| A-318 | $CH_2CCH$ | CHO | Me | Me | 4-$CF_3$ | N | $CCF_3$ | N | CH | |
| A-319 | CHN $(Me)_2$ | | Me | Me | 4-$CF_3$ | N | $CCF_3$ | N | CH | |
| A-320 | H | CHO | Me | Me | 4-$CF_3$ | N | $CCF_3$ | CH | CH | |

Table 1-17

| | | | | | | | | | | |
|---|---|---|---|---|---|---|---|---|---|---|
| A-321 | Me | CHO | Me | Me | 4-$CF_3$ | N | $CCF_3$ | CH | CH | |
| A-322 | Et | CHO | Me | Me | 4-$CF_3$ | N | $CCF_3$ | CH | CH | |
| A-323 | $CH_2CCH$ | CHO | Me | Me | 4-$CF_3$ | N | $CCF_3$ | CH | CH | |

(continued)

| | | | | | | | | | | |
|---|---|---|---|---|---|---|---|---|---|---|
| A-324 | CHN $(Me)_2$ | | Me | Me | 4-$CF_3$ | N | $CCF_3$ | CH | CH | |
| A-325 | H | CHO | Me | Me | 4-I | N | $CCF_3$ | N | CH | |
| A-326 | CHN (Me) 2 | | Me | Me | 4-I | N | $CCF_3$ | N | CH | |
| A-327 | H | CHO | Me | Me | 4-I | N | $CCF_3$ | CH | CH | |
| A-328 | CHN $(Me)_2$ | | Me | Me | 4-I | N | $CCF_3$ | CH | CH | |
| A-329 | H | CHO | Me | Me | 4-Me | N | $CCF_3$ | N | CH | |
| A-330 | CHN $(Me)_2$ | | Me | Me | 4-Me | N | $CCF_3$ | N | CH | |
| A-331 | H | CHO | Me | Me | 4-Me | N | $CCF_3$ | CH | CH | |
| A-332 | CHN $(Me)_2$ | | Me | Me | 4-Me | N | $CCF_3$ | CH | CH | |
| A-333 | H | CHO | Me | Me | 4-CN | N | $CCF_3$ | N | CH | |
| A-334 | CHN $(Me)_2$ | | Me | Me | 4-CN | N | $CCF_3$ | N | CH | |
| A-335 | H | CHO | Me | Me | 4-CN | N | $CCF_3$ | CH | CH | |
| A-336 | CHN $(Me)_2$ | | Me | Me | 4-CN | N | $CCF_3$ | CH | CH | |
| A-337 | H | CHO | Me | Me | 4-$NO_2$ | N | $CCF_3$ | N | CH | |
| A-338 | CHN $(Me)_2$ | | Me | Me | 4-$NO_2$ | N | $CCF_3$ | N | CH | |
| A-339 | H | CHO | Me | Me | 4-$NO_2$ | N | $CCF_3$ | CH | CH | |
| A-340 | CHN $(Me)_2$ | | Me | Me | 4-$NO_2$ | N | $CCF_3$ | CH | CH | |

Table 1-18

| | | | | | | | | | | |
|---|---|---|---|---|---|---|---|---|---|---|
| A-341 | H | CHO | Me | Me | 2-F | N | $CCF_3$ | N | CH | 128-130 |
| A-342 | Me | CHO | Me | Me | 2-F | N | $CCF_3$ | N | CH | oil |
| A-343 | Et | CHO | Me | Me | 2-F | N | $CCF_3$ | N | CH | oil |
| A-344 | $CH_2CCH$ | CHO | Me | Me | 2-F | N | $CCF_3$ | N | CH | oil |
| A-345 | CHN $(Me)_2$ | | Me | Me | 2-F | N | $CCF_3$ | N | CH | 88-92 |
| A-346 | H | CHO | Me | Me | 2-F | N | $CCF_3$ | CH | CH | 119-120 |
| A-347 | Me | CHO | Me | Me | 2-F | N | $CCF_3$ | CH | CH | oil |
| A-348 | Et | CHO | Me | Me | 2-F | N | $CCF_3$ | CH | CH | oil |
| A-349 | $CH_2CCH$ | CHO | Me | Me | 2-F | N | $CCF_3$ | CH | CH | oil |
| A-350 | CHN $(Me)_2$ | | Me | Me | 2-F | N | $CCF_3$ | CH | CH | oil |
| A-351 | H | CHO | Me | Me | 2-Cl | N | $CCF_3$ | N | CH | oil |
| A-352 | Me | CHO | Me | Me | 2-Cl | N | $CCF_3$ | N | CH | |
| A-353 | Et | CHO | Me | Me | 2-Cl | N | $CCF_3$ | N | CH | |
| A-354 | $CH_2CCH$ | CHO | Me | Me | 2-Cl | N | $CCF_3$ | N | CH | |
| A-355 | CHN $(Me)_2$ | | Me | Me | 2-Cl | N | $CCF_3$ | N | CH | |
| A-356 | H | CHO | Me | Me | 2-Cl | N | $CCF_3$ | CH | CH | |
| A-357 | Me | CHO | Me | Me | 2-Cl | N | $CCF_3$ | CH | CH | |
| A-358 | Et | CHO | Me | Me | 2-Cl | N | $CCF_3$ | CH | CH | |
| A-359 | $CH_2CCH$ | CHO | Me | Me | 2-Cl | N | $CCF_3$ | CH | CH | |
| A-360 | CHN $(Me)_2$ | | Me | Me | 2-Cl | N | $CCF_3$ | CH | CH | |

Table 1-19

| | | | | | | | | | | |
|---|---|---|---|---|---|---|---|---|---|---|
| A-361 | H | CHO | Me | Me | 2-Br | N | $CCF_3$ | N | CH | |
| A-362 | Me | CHO | Me | Me | 2-Br | N | $CCF_3$ | N | CH | |
| A-363 | Et | CHO | Me | Me | 2-Br | N | $CCF_3$ | N | CH | |
| A-364 | $CH_2CCH$ | CHO | Me | Me | 2-Br | N | $CCF_3$ | N | CH | |
| A-365 | CHN $(Me)_2$ | | Me | Me | 2-Br | N | $CCF_3$ | N | CH | |
| A-366 | H | CHO | Me | Me | 2-Br | N | $CCF_3$ | CH | CH | |
| A-367 | Me | CHO | Me | Me | 2-Br | N | $CCF_3$ | CH | CH | |
| A-368 | Et | CHO | Me | Me | 2-Br | N | $CCF_3$ | CH | CH | |
| A-369 | $CH_2CCH$ | CHO | Me | Me | 2-Br | N | $CCF_3$ | CH | CH | |
| A-370 | CHN $(Me)_2$ | | Me | Me | 2-Br | N | $CCF_3$ | CH | CH | |
| A-371 | H | CHO | Me | Me | 3-F | N | $CCF_3$ | N | CH | 111-112 |
| A-372 | Me | CHO | Me | Me | 3-F | N | $CCF_3$ | N | CH | oil I |
| A-373 | Et | CHO | Me | Me | 3-F | N | $CCF_3$ | N | CH | oil |
| A-374 | $CH_2CCH$ | CHO | Me | Me | 3-F | N | $CCF_3$ | N | CH | oil |
| A-375 | CHN $(Me)_2$ | | Me | Me | 3-F | N | $CCF_3$ | N | CH | 104-105 |
| A-376 | H | CHO | Me | Me | 3-F | N | $CCF_3$ | CH | CH | 131-132 |
| A-377 | Me | CHO | Me | Me | 3-F | N | $CCF_3$ | CH | CH | oil |
| A-378 | Et | CHO | Me | Me | 3-F | N | $CCF_3$ | CH | CH | oil |
| A-379 | $CH_2CCH$ | CHO | Me | Me | 3-F | N | $CCF_3$ | CH | CH | oil |
| A-380 | CHN $(Me)_2$ | | Me | Me | 3-F | N | $CCF_3$ | CH | CH | 82-85 |

Table 1-20

| | | | | | | | | | | |
|---|---|---|---|---|---|---|---|---|---|---|
| A-381 | H | CHO | Me | Me | 3-Cl | N | $CCF_3$ | | CH | 139-141 |
| A-382 | Me | CHO | Me | Me | 3-Cl | N | $CCF_3$ | N | CH | oil |
| A-383 | Et | CHO | Me | Me | 3-Cl | N | $CCF_3$ | N | CH | oil |
| A-384 | $CH_2CCH$ | CHO | Me | Me | 3-Cl | N | $CCF_3$ | N | CH | oil |
| A-385 | CHN $(Me)_2$ | | Me | Me | 3-Cl | N | $CCF_3$ | N | CH | oil |
| A-386 | H | CHO | Me | Me | 3-Cl | N | $CCF_3$ | CH | CH | 132-134 |
| A-387 | Me | CHO | Me | Me | 3-Cl | N | $CCF_3$ | CH | CH | oil |
| A-388 | Et | CHO . | Me | Me | 3-Cl | N | $CCF_3$ | CH | CH | oil I |
| A-389 | $CH_2CCH$ | CHO | Me | Me | 3-Cl | N | $CCF_3$ | CH | CH | oil |
| A-390 | CHN $(Me)_2$ | | Me | Me | 3-Cl | N | $CCF_3$ | CH | CH | 86-91 |
| A-391 | H | CHO | Me | Me | 3-Br | N | $CCF_3$ | N | CH | oil |
| A-392 | Me | CHO | Me | Me | 3-Br | N | $CCF_3$ | N | CH | oil |
| A-393 | Et | CHO | Me | Me | 3-Br | N | $CCF_3$ | N | CH | oil |
| A-394 | $CH_2CCH$ | CHO | Me | Me | 3-Br | N | $CCF_3$ | N | CH | oil |
| A-395 | CHN $(Me)_2$ | | Me | Me | 3-Br | N | $CCF_3$ | N | CH | oil |
| A-396 | H | CHO | Me | Me | 3-Br | N | $CCF_3$ | CH | CH | 98-104 |
| A-397 | Me | CHO | Me | Me | 3-Br | N | $CCF_3$ | CH | CH | oil |
| A-398 | Et | CHO | Me | Me | 3-Br | N | $CCF_3$ | CH | CH | oil |

(continued)

| | | | | | | | | | | |
|---|---|---|---|---|---|---|---|---|---|---|
| A-399 | $CH_2CCH$ | CHO | Me | Me | 3-Br | N | $CCF_3$ | CH | CH | oil |
| A-400 | CHN $(Me)_2$ | | Me | Me | 3-Br | N | $CCF_3$ | CH | CH | 93-94 |

Table 1-21

| | | | | | | | | | | |
|---|---|---|---|---|---|---|---|---|---|---|
| **A-401** | **H** | **CHO** | **Me** | **Me** | **2, 3-$F_2$** | **N** | **$CCF_3$** | **N** | **CH** | |
| **A-402** | **Me** | **CHO** | **Me** | **Me** | **2, 3-$F_2$** | **N** | **$CCF_3$** | **N** | **CH** | |
| **A-403** | **Et** | **CHO** | **Me** | **Me** | **2, 3-$F_2$** | **N** | **$CCF_3$** | **N** | **CH** | |
| **A-404** | **$CH_2CCH$** | **CHO** | **Me** | **Me** | **2, 3-$F_2$** | **N** | **$CCF_3$** | **N** | **CH** | |
| **A-405** | **CHN $(Me)_2$** | | **Me** | **Me** | **2, 3-$F_2$** | **N** | **$CCF_3$** | **N** | **CH** | |
| **A-406** | **H** | **CHO** | **Me** | **Me** | **2, 3-$F_2$** | **N** | **$CCF_3$** | **CH** | **CH** | |
| **A-407** | **Me** | **CHO** | **Me** | **Me** | **2, 3-$F_2$** | **N** | **$CCF_3$** | **CH** | **CH** | |
| **A-408** | **Et** | **CHO** | **Me** | **Me** | **2, 3-$F_2$** | **N** | **$CCF_3$** | **CH** | **CH** | |
| **A-409** | **$CH_2CCH$** | **CHO** | **Me** | **Me** | **2, 3-$F_2$** | **N** | **$CCF_3$** | **CH** | **CH** | |
| **A-410** | **CHN $(Me)_2$** | | **Me** | **Me** | **2, 3-$F_2$** | **N** | **$CCF_3$** | **CH** | **CH** | |
| **A-411** | **H** | **CHO** | **Me** | **Me** | **2, 4-$F_2$** | **N** | **$CCF_3$** | **N** | **CH** | **128-130** |
| **A-412** | **Me** | **CHO** | **Me** | **Me** | **2, 4-$F_2$** | **N** | **$CCF_3$** | **N** | **CH** | **oil** |
| **A-413** | **Et** | **CHO** | **Me** | **Me** | **2, 4-$F_2$** | **N** | **$CCF_3$** | **N** | **CH** | |
| **A-414** | **$CH_2CCH$** | **CHO** | **Me** | **Me** | **2, 4-$F_2$** | **N** | **$CCF_3$** | **N** | **CH** | **oil** |
| **A-415** | **CHN $(Me)_2$** | | **Me** | **Me** | **2, 4-$F_2$** | **N** | **$CCF_3$** | **N** | **CH** | |
| **A-416** | **H** | **CHO** | **Me** | **Me** | **2, 4-$F_2$** | **N** | **$CCF_3$** | **CH** | **CH** | |
| **A-417** | **Me** | **CHO** | **Me** | **Me** | **2, 4-$F_2$** | **N** | **$CCF_3$** | **CH** | **CH** | |
| **A-418** | **Et** | **CHO** | **Me** | **Me** | **2, 4-$F_2$** | **N** | **$CCF_3$** | **CH** | **CH** | |
| **A-419** | **$CH_2CCH$** | **CHO** | **Me** | **Me** | **2, 4-$F_2$** | **N** | **$CCF_3$** | **CH** | **CH** | |
| **A-420** | **CHN $(Me)_2$** | | **Me** | **Me** | **2, 4-$F_2$** | **N** | **$CCF_3$** | **CH** | **CH** | |

Table 1-22

| | | | | | | | | | | |
|---|---|---|---|---|---|---|---|---|---|---|
| **A-421** | **H** | **CHO** | **Me** | **Me** | **2, 5-$F_2$** | **N** | **$CCF_3$** | **N** | **CH** | **106-109** |
| **A-422** | **Me** | **CHO** | **Me** | **Me** | **2, 5-$F_2$** | **N** | **$CCF_3$** | **N** | **CH** | |
| **A-423** | **Et** | **CHO** | **Me** | **Me** | **2, 5-$F_2$** | **N** | **$CCF_3$** | **N** | **CH** | |
| **A-424** | **$CH_2CCH$** | **CHO** | **Me** | **Me** | **2, 5-$F_2$** | **N** | **$CCF_3$** | **N** | **CH** | |
| **A-425** | **CHN $(Me)_2$** | | **Me** | **Me** | **2, 5-$F_2$** | **N** | **$CCF_3$** | **N** | **CH** | |
| **A-426** | **H** | **CHO** | **Me** | **Me** | **2, 5-$F_2$** | **N** | **$CCF_3$** | **CH** | **CH** | |
| **A-427** | **Me** | **CHO** | **Me** | **Me** | **2, 5-$F_2$** | **N** | **$CCF_3$** | **CH** | **CH** | |
| **A-428** | **Et** | **CHO** | **Me** | **Me** | **2, 5-$F_2$** | **N** | **$CCF_3$** | **CH** | **CH** | |
| **A-429** | **$CH_2CCH$** | **CHO** | **Me** | **Me** | **2, 5-$F_2$** | **N** | **$CCF_3$** | **CH** | **CH** | |
| **A-430** | **CHN $(Me)_2$** | | **Me** | **Me** | **2, 5-$F_2$** | **N** | **$CCF_3$** | **CH** | **CH** | |
| **A-431** | **H** | **CHO** | **Me** | **Me** | **3, 4-$F_2$** | **N** | **$CCF_3$** | **N** | **CH** | **128-130** |
| **A-432** | **Me** | **CHO** | **Me** | **Me** | **3, 4-$F_2$** | **N** | **$CCF_3$** | **N** | **CH** | |
| **A-433** | **Et** | **CHO** | **Me** | **Me** | **3, 4-$F_2$** | **N** | **$CCF_3$** | **N** | **CH** | |
| **A-434** | **$CH_2CCH$** | **CHO** | **Me** | **Me** | **3, 4-$F_2$** | **N** | **$CCF_3$** | **N** | **CH** | |
| **A-435** | **CHN $(Me)_2$** | | **Me** | **Me** | **3, 4-$F_2$** | **N** | **$CCF_3$** | **N** | **CH** | |

(continued)

| | | | | | | | | | | |
|---|---|---|---|---|---|---|---|---|---|---|
| A-436 | H | CHO | Me | Me | 3, 4-$F_2$ | N | $CCF_3$ | CH | CH | |
| A-437 | Me | CHO | Me | Me | 3, 4-$F_2$ | N | $CCF_3$ | CH | CH | |
| A-438 | Et | CHO | Me | Me | 3, 4-$F_2$ | N | $CCF_3$ | CH | CH | |
| A-439 | $CH_2CCH$ | CHO | Me | Me | 3, 4-$F_2$ | N | $CCF_3$ | CH | CH | |
| A-440 | CHN $(Me)_2$ | | Me | Me | 3, 4-F2 | N | $CCF_3$ | CH | CH | |

Table 1-23

| | | | | | | | | | | |
|---|---|---|---|---|---|---|---|---|---|---|
| A-441 | H | CHO | Me | Me | 3, 5-$F_2$ | N | $CCF_3$ | N | CH | solid |
| A-442 | Me | CHO | Me | Me | 3, 5-$F_2$ | N | $CCF_3$ | N | CH | o i l |
| A-443 | Et | CHO | Me | Me | 3, 5-$F_2$ | N | $CCF_3$ | N | CH | |
| A-444 | $CH_2CCH$ | CHO | Me | Me | 3, 5-$F_2$ | N | $CCF_3$ | N | CH | |
| A-445 | CHN $(Me)_2$ | | Me | Me | 3, 5-$F_2$ | N | $CCF_3$ | N | CH | |
| A-446 | H | CHO | Me | Me | 3, 5-$F_2$ | N | $CCF_3$ | CH | CH | |
| A-447 | Me | CHO | Me | Me | 3, 5-$F_2$ | N | $CCF_3$ | CH | CH | |
| A-448 | Et | CHO | Me | Me | 3, 5-$F_2$ | N | $CCF_3$ | CH | CH | |
| A-449 | $CH_2CCH$ | CHO | Me | Me | 3, 5-$F_2$ | N | $CCF_3$ | CH | CH | |
| A-450 | CHN $(Me)_2$ | | Me | Me | 3, 5-$F_2$ | N | $CCF_3$ | CH | CH | |
| A-451 | H | CHO | Me | Me | 2, 3-$Cl_2$ | N | $CCF_3$ | N | CH | |
| A-452 | Me | CHO | Me | Me | 2, 3-$Cl_2$ | N | $CCF_3$ | N | CH | |
| A-453 | Et | CHO | Me | Me | 2, 3-$Cl_2$ | N | $CCF_3$ | N | CH | |
| A-454 | $CH_2CCH$ | CHO | Me | Me | 2, 3-$Cl_2$ | N | $CCF_3$ | N | CH | |
| A-455 | CHN $(Me)_2$ | | Me | Me | 2, 3-$Cl_2$ | N | $CCF_3$ | N | CH | |
| A-456 | H | CHO | Me | Me | 2, 3-$Cl_2$ | N | $CCF_3$ | CH | CH | |
| A-457 | Me | CHO | Me | Me | 2, 3-$Cl_2$ | N | $CCF_3$ | CH | CH | |
| A-458 | Et | CHO | Me | Me | 2,3-$Cl_2$ | N | $CCF_3$ | CH | CH | |
| A-459 | $CH_2CCH$ | CHO | Me | Me | 2, 3-$Cl_2$ | N | $CCF_3$ | CH | CH | |
| A-460 | CHN $(Me)_2$ | | Me | Me | 2, 3-$Cl_2$ | N | $CCF_3$ | CH | CH | |

Table 1-24

| | | | | | | | | | | |
|---|---|---|---|---|---|---|---|---|---|---|
| A-461 | H | CHO | Me | Me | 2, 4-$Cl_2$ | N | $CCF_3$ | N | CH | |
| A-462 | Me | CHO | Me | Me | 2, 4-$Cl_2$ | N | $CCF_3$ | N | CH | |
| A-463 | Et | CHO | Me | Me | 2, 4-$Cl_2$ | N | $CCF_3$ | N | CH | |
| A-464 | $CH_2CCH$ | CHO | Me | Me | 2, 4-$Cl_2$ | N | $CCF_3$ | N | CH | |
| A-465 | CHN $(Me)_2$ | | Me | Me | 2, 4-$Cl_2$ | N | $CCF_3$ | N | CH | |
| A-466 | H | CHO | Me | Me | 2, 4-$Cl_2$ | N | $CCF_3$ | CH | CH | |
| A-467 | Me | CHO | Me | Me | 2, 4-$Cl_2$ | N | $CCF_3$ | CH | CH | |
| A-468 | Et | CHO | Me | Me | 2, 4-$Cl_2$ | N | $CCF_3$ | CH | CH | |
| A-469 | $CH_2CCH$ | CHO | Me | Me | 2, 4-$Cl_2$ | N | $CCF_3$ | CH | CH | |
| A-470 | CHN $(Me)_2$ | | Me | Me | 2, 4-$Cl_2$ | N | $CCF_3$ | CH | CH | |
| A-471 | H | CHO | Me | Me | 2, 5-$Cl_2$ | N | $CCF_3$ | N | CH | |
| A-472 | Me | CHO | Me | Me | 2, 5-$Cl_2$ | N | $CCF_3$ | N | CH | |

(continued)

| | | | | | | | | | | |
|---|---|---|---|---|---|---|---|---|---|---|
| A-473 | Et | CHO | Me | Me | 2, 5-$Cl_2$ | N | $CCF_3$ | N | CH | |
| A-474 | $CH_2CCH$ | CHO | Me | Me | 2, 5-$Cl_2$ | N | $CCF_3$ | N | CH | |
| A-475 | CHN $(Me)_2$ | | Me | Me | 2, 5-$Cl_2$ | N | $CCF_3$ | N | CH | |
| A-476 | H | CHO | Me | Me | 2, 5-$Cl_2$ | N | $CCF_3$ | CH | CH | |
| A-477 | Me | CHO | Me | Me | 2, 5-$Cl_2$ | N | $CCF_3$ | CH | CH | |
| A-478 | Et | CHO | Me | Me | 2, 5-$Cl_2$ | N | $CCF_3$ | CH | CH | |
| A-479 | $CH_2CCH$ | CHO | Me | Me | 2, 5-$Cl_2$ | N | $CCF_3$ | CH | CH | |
| A-480 | CHN $(Me)_2$ | | Me | Me | 2, 5-$Cl_2$ | N | $CCF_3$ | CH | CH | |

Table 1-25

| | | | | | | | | | | |
|---|---|---|---|---|---|---|---|---|---|---|
| A-481 | H | CHO | Me | Me | 3, 4-$Cl_2$ | N | $CCF_3$ | N | CH | |
| A-482 | Me | CHO | Me | Me | 3, 4-$Cl_2$ | N | $CCF_3$ | N | CH | |
| A-483 | Et | CHO | Me | Me | 3, 4-$Cl_2$ | N | $CCF_3$ | N | CH | |
| A-484 | $CH_2CCH$ | CHO | Me | Me | 3, 4-$Cl_2$ | N | $CCF_3$ | N | CH | |
| A-485 | CHN $(Me)_2$ | | Me | Me | 3, 4-$Cl_2$ | N | $CCF_3$ | N | CH | |
| A-486 | H | CHO | Me | Me | 3, 4-$Cl_2$ | N | $CCF_3$ | CH | CH | |
| A-487 | Me | CHO | Me | Me | 3, 4-$Cl_2$ | N | $CCF_3$ | CH | CH | |
| A-488 | Et | CHO | Me | Me | 3, 4-$Cl_2$ | N | $CCF_3$ | CH | CH | |
| A-489 | $CH_2CCH$ | CHO | Me | Me | 3, 4-$Cl_2$ | N | $CCF_3$ | CH | CH | |
| A-490 | CHN $(Me)_2$ | | Me | Me | 3, 4-$Cl_2$ | N | $CCF_3$ | CH | CH | |
| A-491 | H | CHO | Me | Me | 3, 5-$Cl_2$ | N | $CCF_3$ | N | CH | |
| A-492 | Me | CHO | Me | Me | 3, 5-$Cl_2$ | N | $CCF_3$ | N | CH | |
| A-493 | Et | CHO | Me | Me | 3, 5-$Cl_2$ | N | $CCF_3$ | N | CH | |
| A-494 | $CH_2CCH$ | CHO | Me | Me | 3, 5-$Cl_2$ | N | $CCF_3$ | N | CH | |
| A-495 | CHN $(Me)_2$ | | Me | Me | 3,5-$Cl_2$ | N | $CCF_3$ | N | CH | |
| A-496 | H | CHO | Me | Me | 3, 5-$Cl_2$ | N | $CCF_3$ | CH | CH | |
| A-497 | Me | CHO | Me | Me | 3, 5-$Cl_2$ | N | $CCF_3$ | CH | CH | |
| A-498 | Et | CHO | Me | Me | 3, 5-$Cl_2$ | N | $CCF_3$ | CH | CH | |
| A-499 | $CH_2CCH$ | CHO | Me | Me | 3, 5-$Cl_2$ | N | $CCF_3$ | CH | CH | |
| A-500 | CHN $(Me)_2$ | | Me | Me | 3, 5-$Cl_2$ | N | $CCF_3$ | CH | CH | |

Table 1-26

| | | | | | | | | | | |
|---|---|---|---|---|---|---|---|---|---|---|
| A-501 | H | CHO | 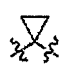 | | 3, 5-$Cl_2$ | N | $CCF_3$ | N | CH | |
| A-502 | H | CHO | Et | H | H | N | $CCF_3$ | N | CH | oil |
| A-503 | Me | CHO | Et | H | H | N | $CCF_3$ | N | CH | oil |
| A-504 | Et | CHO | Et | H | H | N | $CCF_3$ | N | CH | oil |
| A-505 | $CH_2CCH$ | CHO | Et | H | H | N | $CCF_3$ | N | CH | oil |
| A-506 | CHN $(Me)_2$ | | Et | H | H | N | $CCF_3$ | N | CH | oil |
| A-507 | H | CHO | i-Pr | H | H | N | $CCF_3$ | N | CH | oil |
| A-508 | H | CHO | Me | H | 3-$CF_3$ | N | $CCF_3$ | N | CH | oil |

(continued)

| | | | | | | | | | | |
|---|---|---|---|---|---|---|---|---|---|---|
| A-509 | Me | CHO | Me | H | 3-$CF_3$ | N | $CCF_3$ | N | CH | oil |
| A-510 | Et | CHO | Me | H | 3-$CF_3$ | N | $CCF_3$ | N | CH | oil |
| A-511 | $CH_2CCH$ | CHO | Me | H | 3-$CF_3$ | N | $CCF_3$ | N | CH | oil |
| A-512 | Me | CHO | Me | H | 3-Cl | CH | $CCF_3$ | CH | N | oil |
| A-513 | Et | CHO | Me | H | 3-Cl | CH | $CCF_3$ | CH | N | oil |
| A-514 | $CH_2CCH$ | CHO | Me | H | 3-Cl | CH | $CCF_3$ | CH | N | oil |
| A-515 | Me | CHO | Me | H | 3-Cl | CH | $CCF_3$ | N | CH | oil |
| A-516 | Et | CHO | Me | H | 3-Cl | CH | $CCF_3$ | N | CH | oil |
| A-517 | $CH_2CCH$ | CHO | Me | H | 3-Cl | CH | $CCF_3$ | N | CH | oil |
| A-518 | $CH_2CCH$ | CHO | Me | H | 4-Me | N | $CCF_3$ | N | CH | oil |
| A-519 | H | CHO | Me | H | 3-Me | N | $CCF_3$ | N | CH | oil |
| A-520 | Me | CHO | Me | H | 3-Me | N | $CCF_3$ | N | CH | oil |

Table 1-27

| | | | | | | | | | | |
|---|---|---|---|---|---|---|---|---|---|---|
| A-521 | Et | CHO | Me | H | 3-Me | N | $CCF_3$ | N | CH | oil |
| A-522 | $CH_2CCH$ | CHO | Me | H | 3-Me | N | $CCF_3$ | N | CH | oil |
| A-523 | H | CHO | Me | H | 3-$NO_2$ | N | $CCF_3$ | N | CH | oil |
| A-524 | CHOMe | | Me | H | H | N | $CCF_3$ | N | CH | oil |
| A-525 | CHOEt | | Me | H | H | N | $CCF_3$ | N | CH | oil |
| A-526 | H | CHO | Me | H | 3-Cl | CH | $CCF_3$ | CH | N | oil |
| A-527 | H | CHO | Me | H | 3-Cl | CH | $CCF_3$ | N | CH | oil |
| A-528 | H | CHO | Me | H | 4-Cl | N | $CCF_3$ | CH | CCF 3 | oil |
| A-529 | H | CHO | Me | H | 4-Cl | N | $CCF_3$ | CH | CCH 3 | oil |
| A-530 | $CH_2CCH$ | CHO | Me | H | 4-Cl | N | $CCF_3$ | CH | CCH 3 | oil |
| A-531 | Me | CHO | Me | H | 4-Cl | N | $CCF_3$ | CH | CCH 3 | oil |
| A-532 | H | CHO | Me | H | 4-Cl | N | CH | CBr | CH | oil |
| A-533 | H | CHO | Me | H | 4-Cl | N | CH | CCl | CH | oil |
| A-534 | Me | CHO | Me | H | 4-Cl | N | CH | CBr | CH | oil |
| A-535 | $CH_2CCH$ | CHO | Me | H | 4-Cl | N | CH | CBr | CH | oil |
| A-536 | Me | CHO | Me | H | 4-Cl | N | CH | CCl | CH | oil |
| A-537 | $CH_2CCH$ | CHO | Me | H | 4-Cl | N | CH | CCl | CH | oil |

General Formula (2)

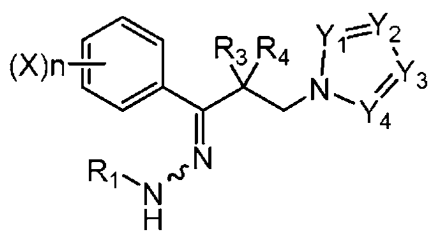

(2)

Table 2

| Compound No. | $R_1$ | $R_3$ | $R_4$ | X | $Y_1$ | $Y_2$ | $Y_3$ | $Y_4$ | State, Melting Point(°C) |
|---|---|---|---|---|---|---|---|---|---|
| B-1 | H | Me | H | H | N | $CCF_3$ | N | CH | |
| B-2 | H | Me | H | 4-F | N | $CCF_3$ | N | CH | |
| B-3 | H | Me | H | 4-F | N | $CCF_3$ | CH | CH | |
| B-4 | H | Me | H | 4-Cl | N | $CCF_3$ | N | CH | |
| B-5 | H | Me | H | 4-Br | N | $CCF_3$ | N | CH | |
| B-6 | H | Me | H | 4-Br | N | $CCF_3$ | CH | CH | |
| B-7 | H | Me | H | 2-F | N | $CCF_3$ | N | CH | |
| B-8 | H | Me | H | 2-Cl | N | $CCF_3$ | N | CH | |
| B-9 | H | Me | H | 2-Cl | N | $CCF_3$ | CH | CH | |
| B.10 | H | Me | H | 3-F | N | $CCF_3$ | N | CH | |
| B-11 | H | Me | H | 3-Cl | N | $CCF_3$ | N | CH | |
| B-12 | H | Me | H | 3-Cl | N | $CCF_3$ | CH | CH | |
| B-13 | H | Me | H | 3-Br | N | $CCF_3$ | N | CH | |
| B-14 | H | Me | H | 3-Br | N | $CCF_3$ | CH | CH | |
| B-15 | H | Me | H | 2,3-$F_2$ | N | $CCF_3$ | N | CH | |
| B-16 | H | Me | H | 2,4-$F_2$ | N | $CCF_3$ | N | CH | |
| B.17 | H | Me | H | 2,5-$F_2$ | N | $CCF_3$ | CH | CH | |
| B-18 | H | Me | H | 3,4-$F_2$ | N | $CCF_3$ | N | CH | |
| B-19 | H | Me | H | 3,4-$Cl_2$ | N | $CCF_3$ | N | CH | |
| B-20 | H | Me | H | 3,4-$Cl_2$ | N | $CCF_3$ | CH | CH | |
| B-21 | H | Et | H | H | N | $CCF_3$ | N | CH | |
| B-22 | H | Et | H | H | N | $CCF_3$ | CH | CH | |
| B-23 | H | Me | Me | H | N | $CCF_3$ | N | CH | |
| B-24 | H | Me | Me | H | N | $CCF_3$ | CH | CH | |
| B-25 | H | Me | Me | 4-F | N | $CCF_3$ | N | CH | |
| B-26 | H | Me | Me | 4-Cl | N | $CCF_3$ | N | CH | |
| B-27 | H | Me | Me | 4-Cl | N | $CCF_3$ | CH | CH | |
| B-28 | H | Me | Me | 4-Br | N | $CCF_3$ | N | CH | |
| B-29 | H | Me | Me | 4-Br | N | $CCF_3$ | CH | CH | |
| B-30 | H | Me | Me | 2-F | N | $CCF_3$ | N | CH | |
| B-31 | H | Me | Me | 2-F | N | $CCF_3$ | CH | CH | |
| B-32 | H | Me | Me | 2-Cl | N | $CCF_3$ | N | CH | |
| B-33 | H | Me | Me | 3-F | N | $CCF_3$ | N | CH | |
| B-34 | H | Me | Me | 3-F | N | $CCF_3$ | CH | CH | |
| B-35 | H | Me | Me | 3-Cl | N | $CCF_3$ | N | CH | |
| B-36 | H | Me | Me | 3-Cl | N | $CCF_3$ | CH | CH | |
| B-37 | H | Me | Me | 3-Br | N | $CCF_3$ | N | CH | |
| B-38 | H | Me | Me | 3-Br | N | $CCF_3$ | CH | CH | |
| B-39 | H | Me | Me | 2,4-$F_2$ | N | $CCF_3$ | N | CH | |

(continued)

| Compound No. | $R_1$ | $R_3$ | $R_4$ | X | $Y_1$ | $Y_2$ | $Y_3$ | $Y_4$ | State, Melting Point(°C) |
|---|---|---|---|---|---|---|---|---|---|
| B-40 | H | Me | Me | 2,5-$F_2$ | N | $CCF_3$ | N | CH | |
| B-41 | H | Me | Me | 3,5-$F_2$ | N | $CCF_3$ | N | CH | |

General Formula (3)

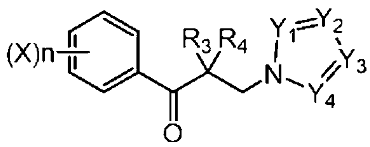
(3)

Table 3

| Compound No. | $R_3$ | $R_4$ | X | $Y_1$ | $Y_2$ | $Y_3$ | $Y_4$ | State, Melting Point(°C) |
|---|---|---|---|---|---|---|---|---|
| C-1 | Me | H | H | N | $CCF_3$ | N | CH | |
| C-2 | Me | H | 4-F | N | $CCF_3$ | N | CH | oil |
| C-3 | Me | H | 4-F | N | $CCF_3$ | CH | CH | oil |
| C-4 | Me | H | 4-Cl | N | $CCF_3$ | N | CH | oil |
| C-5 | Me | H | 4-Br | N | $CCF_3$ | N | CH | oil |
| C-6 | Me | H | 4-Br | N | $CCF_3$ | CH | CH | oil |
| C-7 | Me | H | 2-F | N | $CCF_3$ | N | CH | oil |
| C-8 | Me | H | 2-Cl | N | $CCF_3$ | N | CH | oil |
| C-9 | Me | H | 2-Cl | N | $CCF_3$ | CH | CH | |
| C-10 | Me | H | 3-F | N | $CCF_3$ | N | CH | oil |
| C-11 | Me | H | 3-Cl | N | $CCF_3$ | N | CH | oil |
| C-12 | Me | H | 3-Cl | N | $CCF_3$ | CH | CH | oil |
| C-13 | Me | H | 3-Br | N | $CCF_3$ | N | CH | oil |
| C-14 | Me | H | 3-Br | N | $CCF_3$ | CH | CH | oil |
| C-15 | Me | H | 2,3-$F_2$ | N | $CCF_3$ | N | CH | oil |
| C-16 | Me | H | 2,4-$F_2$ | N | $CCF_3$ | N | CH | oil |
| C-17 | Me | H | 2,5-$F_2$ | N | $CCF_3$ | CH | CH | oil |
| C-18 | Me | H | 3,4-$F_2$ | N | $CCF_3$ | N | CH | oil |
| C-19 | Me | H | 3,4-$Cl_2$ | N | $CCF_3$ | N | CH | oil |
| C-20 | Me | H | 3, 4-$Cl_2$ | N | $CCF_3$ | CH | CH | oil |
| C-21 | Et | H | H | N | $CCF_3$ | N | CH | oil |
| C-22 | Et | H | H | N | $CCF_3$ | CH | CH | oil |
| C-23 | Me | Me | H | N | $CCF_3$ | N | CH | oil |
| C-24 | Me | Me | H | N | $CCF_3$ | CH | CH | oil |
| C-25 | Me | Me | 4-F | N | $CCF_3$ | N | CH | oil |
| C-26 | Me | Me | 4-Cl | N | $CCF_3$ | N | CH | 66-67 |

(continued)

| Compound No. | $R_3$ | $R_4$ | X | $Y_1$ | $Y_2$ | $Y_3$ | $Y_4$ | State, Melting Point(°C) |
|---|---|---|---|---|---|---|---|---|
| C-27 | Me | Me | 4-Cl | N | $CCF_3$ | CH | CH | oil |
| C-28 | Me | Me | 4-Br | N | $CCF_3$ | N | CH | oil |
| C-29 | Me | Me | 4-Br | N | $CCF_3$ | CH | CH | |
| C-30 | Me | Me | 2-F | N | $CCF_3$ | N | CH | oil |
| C-31 | Me | Me | 2-F | N | $CCF_3$ | CH | CH | oil |
| C-32 | Me | Me | 2-Cl | N | $CCF_3$ | N | CH | oil |
| C-33 | Me | Me | 3-F | N | $CCF_3$ | N | CH | oil |
| C-34 | Me | Me | 3-F | N | $CCF_3$ | CH | CH | oil |
| C-35 | Me | Me | 3-Cl | N | $CCF_3$ | N | CH | oil |
| C-36 | Me | Me | 3-Cl | N | $CCF_3$ | CH | CH | oil |
| C-37 | Me | Me | 3-Br | N | $CCF_3$ | N | CH | oil |
| C-38 | Me | Me | 3-Br | N | $CCF_3$ | CH | CH | oil |
| C-39 | Me | Me | 2,4-$F_2$ | N | $CCF_3$ | N | CH | oil |
| C-40 | Me | Me | 2.5-$F_2$ | N | $CCF_3$ | N | CH | oil |
| C-41 | Me | Me | 3,5-$F_2$ | N | $CCF_3$ | N | CH | oil |
| C-42 | i-Pr | H | H | N | $CCF_3$ | N | CH | oil |
| C-43 | Me | H | 3.5-$F_2$ | N | $CCF_3$ | N | CH | oil |
| C-44 | Me | H | 3-Cl | CH | $CCF_3$ | CH | N | oil |
| C-45 | Me | H | 3-Cl | CH | $CCF_3$ | N | CH | oil |
| C-46 | Me | H | 3-$CF_3$ | N | $CCF_3$ | N | CH | oil |
| C-47 | Me | H | 2,5-$F_2$ | N | $CCF_3$ | N | CH | oil |
| C-48 | Me | H | 2,3,5-$F_3$ | N | $CCF_3$ | N | CH | oil |
| C-49 | Me | H | 3, 5-$Cl_2$ | N | $CCF_3$ | N | CH | oil |
| C-50 | Me | H | 4-Me | N | $CCF_3$ | N | CH | oil |
| C-51 | Me | H | 3-Me | N | $CCF_3$ | N | CH | oil |
| C-52 | Me | H | 2-Me | N | $CCF_3$ | N | CH | oil |
| C-53 | Me | H | 3-$NO_2$ | N | $CCF_3$ | N | CH | oil |
| C-54 | Me | H | 4-Me0 | N | $CCF_3$ | N | CH | oil |
| C-55 | Me | H | 4-CN | N | $CCF_3$ | N | CH | oil |
| C-56 | Me | H | 4-$CF_3O$ | N | $CCF_3$ | N | CH | oil |
| C-57 | Me | H | 4-Cl | N | $CCF_3$ | CH | $CCF_3$ | oil |
| C-58 | Me | H | 4-Cl | N | $CCF_3$ | CH | $CCH_3$ | oil |
| C-59 | Me | H | 4-Cl | N | CH | CBr | CH | oil |
| C-60 | Me | H | 4-Cl | N | CH | CCl | CH | oil |

General Formula (16)

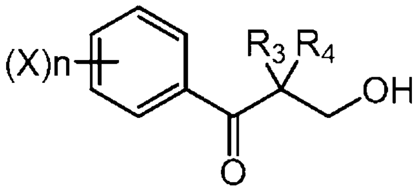

(16)

Table 4

| Compound No. | $R_3$ | $R_4$ | X | State, Melting Point (°C) |
|---|---|---|---|---|
| D-1 | Me | H | H | oil |
| D-2 | Me | H | 4-F | |
| D-3 | Me | H | 4-Cl | |
| D-4 | Me | H | 4-Br | |
| D-5 | Me | H | 2-F | oil |
| D-6 | Me | H | 2-Cl | oil |
| D-7 | Me | H | 2-Br | |
| D-8 | Me | H | 3-F | oil |
| D-9 | Me | H | 3-Cl | oil |
| D-10 | Me | H | 3-Br | oil |
| D-11 | Me | H | 3-$CF_3$ | oil |
| D-12 | Me | H | 2,3-$F_2$ | oil |
| D-13 | Me | H | 2,4-$F_2$ | oil |
| D-14 | Me | H | 2,5-$F_2$ | oil |
| D-15 | Me | H | 3,4-$F_2$ | oil |
| D-16 | Me | H | 3, 4-$Cl_2$ | oil |
| D-17 | Me | Me | H | oil |
| D-18 | Me | Me | 4-F | oil |
| D-19 | Me | Me | 4-Cl | oil |
| D-20 | Me | Me | 4-Br | oil |
| D-21 | Me | Me | 2-F | oil |
| D-22 | Me | Me | 2-Cl | oil |
| D-23 | Me | Me | 2-Br | |
| D-24 | Me | Me | 3-F | oil |
| D-25 | Me | Me | 3-Cl | oil |
| D-26 | Me | Me | 3-Br | oil |
| D-27 | Me | Me | 2,4-$F_2$ | oil |
| D-28 | Me | Me | 2,5-$F_2$ | oil |
| D-29 | Me | Me | 3,4-$F_2$ | oil |
| D-30 | Et | H | H | oil |

(continued)

| Compound No. | $R_3$ | $R_4$ | X | State, Melting Point (°C) |
|---|---|---|---|---|
| D-31 | Me | H | 3, 5-$F_2$ | oil |
| D-32 | Me | H | 2,3,5$F_3$ | oil |
| D-33 | Me | H | 3, 5-$Cl_2$ | oil |
| D-34 | Me | H | 4-Me | oil |
| D-35 | Me | H | 3-Me | oil |
| D-36 | Me | H | 2-Me | oil |
| D-37 | Me | H | 4-MeD | oil |
| D-38 | Me | H | 4-$CF_3O$ | oil |
| D-39 | Me | H | 3-$NO_2$ | oil |
| D-40 | Me | H | 4-CN | oil |
| D-41 | i-Pr | H | H | oil |

General Formula (17)

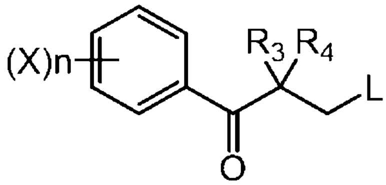

(17)

Table 5

| Compound No. | $R_3$ | $R_4$ | X | L | State, Melting Point (°C) |
|---|---|---|---|---|---|
| E-1 | Me | H | H | Cl | |
| E-2 | Me | H | 4-F | Cl | |
| E-3 | Me | H | 4-Cl | Cl | |
| E-4 | Me | H | 4-Br | Cl | |
| E-5 | Me | H | 2-F | Cl | |
| E-6 | Me | H | 2-Cl | Cl | |
| E-7 | Me | H | 2-Br | Cl | |
| E-8 | Me | H | 3-F | Cl | oil |
| E-9 | Me | H | 3-Cl | Cl | |
| E-10 | Me | H | 3-Br | Cl | |
| E-11 | Me | H | 2,3-$F_2$ | Cl | |
| E-12 | Me | H | 2,5-$F_2$ | Cl | |
| E-13 | Me | Me | H | 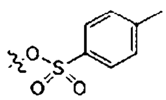 | solid |

(continued)

| Compound No. | $R_3$ | $R_4$ | X | L | State, Melting Point (°C) |
|---|---|---|---|---|---|
| E-14 | Me | Me | 4-F | 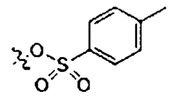 | 81-83 |
| E-15 | Me | Me | 4-Cl | 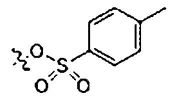 | solid |
| E-16 | Me | Me | 4-Br | 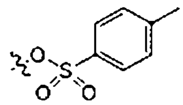 | 102-103 |
| E-17 | Me | Me | 2-F | 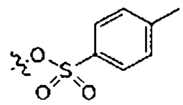 | oil |
| E-18 | Me | Me | 2-Cl | 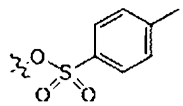 | oil |
| E-19 | Me | Me | 2-Br | 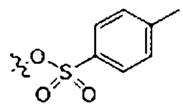 | |
| E-20 | Me | Me | 3-F | 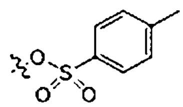 | oil |
| E-21 | Me | Me | 3-Cl | 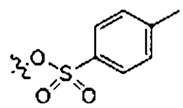 | oil |
| E-22 | Me | Me | 3-Br | 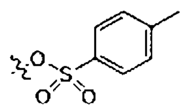 | oil |
| E-23 | Me | Me | $2,4\text{-}F_2$ | 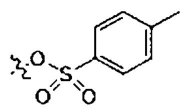 | oil |
| E-24 | Me | Me | $2,5\text{-}F_2$ | 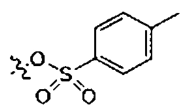 | oil |
| E-25 | Me | Me | $3,4\text{-}F_2$ | 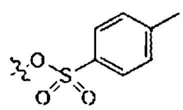 | 98-108 |

The compound of the present invention can be used for the prevention or extermination of organisms that cause harm in various settings, such as in agriculture, indoors or in forests, to livestock, or in terms of sanitation. Specific use scenes, target pests, methods of use, and the like are shown below, but the scope of the present invention is not limited in any way by these use scenes, target pests, or methods of use.

The compound of the present invention represented by the formula (1) can be used to control pests such as arthropods; mollusks; nematodes; fungi from phyla such as Eumycota, Myxomycota, Bacteriomycota, and Actinomycota;

and bacteria that cause damage to agricultural crops, for example, edible crops (rice; grains such as barley, wheat, rye, and oats; corn; potato; sweet potato; taro; legumes such as soybean, adzuki bean, fava bean, pea, kidney bean, and peanut), vegetables (Brassicaceae crops such as cabbage, Chinese cabbage, daikon radish, turnip, broccoli, cauliflower, and komatsuna; cucurbits such as pumpkin, cucumber, watermelon, oriental melon, and melon; eggplant, tomato, bell pepper, pepper, okra, spinach, lettuce, lotus root, carrot, burdock, garlic, onion, and alliaceous plants such as scallions), mushrooms (shiitake, mushroom, etc.), fruit trees and fruits (apple, citrus fruits, pear, grape, peach, plum, cherry, walnut, chestnut, almond, banana, strawberry, etc.), ornamental crops for fragrance and other purposes (lavender, rosemary, thyme, parsley, pepper, ginger, etc.), special crops (tobacco, tea, sugar beet, sugarcane, hop, cotton, hemp, olive, rubber, coffee, etc.), pasture and forage crops (timothy, clover, alfalfa, corn, sorghums, orchard grass, gramineous pasture grass, leguminous pasture grass, etc.), turfgrasses (Zoysia grass, bentgrass, etc.), forest trees (Sakhalin firs, Yezo spruces, pines, thujopsis, Japanese cedar, Japanese cypress, etc.), and ornamental plants (herbaceous plants and flowers such as chrysanthemum, rose, carnation, and orchid; garden trees such as ginkgo, cherry trees, and Japanese laurel). Specific pests include the following.

From the class Insecta of the phylum Arthropoda, the order Lepidoptera, for example, Noctuidae such as the cotton bollworm (Helicoverpa armigera), Heliothis species (Heliothis spp.), turnip moth (Agrotis segetum), beet semilooper (Autographa nigrisigna), cabbage looper (Trichoplusia ni), cabbage moth (Mamestra brassicae), beet armyworm (Spodoptera exigua), and tobacco cutworm (Spodoptera litura); Plutellidae such as the diamondback moth (Plutella xylostella); Tortricidae such as the summer fruit tortrix (Adoxophyes orana fasciata), smaller tea tortrix (Adoxophyes honmai), apple tortrix (Archips fuscocupreanus), tea tortrix (Homona magnanima), tea leafroller (Caloptilia theivora), and oriental fruit moth (Grapholita molesta); Psychidae such as the tea bagworm (Eumeta minuscula), etc.,

Lyonetiidae such as the silver-marked leaf miner (Lyonetia prunifoliella malinella) and the peach leaf miner (Lyonetia clerkella); Gracillariidae such as the citrus leaf miner (Phyllocnistis citrella); Gracillariidae such as the Asiatic apple leaf miner (Phyllonorycter ringoniella); Acrolepiidae such as the Japanese onion moth (Acrolepiopsis sapporensis); Sesiidae such as the cherry tree borer moth (Synanthedon hector); Stathmopodidae such as the persimmon calyx moth (Stathmopoda masinissa); Gelechiidae such as the pink bollworm (Pectinophora gossypiella); Carposinidae such as the peach fruit moth (Carposina niponensis); Cossidae such as the oriental carpenter moth (Cossus jezoensis); Tineidae such as the European grain moth (Nemapogon granella); Limacodidae such as the oriental moth (Monema flavescens), the nettle caterpillar (Parasa lepida), and the lesser black slug moth (Scopelodes contracta); Crambidae such as the rice stem borer (Chilo suppressalis), the yellow stem borer (Scirpophaga incertulas), the rice leaffolder (Cnaphalocrocis medinalis), the cabbage webworm (Hellula undalis), and the yellow peach moth (Conogethes punctiferalis),

the cucumber moth (Diaphania indica), and bluegrass sod webworm (Parapediasia teterrellus); Pyralidae such as the crested snout moth (Locastra muscosalis); Hesperiidae such as the common straight swift (Parnara guttata); Papilionidae such as the Asian swallowtail (Papilio xuthus); Pieridae such as the cabbage white (Pieris rapae crucivora); Lycaenidae such as the pea blue (Lampides boeticus); Geometridae such as the giant looper (Ascotis selenaria); Sphingidae such as the convolvulus hawk-moth (Agrius convolvuli); Notodontidae such as the Japanese buff-tip (Phalera flavescens); Lasiocampidae such as the lackey moth (Malacosoma neustria testacea); Saturniidae such as the Japanese giant silkworm moth (Saturnia japonica); Lymantriidae such as the tea tussock moth (Euproctis pseudoconspersa), white-spotted tussock moth (Orgyia thyellina), and scarce vapourer (Telochurus recens approximans); Arctiidae such as the mulberry tiger moth (Spilosoma imparilis) and fall webworm (Hyphantria cunea); grape berry moth (Endopiza viteana) and codling moth (Laspeyresia pomonella), etc.; adults, larvae, and eggs of these and others;

the order Coleoptera, for example, Scarabaeidae such as the cupreous chafer (Anomala cuprea), Japanese beetle (Popillia japonica), citrus flower chafer (Oxycetonia jucunda), and cherry chafer (Anomala geniculata); Buprestidae such as the flatheaded citrus borer (Agrilus auriventris); Elateridae such as the click beetle (Melanotus fortnumi); Coccinellidae such as the 28-spotted potato ladybird (Epilachna vigintioctopunctata); Cerambycidae such as the white-spotted longicorn beetle (Anoplophora malasiaca) and grape borer (Xylotrechus pyrrhoderus); Chrysomelidae such as the pumpkin beetle (Aulacophora femoralis), rootworm species (Diabrotica spp.), striped flea beetle (Phyllotreta striolata), tortoise beetle (Cassida nebulosa), brassica leaf beetle (Phaedon brassicae), rice leaf beetle (Oulema oryzae), Mexican bean beetle (Epilachna varivestis), and Colorado potato beetle (Leptinotarsa decemlineata); Attelabidae such as the peach curculio (Rhynchites heros); Brentidae such as the sweetpotato weevil (Cylas formicarius); Curculionidae such as the chestnut weevil (Curculio sikkimensis), rice water weevil (Lissorhoptrus oryzophilus), boll weevil (Anthonomus grandis grandis), and hunting billbug (Sphenophorus venatus vestitus); Nitidulidae such as the sap beetle (Epuraea domina); adults, larvae, and eggs of these and others;

Heteroptera in the order Hemiptera, for example, Pentatomidae such as the cabbage bug (Eurydema rugosum), Lewis spined bug (Eysarcoris lewisi), white-spotted spined bug (Eysarcoris parvus), southern green stink bug (Nezara viridula), brown-winged green stink bug (Plautia stali), and brown marmorated stink bug (Halyomorpha mista); Urostylididae such as the pear stink bug (Urochela luteovaria); Lygaeidae such as the short-winged seed bug (Togo hemipterus); Coreidae such as the bean bug (Riptortus clavatus) and rice stink bug (Cletus punctiger); Alydidae such as the rice bug (Leptocorisa chinensis); Pyrrhocoridae such as the red cotton stainer (Dysdercus cingulatus); Tingidae such as the pear

lace bug (Stephanitis nashi) and azalea lace bug (Stephanitis pyrioides); Miridae such as the pale green plant bug (Apolygus spinolae), sorghum plant bug (Stenotus rubrovittatus), and rice leaf bug (Trigonotylus caelestialium); Plataspidae such as the Japanese common plataspid bug (Megacopta punctatissima); adults, larvae, and eggs of these and others;

Homoptera in the order Hemiptera, for example, Cicadidae such as the Kaempfer cicada (Platypleura kaempferi); Cicadellidae such as the two-spotted dwarf leafhopper (Arboridia apicalis), tea green leafhopper (Empoasca onukii), rice green leafhopper (Nephotettix cincticeps), and green leafhopper (Nephotettix virescens); Delphacidae such as the small brown planthopper (Laodelphax striatellus), brown planthopper (Nilaparvata lugens), and white-backed planthopper (Sogatella furcifera); Flatidae such as the green flatid planthopper (Geisha distinctissima); Psyllidae such as the large pear sucker (Psylla pyrisuga) and Asian citrus psyllid (Diaphorina citri); Aleyrodidae such as the orange spiny whitefly (Aleurocanthus spiniferus), the silverleaf whitefly (Bemisia argentifolii), various biotypes of tobacco whitefly (Bemisia tabaci), citrus whitefly (Dialeurodes citri), and greenhouse whitefly (Trialeurodes vaporariorum); Phylloxeridae such as the grape phylloxera (Viteus vitifoliae); Aphididae such as the spirea aphid (Aphis citricola), cowpea aphid (Aphis craccivora), cotton aphid (Aphis gossypii), foxglove aphid (Aulacorthum solani), cabbage aphid (Brevicoryne brassicae), black citrus aphid (Toxoptera aurantii), brown citrus aphid (Toxoptera citricidus), elder long-tailed aphid (Aulacorthum magnoliae), oriental pear aphid (Schizaphis piricola), green pear aphid (Nippolachnus piri), turnip aphid (Lipaphis erysimi), mealy plum aphid (Hyalopterus pruni), chrysanthemum aphid (Pleotrichophorus chrysanthemi), chrysanthemum aphid (Macrosiphoniella sanborni), faba-bean long-tailed aphid (Megoura crassicauda), rose aphid (Sitobion ibarae),

the potato aphid (Macrosiphum euphorbiae), peach-clematis aphid (Myzus varians), green peach aphid (Myzus persicae), rice root aphid (Rhopalosiphum rufiabdominale), bird cherry-oat aphid (Rhopalosiphum padi), long-antenna wheat aphid (Sitobion akebiae), and woolly apple aphid (Eriosoma lanigerum); Monophlebidae such as the cottony cushion scale (Icerya purchasi); Pseudococcidae such as Comstock mealybug (Pseudococcus comstocki), viburnum mealybug (Phenacoccus viburnae), and Japanese mealybug (Phenacoccus kraunhiae); Coccidae such as the Indian wax scale (Ceroplastes ceriferus) and red wax scale (Ceroplastes rubens); Diaspididae such as the California red scale (Aonidiella aurantii), San Jose scale (Comstockaspis perniciosa), white peach scale (Pseudaulacaspis pentagona), and arrowhead scale (Unaspis yanonensis); adults, larvae, and eggs of these and others;

the order Thysanoptera, for example, Thripidae such as the chilli thrips (Scirtothrips dorsalis), melon thrips (Thrips palmi), onion thrips (Thrips tabaci), Japanese flower thrips (Thrips setosus), European flower thrips (Frankliniella intonsa), western flower thrips (Frankliniella occidentalis), and greenhouse thrips (Heliothrips haemorrhoidalis); Phlaeothripidae such as the persimmon thrips (Ponticulothrips diospyrosi) and rice thrips (Haplothrips aculeatus); adults, larvae, and eggs of these and others;

the order Hymenoptera, for example, Tenthredinidae such as the turnip sawfly (Athalia rosae ruficornis) and the large rose sawfly (Arge pagana); Argidae such as the apple sawfly (Arge mali); Cynipidae such as the chestnut gall wasp (Dryocosmus kuriphilus); Megachilidae such as the rose leafcutter bee (Megachile nipponica nipponica); Formicidae such as the black mountain ant (Formica japonica), Mikado carpenter ant (Camponotus kiusiuensis), jet black ant (Lasius fuliginosus), and fire ants (Solenopsis richteri, S. invicta, S. geminata); adults, larvae, and eggs of these and others;

the order Diptera, for example, Cecidomyiidae such as the soybean pod gall midge (Asphondylia yushimai); Tephritidae such as the Japanese cherry fruit fly (Rhacochlaena japonica) and melon fly (Bactrocera cucurbitae); Ephydridae such as the smaller rice leaf miner (Hydrellia griseola); Drosophilidae such as the spotted-wing drosophila (Drosophila suzukii); Agromyzidae such as the American serpentine leaf miner (Liriomyza trifolii), vegetable leaf miner (Liriomyza sativae), pea leaf miner (Chromatomyia horticola), rice leaf miner (Agromyza oryzae), and tomato leaf miner (Liriomyza bryoniae); Anthomyiidae such as the seedcorn maggot (Delia platura) and onion maggot (Delia antiqua); adults, larvae, and eggs of these and others;

the order Orthoptera, for example, Acrididae such as the migratory locust (Locusta migratoria); Tettigoniidae such as the meadow katydid (Ruspolia lineosa); Gryllidae such as the Emma field cricket (Teleogryllus emma) and green tree cricket (Truljalia hibinonis); Gryllotalpidae such as the oriental mole cricket (Gryllotalpa orientalis); Acrididae such as the Japanese grasshopper (Oxya yezoensis); adults, larvae, and eggs of these and others;

the order Isoptera, for example, Termitidae such as the Formosan subterranean termite (Odontotermes formosanus); adults, larvae, and eggs of these and others;

the order Dermaptera, for example, Labiduridae such as the shore earwig (Labidura riparia); adults, larvae, and eggs of these and others;

from the subclass Entognatha of the phylum Arthropoda, the order Collembola, for example, Sminthuridae such as the lucerne flea (Sminthurus viridis); Onychiuridae such as the Matsumoto white springtail (Onychiurus matsumotoi); adults, larvae, and eggs of these and others; from the class Malacostraca of the phylum Arthropoda, the order Isopoda, for example, Armadillidiidae such as the common pill bug (Armadillidium vulgare); adults, larvae, and eggs of these and others;

from the class Arachnida of the phylum Arthropoda, the order Acari, for example, Tarsonemidae such as the broad mite (Polyphagotarsonemus latus) and cyclamen mite (Phytonemus pallidus); Penthaleidae such as the blue oat mite (Penthaleus major); Tenuipalpidae such as the citrus flat mite (Brevipalpus lewisi) and false spider mite (Brevipalpus phoenicis); Tetranychidae such as the citrus red mite (Panonychus citri), European red mite (Panonychus ulmi), two-spotted spider mite (Tetranychus urticae), Kanzawa spider mite (Tetranychus kanzawai), hawthorn spider mite (Amphitetranychus viennensis), spruce spider mite (Oligonychus ununguis), Kikubira spider mite (Bryobia eharai), citrus yellow mite (Eotetranychus kankitus), and clover mite (Bryobia praetiosa); Eriophyidae such as the apple rust mite (Aculus schlechtendali), Japanese citrus rust mite (Aculops pelekassi), Ryukyu citrus rust mite (Phyllocoptruta citri), Japanese pear rust mite (Eriophyes chibaensis), tulip bulb mite (Aceria tulipae), grape erineum mite (Colomerus vitis), peach silver mite (Aculus fockeui), and tea rust mite (Calacarus carinatus); Acaridae such as the mold mite (Tyrophagus putrescentiae) and bulb mite (Rhizoglyphus robini); adults, larvae, and eggs of these and others;
from the class Gastropoda of the phylum Mollusca, the order Architaenioglossa, for example, Ampullariidae such as the channeled applesnail (Pomacea canaliculata); the order Pulmonata, for example, Achatinidae such as the giant African snail (Achatina fulica); Philomycidae such as the Chinese slug (Meghimatium bilineatum); Milacidae such as the black-keeled slug (Milax gagates); Limacidae such as the greenhouse slug (Lehmannia valentiana); Bradybaenidae such as the Korean round snail (Acusta despecta sieboldiana), etc.;
from the class Chromadorea of the phylum Nematoda, the order Tylenchida, for example, Anguinidae such as the potato rot nematode (Ditylenchus destructor); Tylenchorhynchidae such as the stunt nematode (Tylenchorhynchus claytoni); Pratylenchidae such as the northern root-lesion nematode (Pratylenchus penetrans) and coffee root-lesion nematode (Pratylenchus coffeae); Hoplolaimidae such as the spiral nematode (Helicotylenchus dihystera); Heteroderidae such as the golden nematode (Globodera rostochiensis); Meloidogynidae such as the southern root-knot nematode (Meloidogyne incognita); Criconematidae such as the ring nematode (Criconema jaejuense); Anguinidae such as the strawberry nematode (Nothotylenchus acris); Aphelenchoididae such as the strawberry foliar nematode (Aphelenchoides fragariae); from the class Enoplea, the order Dorylaimida, for example, Longidoridae such as the dagger nematode (Xiphinema sp.) and Trichodoridae such as the stubby-root nematode (Trichodorus sp.), etc.;
fungi from phyla such as Eumycota, Myxomycota, Bacteriomycota, and Actinomycota, as well as bacteria.

Diseases to which the compound of the present invention represented by the formula (1) can be applied include, specifically, rice blast (Pyricularia oryzae), brown spot (Cochliobolus miyabeanus), sheath blight (Rhizoctonia solani), bacterial palea browning (Pantoea ananatis), bacterial brown stripe (Acidovorax avenae subsp. avenae), bacterial sheath brown rot (Pseudomonas fuscovaginae), bacterial leaf blight (Xanthomonas oryzae pv. oryzae), and bacterial seedling blight (Burkholderia plantarii) of rice; powdery mildew (Erysiphe graminis), Fusarium head blight (Gibberella zeae), rusts (Puccinia striiformis, P. graminis, P. recondita, P. hordei), snow mold (Typhula sp., Micronectriella nivalis), loose smut (Ustilago tritici, U. nuda), bunt (Tilletia caries), eyespot (Pseudocercosporella herpotrichoides), scald (Rhynchosporium secalis), leaf blotch (Septoria tritici), glume blotch (Leptosphaeria nodorum), net blotch (Pyrenophora teres), spot blotch (Helminthosporium zonatum Ikata), and bacterial black node (Pseudomonas syringae pv. japonica) of wheat and barley; melanose (Diaporthe citri), scab (Elsinoe fawcettii), fruit rot (Penicillium digitatum, P. italicum), brown rot (Phytophthora citrophthora, P. nicotianae), and black spot (Phyllosticta citricarpa) of citrus; Monilinia mali of apple,

Valsa canker (Valsa mali), powdery mildew (Podosphaera leucotricha), Alternaria blotch (Alternaria mali), scab (Venturia inaequalis), black spot (Mycosphaerella pomi), anthracnose (Colletotrichum acutatum), ring rot (Botryosphaeria berengeriana), rust (Gymnosporangium yamadae), and brown rot (Monilinia fructicola) of apple; scab (Venturia nashicola, V. pirina), black spot (Alternaria kikuchiana), rust (Gymnosporangium haraeanum), and brown rot (Monilinia fructigena) of pear; brown rot (Monilinia fructicola), scab (Cladosporium carpophilum), Phomopsis rot (Phomopsis sp.), and bacterial shot hole (Brenneria nigrifluens) of peach; anthracnose (Elsinoe ampelina), ripe rot (Colletotrichum acutatum), powdery mildew (Uncinula necator), rust (Phakopsora ampelopsidis), black rot (Guignardia bidwellii), downy mildew (Plasmopara viticola), brown rot (Monilinia fructigena), scab (Cladosporium viticolum), gray mold (Botrytis cinerea), and bacterial crown gall (Agrobacterium vitis) of grapes; anthracnose (Gloeosporium kaki), and leaf spot (Cercospora kaki, Mycosphaerella nawae) of persimmon; anthracnose (Colletotrichum lagenarium), powdery mildew (Sphaerotheca fuliginea, Oidiopsis taurica), gummy stem blight (Didymella bryoniae), Fusarium wilt (Fusarium oxysporum), downy mildew (Pseudoperonospora cubensis), phytophthora blight (Phytophthora sp.), and damping-off (Pythium sp.) of cucurbits; angular leaf spot (Pseudomonas syringae pv. lachrymans), bacterial leaf blight (Pseudomonas viridiflava), and bacterial fruit blotch (Xanthomonas campestris pv. cucurbitae) of cucumber; bacterial fruit blotch (Xanthomonas campestris pv. cucurbitae), hairy root (Agrobacterium rhizogenes), and gall disease (Streptomyces sp.) of melon; bacterial fruit blotch (Acidovorax avenae pv. citrulli) of watermelon; bacterial wilt (Ralstonia solanacearum) of solanaceous vegetables; early blight (Alternaria solani), leaf mold (Cladosporium fulvum), late blight (Phytophthora infestans), and bacterial canker (Clavibacter michiganensis subsp. michiganensis) of tomato,

pith necrosis (Pseudomonas corrugata), and soft rot (Pectobacterium carotovorum subsp. carotovorum) of tomato; Phomopsis blight (Phomopsis vexans), and powdery mildew (Erysiphe cichoracearum) of eggplant; black spot (Alternaria japonica), white leaf spot (Cercosporella brassicae), and soft rot (Pectobacterium carotovorum subsp. carotovorum) of Brassicaceae vegetables; head rot (Pseudomonas syringae pv. marginalis), and black rot (Xanthomonas campestris pv. campestris) of cabbage; bacterial rot (Pseudomonas cichorii, Pseudomonas viridiflava), and bacterial spot (Xanthomonas campestris pv. vitians) of lettuce; rust (Puccinia allii) of onion; purple seed stain (Cercospora kikuchii), scab (Elsinoe glycines), and pod and stem blight (Diaporthe phaseolorum var. sojae) of soybean; anthracnose (Colletotrichum lindemuthianum) of kidney bean; leaf spot (Cercospora personata) and brown leaf spot (Cercospora arachidicola) of peanut; powdery mildew (Erysiphe pisi) of pea; early blight (Alternaria solani), late blight (Phytophthora infestans), and Rhizoctonia solani of potato; powdery mildew (Sphaerotheca humuli) of strawberry; blister blight (Exobasidium reticulatum), white scab (Elsinoe leucospila), bacterial shoot blight (Pseudomonas syringae pv. theae), and bacterial canker (Xanthomonas campestris pv. theicola) of tea; brown spot (Alternaria longipes), powdery mildew (Erysiphe cichoracearum), anthracnose (Colletotrichum tabacum), blue mold (Peronospora tabacina), black shank (Phytophthora nicotianae), Granville wilt (Ralstonia solanacearum), and hollow stalk (Pectobacterium carotovorum subsp. carotovorum) of tobacco; leaf spot (Cercospora beticola) and damping-off (Aphanomyces cochlioides) of sugar beet;

black spot (Diplocarpon rosae) and powdery mildew (Sphaerotheca pannosa) of rose; leaf spot (Septoria chrysanthemi-indici), white rust (Puccinia horiana), and crown gall (Agrobacterium tumefaciens) of chrysanthemum; gray mold (Botrytis cinerea) and Sclerotinia rot (Sclerotinia sclerotiorum) of various crops such as eggplant, cucumber, and lettuce; snow mold (Pythium iwayamai, Typhula incamata, Fusarium nivale, Sclerotinia borealis), powdery mildew (Erysiphe graminis), fairy ring (Lycoperdon perlatum, Lepista subnuda, Marasmius oreades), yellow patch (Ceratobasidium spp.), take-all patch (Gaeumannomyces graminis), Curvularia leaf blight (Curvularia geniculata), brown patch (Rhizoctonia solani), Pythium blight (Pythium periplocum, Pythium vanterpoolii), rust (Puccinia spp.), and dollar spot (Sclerotinia homoeocarpa) of turfgrass; Pythium blight (Pythium aphanidermatum) and anthracnose (Colletotrichum sp.) of bentgrass.

The compound of the present invention represented by the formula (1) can also be used to control arthropods and fungi that are active indoors in buildings including general houses, and damage or decay wood and its processed products such as wooden furniture, stored food, clothing, books, etc., and cause damage to our lives. Specific pests include the following.

From the class Insecta of the phylum Arthropoda, the order Isoptera, for example, Rhinotermitidae such as the Formosan subterranean termite (Coptotermes formosanus), Japanese subterranean termite (Reticulitermes speratus), other Reticulitermes termites (Reticulitermes hesperus, R. tibialis, R. flavipes, R. lucifugus, R. santonensis, etc.), western drywood termite (Incisitermes minor); Termitidae such as the black-winged subterranean termite (Odontotermes formosanus); Hodotermitidae such as Hodotermopsis japonica; Kalotermitidae such as the domestic drywood termite (Cryptotermes domesticus); adults, larvae, and eggs of these and others;

the order Coleoptera, for example, Dryophthoridae such as the maize weevil (Sitophilus zeamais) and rice weevil (Sitophilus zeamais); Bruchidae such as the adzuki bean weevil (Callosobruchus chinensis), pea weevil (Bruchus pisorum), and broadbean weevil (Bruchus rufimanus); Tenebrionidae such as the red flour beetle (Tribolium castaneum) and confused flour beetle (Tribolium confusum); Silvanidae such as the sawtoothed grain beetle (Oryzaephilus surinamensis) and flat grain beetle (Cryptolestes pusillus); Anobiidae such as the cigarette beetle (Lasioderma serricorne) and drugstore beetle (Stegobium paniceum); Dermestidae such as the black carpet beetle (Attagenus unicolor japonicus), varied carpet beetle (Anthrenus verbasci), and hide beetle (Dermestes maculatus); Ptinidae such as the smooth spider beetle (Gibbium aequinoctiale); Bostrichidae such as the bamboo borer (Dinoderus minutus) and lesser grain borer (Rhyzopertha dominica); Lyctidae such as the powder-post beetle (Lyctus brunneus); adults, larvae, and eggs of these and others;

the order Lepidoptera, for example, Pyralidae such as the warehouse moth (Cadra cautella), Mediterranean flour moth (Ephestia kuehniella), and Indian meal moth (Plodia interpunctella); Gelechiidae such as the Angoumois grain moth (Sitotroga cerealella); Tineidae such as the clothes moth (Tinea translucens) and webbing clothes moth (Tineola bisselliella); adults, larvae, and eggs of these and others; the order Psocoptera, for example, Trogiidae such as the glossy booklouse (Lepinotus reticulatus); Liposcelididae such as the flat booklouse (Liposcelis bostrychophila); adults, larvae, and eggs of these and others;

the order Blattodea, for example, Ectobiidae such as the German cockroach (Blattella germanica); Blattidae such as the smokybrown cockroach (Periplaneta fuliginosa) and Japanese cockroach (Periplaneta japonica); adults, larvae, and eggs of these and others; the order Hymenoptera, for example, Formicidae such as the pharaoh ant (Monomorium pharaonis) and Japanese small ant (Monomorium nipponense); adults, larvae, and eggs of these and others; the order Zygentoma, for example, Lepismatidae such as the oriental silverfish (Ctenolepisma villosa) and silverfish

(Lepisma saccharina); adults, larvae, and eggs of these and others;

the order Diptera, for example, Drosophilidae such as the common fruit fly (Drosophila melanogaster); Piophilidae such as the cheese skipper (Piophila casei); adults, larvae, and eggs of these and others;

from the class Arachnida of the phylum Arthropoda, the order Acari, for example, Acaridae such as the mold mite (Tyrophagus putrescentiae) and fish mite (Lardoglyphus konoi); Carpoglyphidae such as the dried fruit mite (Carpoglyphus lactis); adults, larvae, and eggs of these and others;

wood-decaying fungi such as Tyromyces palustris and Coriolus versicolor;

material-deteriorating microorganisms such as Aspergillus niger, Aspergillus terreus, Aureobasidium pullulans, Chaetomium globosum, Cladosporium cladosporioides, Eurotium tonophilum, Fusarium moniliforme, Gliocladium virens, Myrothecium verrucaria, Penicillium citrinum, Penicillium funiculosum, Rhizopus oryzae, etc.

The compound of the present invention represented by the formula (1) can also be used to control pests that damage or weaken trees in natural forests, artificial forests, and urban green spaces. Specific pests include the following.

From the class Insecta of the phylum Arthropoda, the order Lepidoptera, for example, Lymantriidae such as the Japanese cedar tussock moth (Calliteara argentata), tea tussock moth (Euproctis pseudoconspersa), small white-spotted tussock moth (Orgyia recens approximans), tussock moth (Euproctis subflava), and spongy moth (Lymantria dispar); Lasiocampidae such as the lackey moth (Malacosoma neustria testacea), pine moth (Dendrolimus spectabilis), and Siberian silk moth (Dendrolimus superans); Pyralidae such as the larch knot-horn moth (Cryptoblabes loxiella); Noctuidae such as the turnip moth (Agrotis segetum); Tortricidae such as the larch thread-drawing leafroller (Ptycholoma lecheana circumclusana), chestnut fruit moth (Cydia kurokoi), and Japanese cedar cone moth (Cydia cryptomeriae); Arctiidae such as the mulberry tiger moth (Spilosoma imparilis) and fall webworm (Hyphantria cunea); Nepticulidae such as the Castanopsis leaf miner pygmy moth (Stigmella castanopsiella); Limacodidae such as the nettle caterpillar (Parasa lepida), small black slug moth (Scopelodes contracta), and long-palped slug moth (Microleon longipalpis); adults, larvae, and eggs of these and others; the order Coleoptera, for example, Scarabaeidae such as the small chafer (Anomala rufocuprea) and yellowish elongate chafer (Heptophylla picea); Buprestidae such as the zelkova jewel beetle (Agrilus spinipennis); Cerambycidae such as the Japanese pine sawyer (Monochamus alternatus); Chrysomelidae such as the Japanese cedar leaf beetle (Basilepta pallidula); Curculionidae such as the rusty gourd-shaped weevil (Scepticus griseus) and white-spotted pine weevil (Shirahoshizo insidiosus); Dryophthoridae such as the Japanese giant weevil (Sipalinus gigas); Scolytidae such as the pine shoot beetle (Tomicus piniperda) and maple bark beetle (Indocryphalus aceris); Bostrichidae such as the lesser grain borer (Rhyzopertha dominica); adults, larvae, and eggs of these and others;

the order Hemiptera, for example, Aphididae such as the todo-fir aphid (Cinara todocola); Adelgidae such as the spruce gall aphid (Adelges japonicus); Diaspididae such as the Cryptomeria scale (Aspidiotus cryptomeriae); Coccidae such as the Indian wax scale (Ceroplastes ceriferus); adults, larvae, and eggs of these and others;

the order Hymenoptera, for example, Tenthredinidae such as the larch red sawfly (Pachynematus itoi); Diprionidae such as the European pine sawfly (Neodiprion sertifer); Cynipidae such as the oriental chestnut gall wasp (Dryocosmus kuriphilus); adults, larvae, and eggs of these and others; the order Diptera, for example, Tipulidae such as the rice crane fly (Tipula aino); Anthomyiidae such as the larch cone fly (Strobilomyia laricicola); Cecidomyiidae such as the cryptomeria needle gall midge (Contarinia inouyei) and pine bud gall midge (Contarinia matsusintome); adults, larvae, and eggs of these and others;

from the class Arachnida of the phylum Arthropoda, the order Acari, for example, the sugi spider mite (Oligonychus hondoensis) and spruce spider mite (Oligonychus ununguis); adults, larvae, and eggs of these and others;

from the class Chromadorea of the phylum Nematoda, the order Tylenchida, for example, Parasitaphelenchidae such as the pine wood nematode (Bursaphelenchus xylophilus), etc.

The compound of the present invention represented by the formula (1) can also be used for preventing, treating, or controlling arthropods, nematodes, flukes, tapeworms, and protozoa that internally or externally parasitize vertebrates, particularly warm-blooded vertebrates such as cattle, sheep, goats, horses, pigs, poultry, dogs, cats, fish, and other livestock and pets. In addition to the above, the target animal species also include rodents such as mice, rats, hamsters, and squirrels; carnivores such as ferrets; and birds such as ducks and pigeons, which may be pets or laboratory animals. Specific pests include the following.

From the class Insecta of the phylum Arthropoda, the order Diptera, for example, Tabanidae such as the Yamato horsefly (Tabanus rufidens) and the red cattle horsefly (Tabanus chrysurus); Muscidae such as the black house fly (Musca bezzii), the housefly (Musca domestica), and the stable fly (Stomoxys calcitrans); Gasterophilidae such as the horse bot fly (Gasterophilus intestinalis); Hypodermatidae such as the cattle grub (Hypoderma bovis); Oestridae such as the sheep bot fly (Oestrus ovis); Calliphoridae such as the blow fly (Aldrichina grahami); Phoridae such as the large-spiracle scuttle fly (Megaselia spiracularis); Sepsidae such as the one-spotted sepsid (Sepsis punctum); Psychodidae such as the large drain fly (Telmatoscopus albipunctatus) and the spotted drain fly (Psychoda alternata); Culicidae such as the underground

house mosquito (Culex pipiens molestus), the red house mosquito (Culex pipiens pallens), the Chinese anopheles (Anopheles sinensis), the small red house mosquito (Culex pipiens triaeniorhynchus summorosus), and the Asian tiger mosquito (Aedes albopictus); Simuliidae such as the clawspined black fly (Simulium iwatense) and the yellow-legged large black fly (Prosimulium yezoense); Ceratopogonidae such as the cattle biting midge (Culicoides schulzei) and the chicken biting midge (Culicoides arakawae); adults, larvae, and eggs of these and others;

the order Siphonaptera, for example, Pulicidae such as the cat flea (Pulex irritans) and the dog flea (Ctenocephalides canis); adults, larvae, and eggs of these;
the order Phthiraptera, for example, Haematopinidae such as the hog louse (Haematopinidae suis) and short-nosed cattle louse (Haematopinidae eurysternus); Trichodectidae such as the horse biting louse (Damalinia bovis); Linognathidae such as the long-nosed cattle louse (Linognathus vituli); Menoponidae such as the shaft louse (Menopon gallinae); adults, larvae, and eggs of these and others;
from the class Arachnida of the phylum Arthropoda, the order Acari, for example, Varroidae such as the Varroa mite (Varroa jacobsoni); Ixodidae such as the long-homed tick (Haemaphysalis longicornis), Japanese hard tick (Ixodes ovatus), southern cattle tick (Boophilus microplus), and Amblyomma testudinarium; Macronyssidae such as the northern fowl mite (Ornithonyssus sylviarum); Dermanyssidae such as the poultry red mite (Dermanyssus gallinae); Demodicidae such as the pig follicle mite (Demodex phylloides); Sarcoptidae such as the cattle scab mite (Sarcoptes scabiei bovis) and scaly-leg mite (Knemidocoptes mutans); Psoroptidae such as the ear mite (Otodectes cynotis) and common scab mite (Psoroptes communis); adults, larvae, and eggs of these and others;
from the class Secernentea of the phylum Nematoda, the order Strongylida, for example, cattle hookworm, swine kidney worm, swine lungworm, threadworm, cattle nodular worm, etc.:
the order Ascaridida, for example, swine roundworm, chicken roundworm, etc.;
from the class Trematoda of the phylum Platyhelminthes, for example, Japanese blood fluke, liver fluke, deer paramphistome, Oriental lung fluke, Japanese poultry oviduct fluke, etc.;
the class Cestoda, for example, broad fish tapeworm, Moniezia expansa, Moniezia benedeni, Raillietina tetragona, Spirometra erinacei, Taenia solium, etc.:
from the class Zoomastigophora of the phylum Protozoa, the order Rhizomastigida, for example, Histomonas, etc.; the order Kinetoplastida, for example, Leishmania, Trypanosoma, etc.; the order Diplomonadida, for example, Giardia, etc.; the order Trichomonadida, for example, Trichomonas, etc.:
from the class Sarcodina, the order Amoebida, for example, Entamoeba, etc.;
from the class Sporozoa, the subclass Piroplasmia, for example, Theileria, Babesia, etc.; the subclass Coccidia, for example, Eimeria, Plasmodium, Toxoplasma, etc.

The compound of the present invention represented by the formula (1) can also be used to exterminate pests that cause direct harm or discomfort to humans, or to maintain public health conditions against pests that carry or transmit pathogens. Specific pests include the following.

From the class Insecta of the phylum Arthropoda, the order Lepidoptera, for example, Lymantriidae such as the yellow-tail moth (Sphrageidus similis); Lasiocampidae such as the oak lappet moth (Kunugia undans); Limacodidae such as the green slug caterpillar (Parasa consocia); Zygaenidae such as the bamboo moth (Artona martini); adults, larvae, and eggs of these and others;

the order Coleoptera, for example, Oedemeridae such as the green false blister beetle (Xanthochroa waterhousei); Meloidae such as the bean blister beetle (Epicauta gorhami); Staphylinidae such as the rove beetle (Paederus fuscipes); adults, larvae, and eggs of these and others;
the order Hymenoptera, for example, Vespidae such as the yellow hornet (Vespa simillima xanthoptera); Formicidae such as the Asian needle ant (Brachyponera chinensis); Pompilidae such as the yellow-banded spider wasp (Batozonellus annulatus); adults, larvae, and eggs of these and others;
the order Diptera, for example, Culicidae such as the large black bush mosquito (Armigeres subalbatus); Ceratopogonidae such as the Japanese biting midge (Culicoides nipponensis); Chironomidae such as the striped non-biting midge (Chironomus yoshimatsui); Simuliidae such as the large-legged mottled black fly (Simulium nikkoense); Tabanidae such as the blue-green algae horsefly (Hirosia humilis); Muscidae such as the housefly (Musca domestica); Fanniidae such as the lesser house fly (Fannia canicularis); Calliphoridae such as the black blow fly (Phormia regina); Sarcophagidae such as the gray flesh fly (Sarcophaga peregrina); adults, larvae, and eggs of these and others;
the order Siphonaptera, for example, Pulicidae such as the human flea (Pulex irritans); adults, larvae, and eggs of these and others;
the order Blattodea, for example, Ectobiidae such as the German cockroach (Blattella germanica); Blattidae such as the American cockroach (Periplaneta americana), smokybrown cockroach (Periplaneta fuliginosa), and Japanese

cockroach (Periplaneta japonica); adults, larvae, and eggs of these and others;
the order Orthoptera, for example, Rhaphidophoridae such as the mottled camel cricket (Diestrammena japonica) and camel cricket (Diestrammena apicalis); adults, larvae, and eggs of these and others;
the order Phthiraptera, for example, Pediculidae such as the head louse (Pediculus humanus humanus); Pthiridae such as the crab louse (Pthirus pubis); adults, larvae, and eggs of these and others;
the order Hemiptera, for example, Cimicidae such as the bed bug (Cimex lectularius); Reduviidae such as the large flying assassin bug (Isyndus obscurus); adults, larvae, and eggs of these and others;
from the class Entognatha of the phylum Arthropoda, the order Collembola, for example, Hypogastruridae such as the purple springtail (Hypogastrura communis); adults, larvae, and eggs of these and others;
from the class Arachnida of the phylum Arthropoda, the order Acari, for example, Ixodidae such as the taiga tick (Ixodes persulcatus); Macronyssidae such as the tropical rat mite (Ornithonyssus bacoti); Cheyletidae such as the southern cheyletid mite (Chelacaropsis moorei); Pyemotidae such as the straw itch mite (Pyemotes ventricosus); Demodicidae such as the follicle mite (Demodex folliculorum); Pyroglyphidae such as the European house dust mite (Dermatophagoides pteronyssinus); Sarcoptidae such as the itch mite (Sarcoptes scabiei); Trombiculidae such as the chigger mite (Trombicula akamushi); adults, larvae, and eggs of these and others;
the order Araneae, for example, Clubionidae such as the Japanese yellow sac spider (Cheiracanthium japonicum); Sparassidae such as the huntsman spider (Heteropoda venatoria); Pholcidae such as the cellar spider (Spermophora senoculata) and daddy longlegs spider (Pholcus phalangioides); Oecobiidae such as the flat spider (Uroctea compactilis); Salticidae such as the pantropical jumping spider (Plexippus paykulli) and Adanson's house jumper (Plexippus adansoni); adults, larvae, and eggs of these and others;
the order Scorpiones, for example, Buthidae such as the marbled scorpion (Isometrus europaeus); adults, larvae, and eggs of these and others;
from the class Chilopoda of the phylum Arthropoda, the order Scolopendromorpha, for example, Scolopendridae such as the Chinese red-headed centipede (Scolopendra subspinipes mutilans) and Japanese giant centipede (Scolopendra subspinipes japonica); adults, larvae, and eggs of these and others;
the order Scutigeromorpha, for example, Scutigeridae such as the house centipede (Thereuonema hilgendorfi); adults, larvae, and eggs of these and others;
from the class Diplopoda of the phylum Arthropoda, the order Polydesmida, for example, Paradoxosomatidae such as the greenhouse millipede (Oxidus gracilis); adults, larvae, and eggs of these and others;
from the class Crustacea of the phylum Arthropoda, the order Isopoda, for example, Porcellionidae such as the common rough woodlouse (Porcellio scaber); adults, larvae, and eggs of these and others;
from the class Hirudinea of the phylum Annelida, the order Arhynchobdellida, for example, Haemadipsidae such as the Japanese land leech (Haemadipsa zeylanica japonica), etc.;
fungi such as Trichophyton rubrum, Trichophyton mentagrophytes, which are dermatophytes; Candida albicans, which is a Candida fungus; Aspergillus fumigatus, which is an Aspergillus fungus; gram-negative bacteria such as Escherichia coli and Pseudomonas aeruginosa; and gram-positive bacteria such as Staphylococcus aureus.

The compound of the present invention represented by the formula (1) is particularly valuable for controlling pests that damage agricultural crops, trees in natural and artificial forests and urban green spaces, and ornamental plants, such as arthropods, gastropods, nematodes, and fungi. In such situations, the compound of the present invention can also be present as a mixture with other active compounds, for example, insecticides, acaricides, nematicides, fungicides, synergists, plant growth regulators, poison baits, or herbicides, in their commercially useful formulations and in the use forms prepared from these formulations.

As for the use forms, wettable powders, water-dispersible granules, dry flowables, water-soluble powders, emulsifiable concentrates, solutions, oil solutions, suspension concentrates, suspoemulsions, oil-based suspension formulations, capsules, dusts, granules, fine granules, baits, tablets, sprays, fogs, aerosols, etc., can be taken. To prepare these dosage forms, various agrochemical adjuvants conventionally used in the technical field of agricultural and horticultural chemical agents can be appropriately used. Such agrochemical adjuvants can be used for purposes such as improving the effect, stabilization, and dispersibility of the agricultural and horticultural chemical agent.

Examples of agrochemical adjuvants include carriers (diluents), emulsifiers, wetting agents, dispersing agents, and disintegrants. Examples of liquid carriers include water, aromatic hydrocarbons such as toluene and xylene, alcohols such as methanol, butanol, and glycol, ketones such as acetone, amides such as dimethylformamide, sulfoxides such as dimethyl sulfoxide, methylnaphthalene, cyclohexane, animal and vegetable oils, and fatty acids. As solid carriers, clay, kaolin, talc, diatomaceous earth, silica, calcium carbonate, montmorillonite, bentonite, feldspar, quartz, alumina, sawdust, nitrocellulose, starch, gum arabic, etc., can be used.

As emulsifiers and dispersing agents, ordinary surfactants can be used, for example, anionic surfactants such as sodium higher alcohol sulfate, stearyltrimethylammonium chloride, polyoxyethylene alkylphenyl ether, and lauryl betaine; cationic surfactants; nonionic surfactants; and amphoteric surfactants can be used.

Wetting agents such as dialkyl sulfosuccinates; binders such as carboxymethyl cellulose and polyvinyl alcohol; and disintegrants such as sodium ligninsulfonate and sodium lauryl sulfate can also be used.

Of course, one or a combination of two or more of the compounds of the present invention can also be blended as the active ingredient. The content of the compound of the present invention as an active ingredient in these formulations is, for example, 0.01 to 99.5% by mass, and is preferably selected from the range of 0.5 to 90% by mass, which may be appropriately determined depending on various conditions such as the formulation form and application method; for example, dusts can be manufactured to contain about 0.5 to 20% by mass, preferably 1 to 10% by mass of the active ingredient; wettable powders about 1 to 90% by mass, preferably 10 to 80% by mass of the active ingredient; and emulsifiable concentrates about 1 to 90% by mass, preferably 10 to 40% by mass of the active ingredient.

To control arthropods, gastropods, nematodes, and fungi, in addition to spraying on the stems and leaves of plants, the compound can be used by treating the soil, etc., and having it absorbed from the roots, in places where damage from these pests has occurred or is likely to occur; such methods include mixing into the entire soil layer, furrow application, mixing into nursery soil, cell seedling treatment, planting hole treatment, treatment at the base of the plant, top dressing, rice seedling box treatment, and application to the water surface. It can also be used by seed treatment such as dipping seeds in the chemical, seed coating, or calper treatment; application to the nutrient solution in hydroponic culture; or by fumigation, trunk injection, etc. When used, the amount varies depending on the type and abundance of the pest, and the type, cultivation form, and growth stage of the target crop or tree, but generally, 0.1 to 1000 g, preferably 1 to 100 g, of the active ingredient is applied per 10 ares. To carry out this treatment, wettable powders, water-dispersible granules, dry flowables, water-soluble powders, emulsifiable concentrates, solutions, suspension concentrates, suspoemulsions, oil-based suspension formulations, capsules, etc., are diluted with water and sprayed on crops, etc., at an application rate of generally 10 to 1000 liters per 10 ares, although this varies depending on the type, cultivation form, and growth stage of the target plant. For dusts, sprays, or aerosols, they may be applied to crops, etc., in their formulated state.

When the target pest primarily damages plants in the soil, or when controlling the target pest by having the chemical absorbed from the roots, application methods include, for example, applying the formulation diluted or undiluted with water to the base of the plant or to a nursery bed, etc.; applying granules to the base of the plant or to a nursery bed, etc.; applying dusts, wettable powders, water-dispersible granules, granules, etc., before sowing or transplanting and mixing them with the entire soil; and applying dusts, wettable powders, water-dispersible granules, granules, fine granules, etc., to planting holes, furrows, etc., before sowing or planting. Wettable powders, water-dispersible granules, water-soluble powders, emulsifiable concentrates, solutions, suspension concentrates, suspoemulsions, oil-based suspension formulations, capsules, etc., are diluted with water and generally applied at a rate of 5 to 500 liters per 10 ares, by spraying evenly over the entire treated area's soil surface or drenching into the soil; for dusts, granules, fine granules, baits, or the like, they are applied in their formulated state evenly over the entire treated area's soil surface. Spraying or drenching may be done around the seeds or crops/trees to be protected from damage. It is also possible to till during or after application to mechanically disperse the active ingredient.

As for the method of application to seedling boxes for paddy rice, the dosage form may vary depending on the application timing, such as application at sowing time, greening stage application, or transplanting time application, but for example, it may be applied in the form of a dust, water-dispersible granule, granule, fine granule, emulsifiable concentrate, suspension concentrate, or the like. It can also be applied by mixing with nursery soil; specifically, by mixing the nursery soil with dusts, water-dispersible granules, granules, or fine granules, etc.; for example, by mixing with bed soil, mixing with covering soil, or mixing throughout the entire nursery soil, etc. It is also possible to simply apply the nursery soil and various formulations in alternating layers.

As for the method of application to paddy fields, solid formulations such as jumbo packs, pack formulations, granules, and water-dispersible granules, and liquid formulations such as emulsifiable concentrates and suspension concentrates are usually applied to the paddy field in a flooded state. In addition, at the time of rice planting, a suitable formulation can be applied directly or mixed with fertilizer or the like and applied to or injected into the soil. By utilizing a chemical solution such as an emulsifiable concentrate or suspension concentrate at the water inlet of the paddy field, such as a water gate or irrigation system, it can also be applied labor-savingly with the supply of water.

As for seed treatment methods, examples include a method of immersing seeds in a liquid or solid formulation, diluted or undiluted, in a liquid state to adhere and penetrate the chemical; a method of mixing a solid or liquid formulation with seeds for coating treatment to adhere it to the seed surface; a method of coating the seeds by mixing with an adhesive carrier such as resin or polymer; and a method of applying it near the seeds simultaneously with planting. The "seed" to be subjected to the seed treatment means a plant body at the early stage of cultivation used for plant propagation, and includes, for example, in addition to seeds, bulbs, tubers, seed potatoes, basal buds, bulbils, true bulbs, or plant bodies for vegetative propagation for cutting cultivation. The "soil" or "growing medium" of the plant for application refers to a support for growing crops, particularly a support for rooting, and the material is not particularly limited as long as it is a material in which plants can grow; it may be so-called soil, nursery mats, water, etc., and specific materials include, for example, sand, pumice, vermiculite, diatomaceous earth, agar, gel-like substances, high-molecular-weight substances, rock wool, glass wool, wood chips, and bark.

For the treatment during sowing and nursery periods of cultivated plants to be transplanted, in addition to direct treatment of seeds, drenching of the liquid chemical or application of granules to the nursery bed is preferable. It is also a preferable treatment to apply granules to planting holes at the time of transplanting, or to mix them into the growing medium near the transplanting site.

The compound of the present invention represented by the formula (1) is also valuable for protecting wood (standing trees, fallen trees, processed wood, stored wood, or structural wood) from damage by insects such as termites or beetles, and fungi. In such situations, control can be achieved by methods such as spraying, injecting, drenching, or coating the wood, its surrounding soil, or the like with oil solutions, emulsions, wettable powders, or suspension concentrates, or by applying dusts, granules, etc. The oil solutions, emulsions, wettable powders, dusts, etc., used in this situation can also exist as a mixture with other active compounds, such as insecticides, acaricides, nematicides, fungicides, repellents, or synergists; these formulations may contain the active ingredient compound in a total amount of 0.0001 to 95% by mass, preferably 0.005 to 10% by mass for oil solutions, dusts, and granules, and 0.01 to 50% by mass for emulsions, wettable powders, and suspension concentrates. When controlling arthropods, fungi, or the like, 0.01 to 100 g of the active ingredient compound per 1 $m^2$ is applied to the soil or wood surface.

The compound of the present invention represented by the formula (1) can be used to protect products such as grains, fruits, nuts, spices, and tobacco in their whole, powdered, or incorporated state during storage from damage by Lepidoptera, Coleoptera, Acari, fungi, and the like. It can also protect animal products (hides, fur, wool, feathers, etc.) and plant products (cotton, paper, etc.) in their natural or converted state during storage from attack by Lepidoptera, Coleoptera, Zygentoma, and cockroaches, and further protect foods such as meat and fish during storage from attack by Lepidoptera, Coleoptera, Diptera, and Acari. In such situations, control can be achieved by methods such as spraying oil solutions, emulsions, wettable powders, dusts, etc.; installing resin vaporizers, etc.; treating with fumigants or fogs; placing granules, tablets, and poison baits; or spraying aerosols. These formulations can also exist as a mixture with other active compounds, such as insecticides, acaricides, nematicides, fungicides, repellents, or synergists, and these formulations may contain the active ingredient compound in a total amount of 0.0001 to 95% by mass.

The compound of the present invention represented by the formula (1) is valuable for exterminating or preventing arthropods that parasitize the body surface of humans and livestock and cause direct harm such as skin feeding or blood-sucking, fungi, arthropods, nematodes, flukes, tapeworms, and protozoa that spread diseases of humans and livestock or are vectors of such diseases, and arthropods that cause discomfort to humans. In such situations, the compound of the present invention can be orally administered by mixing a small amount into meals, feed, or the like, or as a suitable orally ingestible pharmaceutical composition or the like, for example, as tablets, pills, capsules, pastes, gels, beverages, medicated feed, medicated drinking water, medicated supplements, sustained-release boluses, or other sustained-release devices designed to be retained in the gastrointestinal tract, containing a pharmaceutically acceptable carrier or coating material; it can also be transdermally administered as a spray, powder, grease, cream, ointment, emulsion, lotion, spot-on, pour-on, shampoo, etc. To achieve efficacy in such applications, the formulation generally contains 0.0001 to 0.1% by mass, preferably 0.001 to 0.01% by mass of the active ingredient compound. For transdermal or topical administration, devices attached to the animal to control arthropods locally or systemically (e.g., collars, medallions, ear tags, etc.) can also be used.

Specific oral and transdermal administration methods for using the compound of the present invention represented by the formula (1) as an anthelmintic for animals such as livestock and pets, or for humans are shown, but the methods are not necessarily limited to these.

When administered orally as medicated drinking water, the beverage is usually a solution, suspension, or dispersion in a suitable non-toxic solvent or water, together with a suspending agent such as bentonite, a wetting agent, or other excipients. The beverage generally also contains an antifoaming agent. Beverage formulations generally contain 0.01 to 1.0% by mass, preferably 0.01 to 0.1% by mass of the active ingredient compound.

When it is desirable to administer orally in a dry, individual unit dosage form, capsules, pills, or tablets containing a predetermined amount of the active ingredient are usually used. These dosage forms are prepared by appropriately pulverizing the active ingredient and homogeneously mixing it with diluents, fillers, disintegrants, and/or binders such as starch, lactose, talc, magnesium stearate, vegetable gums, and the like. The weight and content of the anthelmintic in such unit dosage formulations can be widely varied depending on the type of host animal being treated, the degree of infection, the type of parasite, and the weight of the host.

When administered via animal feed, it can be homogeneously dispersed in the feed, used as a top dressing, or used in the form of pellets. To achieve the desired antiparasitic effect, the final feed usually contains 0.0001 to 0.05% by mass, preferably 0.0005 to 0.01% by mass of the active ingredient compound.

A solution or dispersion in a liquid carrier excipient can be administered to the animal parenterally by intra-ruminal, intramuscular, intratracheal, or subcutaneous injection. For parenteral administration, the active compound is preferably mixed with a suitable vegetable oil such as peanut oil or cottonseed oil. Such formulations are generally made to contain 0.05 to 50% by mass, preferably 0.1 to 5.0% by mass of the active ingredient compound.

It can also be administered topically by mixing with a suitable carrier such as dimethyl sulfoxide or a hydrocarbon

solvent. This formulation is applied directly to the external surface of the animal by spraying or direct pouring.

The compound of the present invention represented by the formula (1) can also be used as an anthelmintic against arthropods and the like that cause direct harm or are disease vectors, by applying it to the surrounding environment where these pests may be present; methods include spraying, injecting, drenching, or coating with oil solutions, emulsions, wettable powders, etc.; applying dusts; fumigation; heated fumigants such as mosquito coils, self-burning fumigants, and chemical reaction-type fogs; smoke agents such as fogging; ULV agents; placing granules, tablets, and poison baits; or adding floating powders, granules, etc., dropwise to waterways, wells, reservoirs, water tanks, and other running or standing water. Furthermore, against pests that are also agricultural and forest pests, such as tussock moths, control is possible in the same manner as described above; against pests such as flies, a method of mixing it into livestock feed so that it is mixed with feces, and against pests such as mosquitoes, a method of vaporizing it into the air with an electric mosquito repellent, etc., are also effective. The formulations in these use forms can also exist as a mixture with other active compounds, such as pest control agents, mite control agents, nematode control agents, disease control agents, repellents, or synergists, and these formulations contain the active ingredient compound in a total amount of 0.0001 to 95% by mass.

The compound of the present invention represented by the formula (1) can also be used as a mixture in combination with other active compounds. In particular, by using it in a mixture with a compound having pest control activity, mite control activity, or nematode control activity (insecticide), it becomes possible to expand the range of target pests for the control of arthropods, gastropods, nematodes, and other pests that damage plants, and synergistic effects and the like such as a reduction in the amount of chemical can also be expected. Specific examples of active compounds include the following.

Organophosphates, for example, acephate, azinphos-methyl, chlorpyrifos, diazinon, dichlorvos, dimeton-S-methyl, dimethoate, dimethylvinphos, disulfoton, ethion, fenitrothion, fenthion, isoxathion, malathion, methamidophos, methidathion, monocrotophos, naled, oxydeprofos, parathion, phenthoate, phosalone, pirimiphos-methyl, pyridafenthion, profenofos, prothiophos, propaphos, pyraclofos, salithion, sulprofos, thiometon, tetrachlorvinphos, trichlorfon, vamidothion, etc.;

carbamates, for example, alanycarb, bendiocarb, benfuracarb, carbaryl, carbofuran, carbosulfan, ethiofencarb, fenobucarb, furathiocarb, isoprocarb, methomyl, metolcarb, pirimicarb, propoxur, thiodicarb, etc.;
organochlorines, for example, aldrin, chlordane, DDT (p,p'-DDT), endosulfan, lindane, etc.;
pyrethroids, for example, acrinathrin, allethrin, bifenthrin, cycloprothrin, cyfluthrin, cyhalothrin, cyphenothrin, cypermethrin, deltamethrin, ethofenprox, fenpropathrin, fenvalerate, flucythrinate, flufenprox, fluvalinate, furamethrin, halfenprox, imiprothrin, permethrin, phenothrin, prallethrin, pyrethrins, resmethrin, silafluofen, tefluthrin, tralomethrin, transfluthrin, etc.;
neonicotinoids, for example, acetamiprid, clothianidin, dinotefuran, imidacloprid, nitenpyram, thiacloprid, thiamethoxam, etc.;
diamides, for example, chlorantraniliprole, cyantraniliprole, cyclaniliprole, flubendiamide, tetraniliprole, etc.;
phenylpyrazoles, for example, ethiprole, fipronil, acetoprole, pyrafluprole, pyriprole, etc.;
nereistoxin agents, for example, bensultap, cartap, thiocyclam, thiosultap, etc.;
insect growth regulators such as phenylbenzoylureas and diacylhydrazines, for example, chlorfluazuron, diflubenzuron, flufenoxuron, hexaflumuron, lufenuron, novaluron, teflubenzuron, triflumuron, buprofezin, chromafenozide, halofenozide, methoxyfenozide, tebufenozide, cyromazine, etc.;
juvenile hormone agents, for example, diofenolan, fenoxycarb, hydroprene, methoprene, pyriproxyfen, etc.;
insecticidal substances produced by microorganisms, etc., for example, abamectin, emamectin-benzoate, ivermectin, lepimectin, milbemectin, nemadectin, Nikkomycin, polyoxin complex, spinetoram, spinosad, BT agents, etc.;
insecticidal substances derived from natural products, etc., for example, anabasine, azadirachtin, deguelin, fatty acid glycerides (decanoyloctanoylglycerol), hydroxypropyl starch, soy lecithin, nicotine, nornicotine, sodium oleate (oreic acid sodium salt), petroleum oil, propylene glycol mono-fatty acid ester (propylene glycol monolaurate), rape oil, rotenone, sorbitan fatty acid ester, starch, etc.;
other insecticides, for example, afidopyropen, benzpyrimoxan, broflanilide, chlorfenapyr, diafenthiuron, dicloromezotiaz, dimpropyridaz, DBEDC (Dodecylbenzenesulfonic acid bisethylenediamine copper [II] salt), flonicamid, flometoquin, flufenerim, flupyradifurone, flupyrimin, fluralaner, fluhexafon, fluxametamide, hydramethylnon, indoxacarb, isocycloseram, metaflumizone, metaldehyde, nicotine sulfate, oxazosulfyl, pymetrozine, pyridalyl, pyrifluquinazon, spirotetramat, sulfoxaflor, tolfenpyrad, triazamate, triflumezopyrim, tyclopyrazoflor, etc.;
acaricides, for example, acequinocyl, acynonapyr, amidoflumet, amitraz, azocyclotin, benzoximate, bifenazate, binapacryl, bromopropylate, chinomethionat, clofentezine, cyenopyrafen, cyflumetofen, cyhexatin, dicofol, dienochlor, etoxazole, fenazaflor, fenazaquin, fenbutatin oxide, fenothiocarb, fenpyroximate, fluacrypyrim, hexythiazox, pyrimidifen, polynactins, propargite, pyflubumide, pyridaben, spirodiclofen, spiropidion, spiromesifen, tebufenpyrad, tetradifon, and flupentiofenox, etc.:

nematicides, for example, aluminium phosphide, benclothiaz, cadusafos, ethoprophos, fluazaindolizine, fluensulfone, fosthiazate, furfural, imicyafos, levamisole hydrochloride, mesulfenfos, metam-ammonium, methyl isocyanate (methyl isothiocyanate), morantel tartrate, oxamyl, tioxazafen, etc.;
poison baits, for example, chlorophacinone, coumatetralyl, diphacinone, sodium fluoroacetate, warfarin, etc., can be cited.

The compound of the present invention represented by the formula (1) can also be used as a mixture with other active compounds other than compounds having pest control activity, mite control activity, or nematode control activity. By using it in a mixture with a compound having fungicidal activity, herbicidal activity, or plant growth regulating activity to control diseases and/or weeds that occur simultaneously at the time of use, synergistic effects and the like such as a reduction in control labor and a reduction in the amount of chemical can also be expected. By using it in a mixture with a repellent, a synergist, or the like, a more effective control effect such as a synergistic effect can be expected.
Specific examples of active compounds include disease control agents, for example, D-D (1,3-dichloropropene), acibenzolar-S-methyl, aldimorph, ametoctradin, amisulbrom, andoprim, triazin (anilazine), azaconazole, azoxystrobin, basic copper sulfate, benodanil, benomyl, benthiavalicarb-isopropyl, benthiazole, bitertanol, bixafen, blasticidin S, boscalid, bromuconazole, calcium carbonate, buthiobate, calcium polysulfide, captafol, captan, carbendazim, carboxin, carpropamid, chinomethionat, chlorfenazole, chloroneb, chloropicrin, chlorothalonil, chlozolinate, DBEDC (complex of bis(ethylenediamine)copper-bis-(dodecylbenzenesulfonic acid)),

copper hydroxide, copper nonylphenol sulfonate, copper oxychloride, cyazofamid, cyflufenamid, cymoxanil, cyproconazole, cyprodinil, dazomet, diclobutrazol, dichlofluanid, dichlone, diclocymet, diclomezine, diethofencarb, difenoconazole, diflumetorim, dimethomorph, dimoxystrobin, diniconazole, diniconazole-M, dithane-stainless, dithianon, dodine, echlomezole, edifenphos, enestrobin, epoxiconazole, etaconazole, ethaboxam, extract from mushroom, famoxadone, fenamidone, fenarimol, fenbuconazole, fenfuran, fenhexamid,
fenoxanil, fenpiclonil, fenpropidin, fenpyrazamine, ferimzone, fluazinam, flumetover, flumorph, fluopicolide, fluopyram, fluoroimide, fluotrimazole, fluoxastrobin, fluquinconazole, flusulfamide, flutolanil, fluxapyroxad, folpet, fosetyl-AL, fthalide, fuberidazole, fludioxonil, flusilazole, flutianil, flutriafol, furametpyr, fluconazole, guazatine, hexaconazole, hydroxyisoxazole, hymexazol, imazalil, imazalil sulfate, imibenconazole, iminoctadine acetate, iminoctadine-DBS, ipconazole,
IBP (iprobenfos), iprodione, iprovalicarb, isofetamid, isoprothiolane, isopyrazam, isotianil, kasugamycin, kresoxim-methyl, mancozeb, mandipropamid, maneb, manzeb, mepanipyrim, mepronil, metalaxyl, carbam (metam-ammonium), carbam sodium (metamsodium), metconazole, methasulfocarb, methyl bromide, methyl isothiocyanate, metominostrobin, metrafenone, mildiomycin, myclobutanil, nickel dimethyldithiocarbamate, nuarimol, orysastrobin, oxadixyl, oxathiapiprolin, oxine-copper, oxolinic acid, oxpoconazole fumarate, oxycarboxin, oxytetracycline, pebulate, pefurazoate, penconazole, pencycuron, penflufen, penthiopyrad, picarbutrazox, picoxystrobin,
piperalin, polycarbamate, polyoxin-B, polyoxins, potassium hydrogen carbonate, probenazole, prochloraz, procymidone, propamocarb, propiconazole, propineb, proquinazid, prothioconazole, pyraclostrobin, pyrametostrobin, pyraoxystrobin, pyrazophos, pyribencarb, pyridinitril, pyrifenox, pyrimethanil, pyriofenone, pyroquilon, quinoxyfen, quintozene, sedaxane, silver, simeconazole, sodium hydrogen carbonate, sodium hypochlorite, spiroxamine, streptomycin, sulfur, tebufloquin, tebuconazole, tecloftalam, terbinafine, tetraconazole, thiabendazole, thiadiazin, thifluzamide, thiophanate, thiophanate-methyl, thiram, tiadinil, tolclofos-methyl, tolprocarb, tolylfluanid, triadimefon, triadimenol, triclopyricarb, tricyclazole, tridemorph, trifloxystrobin, triflumizole, triforine, triticonazole, uniconazole-P, validamycin(-A), vinclozolin, zarilamid, zinc sulfate, zineb, ziram, zoxamide, etc.

As a compound having herbicidal activity, for example, aclonifen, acifluorfensodium, alachlor, alloxydim, amicarbazone, amidosulfuron, anilofos, asulam, atrazine, azimsulfuron, benfuresate, bensulfuron-methyl, bentazone, benthiocarb, benzobicyclon, benzofenap, bialaphos, bifenox, bromobutide, bromoxynil, butamifos, cafenstrole, calcium peroxide, carbetamide, cinosulfuron, clomeprop, cyclosulfamuron, cyhalofop-butyl, daimuron, desmedipham, diclofop-methyl, diflufenican, dimefuron, dimethametryn, dinoterb, diquat, diuron, esprocarb, ethiozin, ethofumesate, ethoxysulfuron, etobenzanid, fenoxaprop-P-ethyl, fentrazamide, flucarbazone, flufenacet, flurtamone, fluthiacet-methyl, foramsulfuron, glufosinate-ammonium, glyphosate-isopropyl amine, glyphosate-trimesium, imazapyr, imazosulfuron, indanofan,
iodosulfuron, ioxynil-octanoate, isoproturon, isoxadifen, isoxaflutole, lactofen, linuron, mefenacet, mesosulfuron, metamitron, methabenzthiazuron, metosulam, metribuzin, napropamide, neburon, oxadiargyl, oxadiazon, oxaziclomefone, paraquat, pendimethalin, pentoxazone, phenmedipham, pretilachlor, propoxycarbazone, prosulfocarb, pyraclonil, pyraflufen-ethyl, pyrazolate, pyrazosulfuron-ethyl, pyributicarb, pyriftalid, pyriminobacmethyl, quizalofop-ethyl, sethoxydim, simazine, sulcotrione, sulfentrazone, thenylchlor, triaziflam, tribufos and the like can be exemplified.
As a compound having plant growth regulating activity, for example, 1-naphthylacetic acid, 4-CPA, 6-benzylaminopurine, butralin, calcium chloride, calcium formate, calcium peroxide, calcium sulfate, chlormequat chloride, choline, cyanamide, cyclanilide, daminozide, decyl alcohol, dichlorprop, ethephon, ethychlozate, flurprimidol, forchlorfenuron, gibberellic acid, indolebutyric acid, maleic hydrazide potassium salt, mefenpyr, mepiquat chloride, oxine sulfate (8-hydroxyquinoline sulfate), paclobutrazol, paraffin, prohexadione-calcium, prohydrojasmon, thidiazuron, trinexapac-ethyl, uniconazole-P, wax, and the like can also be used in mixture.

Repellents, for example, capsaicin, carane-3,4-diol, citronellal, DEET, dimethyl phthalate, hinokitiol, limonene, linalool, menthol, menthone, naphthalene, thiram, etc.; synergists, for example, methylenedioxynaphthalene, naphthyl propynyl ester (naphthyl propynyl ether), nitrobenzyl thiocyanate, octachlorodipropyl ester (octachlorodipropyl ether), pentynyl phthalimide, phenyl salioxon, piperonyl butoxide, safrole, sesamex, sesamin, sulfoxide, triphenyl phosphate, verbutin, etc. can be mentioned.

The compound of the present invention can be used in combination with biological pesticides, for example, viral preparations such as Cytoplasmic polyhedrosis virus (CPV), Entomopox virus (EPV), Granulosis virus (GV), and Nuclear polyhedrosis virus (NPV); microbial pesticides used as insecticides or nematicides such as Beauveria bassiana, Beauveria brongniartii, Monacrosporium phymatophagum, Paecilomyces fumosoroseus, Pasteuria penetrans, Steinemema carpocapsae, Steinemema glaseri, Steinernema kushidai, and Verticillium lecanii; microbial pesticides used as fungicides such as Agrobacterium radiobacter, Bacillus subtilis, non-pathogenic Erwinia carotovora, non-pathogenic Fusarium oxysporum, Pseudomonas CAB-02, Pseudomonas fluorescens, Talaromyces flavus, Trichoderma atroviride, and Trichoderma lignorum; and biological pesticides used as herbicides such as Xanthomonas campestris; thereby, similar effects can be expected when used in combination.

Furthermore, it is also possible to use in combination with biological pesticides, for example, natural enemy organisms such as Amblyseius californicus, Amblyseius cucumeris, Amblyseius degenerans, Aphidius colemani, Aphidoletes aphidimyza, Chrysoperla carnea, Dacnusa sibirica, Diglyphus isaea, Encarsia formosa, Eretmocerus eremicus, Franklinothrips vespiformis, Harmonia axyridis, Hemiptarsenus varicornis, Neochrysocharis formosa, Orius sauteri, Orius strigicollis, Phytoseiulus persimilis, Pilophorus typicus, and Piocoris varius; and pheromone agents such as codlelure, cuelure, geraniol, gyptol, liblure, looplure, methyl eugenol, orfralure, peachflure, phycilure, pyrimalure, and turpentine.

Examples

Hereinafter, the present invention will be described in more detail with reference to Examples, Formulation Examples, and Test Examples, but the scope of the present invention is not limited in any way by these Examples, Formulation Examples, and Test Examples.

Example 1

Synthesis of N'-(1-(3-fluorophenyl)-2-methyl-3-(3-(trifluoromethyl)-1H-1,2,4-triazol-1-yl)propylidene)formohydrazide (Compound No.: A-121) represented by the following formula

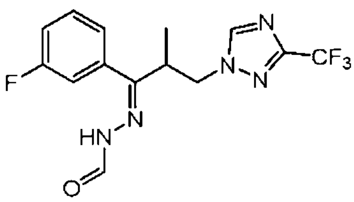

A－１２１

1-1: Synthesis of 1-(3-fluorophenyl)-3-hydroxy-2-methylpropan-1-one (Compound No. D-8) represented by the following formula

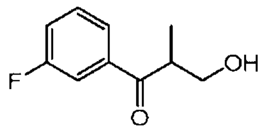

D－８

3'-Fluoropropiophenone (3.1 g) was diluted with DMF (30 mL), a 37% aqueous formaldehyde solution (1.94 g) was added, potassium carbonate (0.3 g) was added at room temperature, and after stirring, the mixture was allowed to

stand overnight. Water and ethyl acetate were added to the reaction solution, the layers were separated, and the organic layer was washed with water and brine; after drying over magnesium sulfate, the ethyl acetate was distilled off under reduced pressure, and the resulting crude product was purified by column chromatography to obtain 2.2 g of 1-(3-fluorophenyl)-3-hydroxy-2-methylpropan-1-one as compound (D-8) (oily substance).

1-2: Synthesis of 3-chloro-1-(3-fluorophenyl)-2-methylpropan-1-one (Compound No.: E-8) represented by the following formula

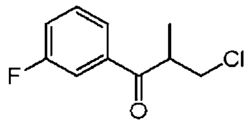

E－8

1-(3-Fluorophenyl)-3-hydroxy-2-methylpropan-1-one (D-8) (2.1 g) was diluted with toluene (20 mL), thionyl chloride (1.6 g) and DMF (2 drops) were added, and the mixture was stirred at 60°C for 2 hours; thereafter, excess thionyl chloride and toluene were distilled off from the reaction solution. Ethyl acetate and an aqueous sodium bicarbonate solution were added to the residue, the layers were separated, washed with brine, and dried over magnesium sulfate; the ethyl acetate was then distilled off under reduced pressure to obtain 2.3 g of 3-chloro-1-(3-fluorophenyl)-2-methylpropan-1-one as compound (E-8) (oily substance).

1-3: Synthesis of 1-(3-fluorophenyl)-2-methyl-3-(3-(trifluoromethyl)-1H-1,2,4-triazol-1-yl)propan-1-one (Compound No.: C-10) represented by the following formula

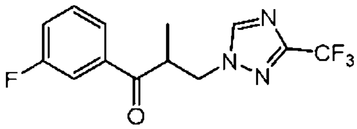

C－1 0

3-Chloro-1-(3-fluorophenyl)-2-methylpropan-1-one (E-8) (2.2 g) was diluted with DMF (20 ml), 3-(trifluoromethyl)-1,2,4-triazole (1.7 g) was added, potassium carbonate (1.80 g, 13 mmol) was added under ice cooling, stirred at room temperature for 6 hours, and allowed to stand overnight. After completion of the reaction, ethyl acetate and water were added to the reaction solution, and the layers were separated. The organic layer was washed with water and brine, dried over magnesium sulfate, and then the ethyl acetate was distilled off under reduced pressure; the resulting crude product was purified by column chromatography to obtain 2.0 g of 1-(3-fluorophenyl)-2-methyl-3-(3-(trifluoromethyl)-1H-1,2,4-triazol-1-yl)propan-1-one as compound (C-10) (oily substance).

1-4: Synthesis of N'-(1-(3-fluorophenyl)-2-methyl-3-(3-(trifluoromethyl)-1H-1,2,4-triazol-1-yl)propylidene)formohydrazide (Compound No.: A-121) represented by the following formula

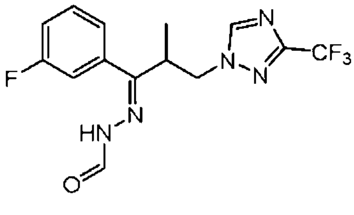

A－1 2 1

1-(3-Fluorophenyl)-2-methyl-3-(3-(trifluoromethyl)-1H-1,2,4-triazol-1-yl)propan-1-one (C-10) (1.0 g) and acetic acid (91 μL) were dissolved in ethanol (10 mL), and hydrazine monohydrate (617 μL) was added thereto. After heating under reflux for 7 hours, water was added, the mixture was extracted with ethyl acetate, and washed with saturated brine. After drying over sodium sulfate and concentrating, 924 mg of 1-(3-(3-fluorophenyl)-3-hydrazinylidene-2-methylpropyl)-3-(trifluoromethyl)-1H-1,2,4-triazole (B-10) was obtained as a residue. Without purification, 724 mg of this compound

was dissolved in ethyl formate (10 mL), and formic acid (33 μL) was added thereto. After stirring overnight at room temperature, water was added, the mixture was extracted with ethyl acetate, and washed with saturated brine. After drying over sodium sulfate and concentrating, the residue was purified by silica gel chromatography to obtain 341 mg of N'-(1-(3-fluorophenyl)-2-methyl-3-(3-(trifluoromethyl)-1H-1,2,4-triazol-1-yl)propylidene)formohydrazide as compound A-121 (oily substance).

Example 2

Synthesis of N'-(1-(3-fluorophenyl)-2-methyl-3-(3-(trifluoromethyl)-1H-1,2,4-triazol-1-yl)propylidene)-N,N-dimethylformohydrazonamide (Compound No.: A-125) represented by the following formula

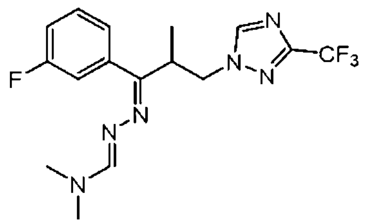

A-125

1-(3-(3-Fluorophenyl)-3-hydrazinylidene-2-methylpropyl)-3-(trifluoromethyl)-1H-1,2,4-triazole (B-10) (187 mg) was dissolved in toluene (5 mL), N,N-dimethylformamide dimethyl acetal (228 μL) was added, and the reaction solution was heated under reflux for 3.5 hours. The organic solvent was distilled off under reduced pressure, and the residue was purified by silica gel column chromatography to obtain 80 mg of N'-(1-(3-fluorophenyl)-2-methyl-3-(3-(trifluoromethyl)-1H-1,2,4-triazol-1-yl)propylidene)-N,N-dimethylformohydrazonamide as compound A-125 (oily substance).

Example 3

Synthesis of N'-(1-(4-chlorophenyl)-2,2-dimethyl-3-(3-(trifluoromethyl)-1H-1,2,4-triazol-1-yl)propylidene)formohydrazide (Compound No.: A-295) represented by the following formula

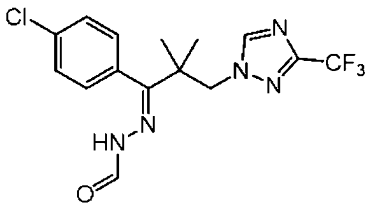

A-295

3-1: Synthesis of 1-(4-chlorophenyl)-3-hydroxy-2,2-dimethylpropan-1-one (Compound No.: D-19) represented by the following formula

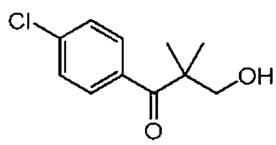

D-19

4'-Chloroisobutyrophenone (3.2 g) was dissolved in trifluoroacetic acid (15 mL), paraformaldehyde (1.1 g) was added thereto, and the mixture was heated under reflux for 14 hours. Thereafter, a saturated aqueous sodium bicarbonate solution (20 mL), a 48% aqueous sodium hydroxide solution (4.5 mL), and ethanol (5 mL) were added under ice cooling. After stirring at room temperature for 6 hours, the mixture was extracted with ethyl acetate and washed with saturated brine. After drying over sodium sulfate and concentrating, the residue was purified by silica gel chromatography to obtain

3.3 g of 1-(4-chlorophenyl)-3-hydroxy-2,2-dimethylpropan-1-one as compound D-19 (oily substance).

3-2: Synthesis of 3-(4-chlorophenyl)-2,2-dimethyl-3-oxopropyl 4-methylbenzenesulfonate (Compound No.: E-15) represented by the following formula

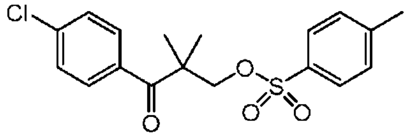

E－15

1-(4-Chlorophenyl)-3-hydroxy-2,2-dimethylpropan-1-one (D-19) (3.3 g) was dissolved in pyridine (15 mL), tosyl chloride (5.9 g) was added thereto under ice cooling, and the mixture was stirred at 60°C for 5.5 hours. Thereafter, IN hydrochloric acid was added, the mixture was extracted with ethyl acetate, and washed with saturated brine. After drying over sodium sulfate and concentrating, 4.2 g of 3-(4-chlorophenyl)-2,2-dimethyl-3-oxopropyl 4-methylbenzenesulfonate was obtained as compound E-15 (solid).

3-3: Synthesis of 1-(4-chlorophenyl)-2,2-dimethyl-3-(3-(trifluoromethyl)-1H-1,2,4-triazol-1-yl)propan-1-one (Compound No.: C-26) represented by the following formula

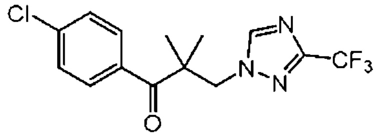

C－26

In a reaction vessel for microwave synthesis, 3-(4-chlorophenyl)-2,2-dimethyl-3-oxopropyl 4-methylbenzenesulfonate (E-15) (2.1 g), 3-(trifluoromethyl)-1,2,4-triazole (1.6 g), potassium carbonate (1.2 g), and N,N-dimethylformamide (15 mL) were added, irradiated with microwaves, and reacted at 120°C for 2 hours. Thereafter, water was added, the mixture was extracted with ethyl acetate, and washed with saturated brine. After drying over sodium sulfate and concentrating, the residue was purified by silica gel chromatography to obtain 1.8 g of 1-(4-chlorophenyl)-2,2-dimethyl-3-(3-(trifluoromethyl)-1H-1,2,4-triazol-1-yl)propan-1-one as compound (C-26) (oily substance).

3-4: Synthesis of N'-(1-(4-chlorophenyl)-2,2-dimethyl-3-(3-(trifluoromethyl)-1H-1,2,4-triazol-1-yl)propylidene)formohydrazide (Compound No.: A-295) represented by the following formula

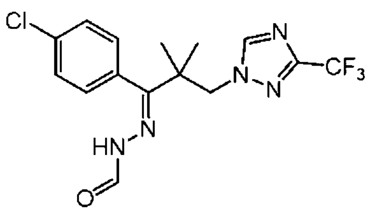

A－295

1-(4-Chlorophenyl)-2,2-dimethyl-3-(3-(trifluoromethyl)-1H-1,2,4-triazol-1-yl)propan-1-one (C-26) (1.3 g) and acetic acid (118 μL) were dissolved in ethanol (15 mL), and hydrazine monohydrate (762 μL) was added thereto. After heating under reflux for 7 hours, water was added, the mixture was extracted with ethyl acetate, and washed with saturated brine. After drying over sodium sulfate and concentrating, 1.5 g of 1-(3-(4-chlorophenyl)-3-hydrazinylidene-2,2-dimethylpropyl)-3-(trifluoromethyl)-1H-1,2,4-triazole (B-26) was obtained as a residue. Without purification, 1.2 g of this compound was dissolved in ethyl formate (10 mL), and formic acid (53 μL) was added thereto. After stirring overnight at room temperature, water was added, the mixture was extracted with ethyl acetate, and washed with saturated brine. After drying over sodium sulfate and concentrating, the residue was purified by silica gel chromatography to obtain 662 mg of N'-(1-(4-chlorophenyl)-2,2-dimethyl-3-(3-(trifluoromethyl)-1H-1,2,4-triazol-1-yl)propylidene)formohydrazide as compound A-295

(m.p. 120-121°C).

Example 4

Synthesis of N'-(1-(4-fluorophenyl)-2,2-dimethyl-3-(3-(trifluoromethyl)-1H-1,2,4-triazol-1-yl)propylidene)-N-(2-propynyl)formohydrazide (Compound No.: A-298) represented by the following formula

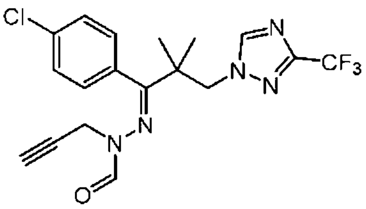

A－２９８

N'-(1-(4-Chlorophenyl)-2,2-dimethyl-3-(3-(trifluoromethyl)-1H-1,2,4-triazol-1-yl)propylidene)formohydrazide (Compound No.: A-295) (100 mg) was dissolved in N,N-dimethylformamide (2 mL), potassium carbonate (50 mg) was added, the mixture was stirred, propargyl bromide (44 μL) was added at room temperature, and the mixture was stirred overnight at room temperature. Ethyl acetate and water were added to the reaction solution, the organic layer was separated, and the organic layer was further washed with water. After drying the organic layer with $MgSO_4$, the organic solvent was distilled off under reduced pressure, and the residue was purified by silica gel column chromatography to obtain 111 mg of N'-(1-(4-fluorophenyl)-2,2-dimethyl-3-(3-(trifluoromethyl)-1H-1,2,4-triazol-1-yl)propylidene)-N-(2-propynyl)formohydrazide as compound A-298 (oily substance).

Example 5

Synthesis of N'-(1-(4-chlorophenyl)-2,2-dimethyl-3-(3-(trifluoromethyl)-1H-1,2,4-triazol-1-yl)propylidene)-N,N-dimethylformohydrazonamide (Compound No.: A-299) represented by the following formula

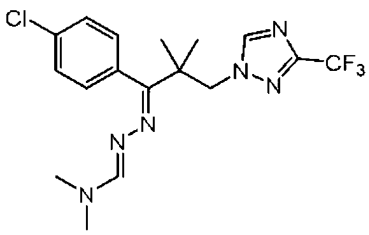

A－２９９

1-(3-(4-Chlorophenyl)-3-hydrazinylidene-2,2-dimethylpropyl)-3-(trifluoromethyl)-1H-1,2,4-triazole (B-26) (245 mg) was dissolved in toluene (5 mL), N,N-dimethylformamide dimethyl acetal (237 μL) was added, and the reaction solution was heated under reflux for 3.5 hours. The organic solvent was distilled off under reduced pressure, and the residue was purified by silica gel column chromatography to obtain 134 mg of N'-(1-(4-chlorophenyl)-2,2-dimethyl-3-(3-(trifluoromethyl)-1H-1,2,4-triazol-1-yl)propylidene)-N,N-dimethylformohydrazonamide as compound A-299 (oily substance).

Example 6

Synthesis of methyl N-(1-phenyl-2-methyl-3-(3-(trifluoromethyl)-1H-1,2,4-triazol-1-yl)propylidene)formohydrazonate (Compound No.: A-524) represented by the following formula

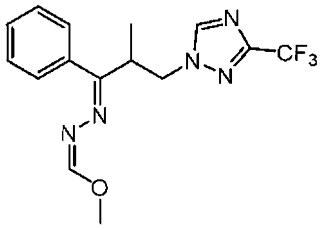

A－524

1-(3-Phenyl-3-hydrazinylidene-2-methylpropyl)-3-(trifluoromethyl)-1H-1,2,4-triazole (B-1) (526 mg) was dissolved in toluene (5 mL), trimethyl orthoformate (386 μL) was added, and the reaction solution was heated under reflux for 3 hours. The organic solvent was distilled off under reduced pressure, and the residue was purified by silica gel column chromatography to obtain 290 mg of methyl N-(1-phenyl-2-methyl-3-(3-(trifluoromethyl)-1H-1,2,4-triazol-1-yl)propylidene)formohydrazonate as compound A-524 (oily substance).

Including the compounds produced in the same manner as in the above Examples, the $^1$H-NMR data for the synthesized compounds are shown in Tables 6 to 9 below. In Tables 6 to 9 below, the compound numbers are those described in Tables 1 to 5, respectively.

Table 6

| Compound No. | 1H-NMR [$CDCl_3$/TMS, 400 MHz] |
|---|---|
| A-1 | 1.16 (3H, d), 3.37-3.48 (1H, m), 4.29 (1H, dd), 4.71 (1H, dd), 7.07-7.12 (2H, m), 7.47-7.54 (3H, m), 8.18 (1H,s), 8.37 (1H,d), 8.69 (1H, d) |
| A-5 | 1.13 (3H, d), 2.86 (6H, br), 3.43-3.51 (1H, m), 4.29 (1H, dd), 4.78 (1H, dd), 7.25-7.35 (5H, m), 7.94 (1H, s), 8.19 (1H, s) |
| A-17 | 1.13 (3H, d), 3.36-3.46 (1H, m), 4.23 (1H, dd), 4.58 (1H, dd), 6.50 (1H, d), 7.08-7.13 (2H, m), 7.16-7.21 (2H, m), 8.29 (1H, d), 8.18 (1H, s) |
| A-21 | 1.13 (3H, d), 2.88 (6H, br), 3.41-3.50 (1H, m), 4.28 (1H, dd), 4.77 (1H, dd), 6.98-7.04 (2H, m), 7.33-7.39 (2H, m), 7.94 (1H, s), 8.18 (1H, s) |
| A-22 | 1.13 (3H, d), 3.36-3.46 (1H, m), 4.23 (1H, dd), 4.58 (1H, dd), 6.50 (1H, d), 7.07-7.13 (2H, m), 7.16-7.22 (2H, m), 7.45 (1H, d), 8.29 (1H, d), 8.68 (1H, d) |
| A-26 | 1.09 (3H, d), 2.87 (6H, br), 3.38-3.48 (2H, m), 4.24 (1H, dd), 4.67 (1H, dd), 6.43 (1H, d), 6.96-7.03 (2H, m), 7.31-7.37 (2H, m), 7.45 (1H, d), 7.96 (1H, d) |
| A-27 | 1.15 (3H, d), 3.35-4.45 (1H, m), 4.29 (1H, dd), 4.70 (1H, dd), 7.08 (2H, dt), 7.50 (2H, dt), 8.18 (1H, s), 8.33 (1H, d), 8.67 (1H, d) |
| A-28 | 1.17 (2.25H, d), 1.24 (0.75H, d), 2.63 (2.25H, s), 2.87 (0.75H, s), 3.39 (1H, m), 4.30 (0.75H, dd), 4.37 (0.25H, dd), 4.70 (0.75H, dd), 4.81 (0.25H, dd), 7.09 (1H, dt), 7.35 (0.25H, dt), 7.40 (0.75H, dt), 7.65 (0.25H, s), 8.15 (0.75H, s), 8.33 (0.25H, s), 8.85 (0.75H, s) |
| A-33 | 1.12 (3H, d), 2.87 (6H, br), 3.39-3.48 (1H, m), 4.28 (1H, dd), 4.77 (1H, dd), 7.30 (4H, s), 7.94 (1H, s), 8.18 (1H, s) |
| A-42 | 1.13 (2.7H, d), 1.15 (0.3H, d), 3.36-3.48 (1H, m), 4.22 (0.9H, dd), 4.30 (0.1H, dd), 4.58 (0.9H, dd), 4.74 (0.1H, dd), 6.45 (0.1H, d), 6.50 (0.9H, d), 7.05 (2H, dt), 7.43-7.50 (3H, m), 8.27 (1H, d), 8.68 (1H, d) |
| A-46 | 1.08 (3H, d), 2.87 (6H, br), 3.36-3.46 (1H, m), 4.24 (1H, dd), 4.66 (1H, dd), 6.43 (1H, d), 7.28 (4H, s), 7.44 (1H, d), 7.95 (1H, s) |
| A-47 | 1.15(3H, d), 3.34-3.44(1H, m), 4.29(0.8H, dd), 4.35(0.2H, dd), 4.69 (0.8H, dd), 4.90(0.2H, dd), 6.95-7.03(1H, m), 7.44-7.49(0.4H, m), 7 .64-7.69(1.6H, m), 8.04(0.2H, s), 8.18(0.8H, s), 8.31(0.8H, d), 8.5 8(0. 2H, s), 8.65-8.71(1H, m) |
| A-51 | 1,12(3H, d), 2.87(6H, br), 3.39-3.48(1H, m), 4.28(1H, dd), 4.77(1H, dd), 7.23(2H, d), 7.46(2H, d) . 7.93(1H, s), 8.18(1H, s) |
| A-52 | 1.13(2.7H, d), 1.15(0.3H, d), 3.36-3.45(1H, m), 4.23(0.9H, dd), 4.2 9(0.1H, dd), 4.57(0.9H, dd), 4.74(0.1H, dd), 6.45(0.1H, d), 6.49(0. 9H,d), 6.98(2H,dt), 7.44(1H,d), 7.61-7.66(2H, m), 8.01(0.1H, s), 8.14(0. 1H, br), 8.26(0.9H,brd), 8.68(0. 9H, d) |

(continued)

| Compound No. | 1H-NMR [$CDCl_3$/TMS, 400 MHz] |
|---|---|
| A-56 | 1.08(3H, d), 2.87(6H, br), 3.36-3.46(1H, m), 4.24(1H, dd), 4.66(1H, dd), 6.43(1H, d), 7.19-7.24(2H, m), 7,42-7.46(3H, m), 7.95(1H,s) |
| A-71 | 1.14(3H, d), 2.40(3H,s), 3.35-3.45(1H, m), 4.28 (1H, dd), 4.70(1H, dd), 6.99(2H, d), 7.30(2H, d), 8.18(1H,s), 8.43(1H,d), 8.68(1H, d) |
| A-75 | 1.18 (3H,d), 3.40-3.46(1H,m), 4.32(1H, dd), 4.71(1H,dd), 7.30(2H , d), 7.82(2H,d), 8.21(1H, s). 8. 50 (1H, d), 8.64 (1H, d). |
| A-83 | 1.13-1.16 (3H, m), 3.40(1H, q), 3.85 (3H, s), 4.27-4.4.36(1H, m), 4.69 (0. 8H, dd), 4.90(0. 2H, dd), 7.02 (4H, m), 8.03 (0.2H, s), 8.17 (0.8H, s), 8.38 ( 0.2H, d) , 8.44 (0.8H, d), 8.57 (0. 2H, d), 8.69 (0.8H, d) |
| A-87 | 1.17(3H,d), 3.39-3. 44(1H, m), 4.30(1H,dd), 4.70(1H,dd), 7.19(2H, d), 7.37(2H, d), 8.18(1H,s), 8.31(1H,d), 8. 68(1H, d). |
| A-91 | 1.16(3H, d), 3.36-3.46(1H, m), 4.30(1H,dd), 4.70(1H, dd), 6.84-6.9 3(2H, m), 7.19(1H,ddt), 7.47-7.54(1H, m), 8.18(1H,s), 8.36(1H, d), 8. 68(1H, s) |
| A-95 | 1.13(3H,d), 2.88(6H,br), 3.40-3.50(1H,m), 4.28(1H,dd), 4.77(1H, dd), 6.92(1H, ddt), 7.06(1H,dt), 7.16(1H,ddd), 7.27-7.33(1H, m), 7.94(1H, s), 8.18(1H, s) |
| A-121 | 1.31 (3H, d), 4.06-4.18(1H, m), 4.26-4.32(1H, m), 4.72-4.79(1H, m), 7.14(0.3H,dd), 7.25(0.3H,t), 7.30(0.7H, dt), 7.47(0.7H, dt), 7.52 -7.62(1H, m), 7.69(0.7H,d), 7.80(0.3H,dt), 8.20(0.7H,s), 8.24(0. 3H, s) |
| A-125 | 1.13(3H,d), 2.88(6H, brs), 3.41-3.50(1H,m), 4.29(1H,dd), 4.77(1H , dd), 6.98(1H, ddt), 7.06(1H, dt), 7.17(1H,ddd), 7.26-7.33(1H,m), 7.94(1H,s), 8.18(1H, s) |
| A-131 | 1.12(3H,d), 3.38(1H, m), 4.37 (1H, dd), 4.70 (1H, dd), 6.99 (1H, d), 7.00(1 H, s), 7.45-7.47(2H,m),8.17(1H, s), 8.30(1H, d), 8.67 (1H, d) |
| A-132 | 1.17(3H,d),2.64(3H,s),3.42(1H,m),4.31 (1H,dd),4.68(1H,dd), 7.00(1 H,d), 7.02(1H,s), 7.33-7.41 (2H, m), 8.14(1H, s), 8.56(1H, s) |
| A-133 | 1.08(3H, t), 1.25(3H, d), 3.36 (1H, m), 3.53(1H, m), 4.34 (1H, dd), 4.79(1H ,dd), 7.04(1H,d), 7.06(1H, d) 7.17-7.39 (2H, m), 7.66 (1H, s), 8.27 (1H, s) |
| A-134 | 1.22 (3H, d), 2.09(1H, s), 3.54 (1H, m), 3.65 (1H, d), 4.30(1H, dd), 4.54(1H ,d), 4.80(1H, dd), 7.08(1H, d), 7.22(1H, s), 7.35-7.40 (2H, m), 8.18(1H, s ).8.30(1H, s) |
| A-135 | 1.12(3H, d), 2.88(6H, br), 3.44 (1H, m), 4.26 (1H, dd), 4.77 (1H, dd), 7.16( 1H, d), 7.25 (1H, m), 7.40(1H, s) , 7.93 (1H, s), 8.18(1H, s) |
| A-136 | 1.13(1H, d), 3.40(1H, m), 4.22 (1H, dd), 4.58 (1H, dd), 6.50(1H, d), 6.99 (1 H, m), 7.09(1H, s), 7.42-7.7.45(3H, m), 8.28(1H, d), 8.69 (1H, d) |
| A-137 | 1.15(3H, d), 2.63(3H, s), 3.46 (1H, m), 4.26 (1H, dd), 4.58 (1H, dd), 6.49(1 H, s), 7.00(1H,d), 7.11 (1H, s), 7.30-7.36 (2H, m), 7.43 (1H, s), 8.52(1H, s ) |
| A-138 | 0.83(3H,t), 1.20 (3H, d), 3.33(1H, m), 3.35-3.64 (2H, m), 4.28 (1H, m), 4.6 2(1H, dd), 6.50 (1H, d), 7.05(1H, m), 7.17 (1H, d), 7.27-7.35 (2H, m), 7.67( 1H, s), 8.08(1H, s) |
| A-139 | 1.18(3H, d), 2.12(1H, s), 3.56 (1H, m), 3.80(1H, d), 4.26(1H, dd), 4.47(1H ,d), 4.65(1H, dd),6.48(1H, d), 7.12(1H, d), 7.22(1H, s), 7.33-7.46(2H, m ), 7.50(1H, s),8.15(1H, s) |
| A-140 | 1.10(3H, d), 2, 90(6H, br) , 3.43(1H, m), 4. 25 (1H, dd), 4. 68 (1H, dd), 6.43( 1H, d), 7.15(1H, m), 7. 23-7. 26 (2H, m), 7. 44 (1H, dd), 7. 95 (1H, s) |
| A-141 | 1.15(3H, d), 3. 39(1H, m), 4. 30(1H, dd), 4. 70 (1H, dd), 7.04(1H, d), 7.26(1 H,d), 7.28(1H, dd) , 7.39(1H, d), 8.17 (1H, s), 8. 32 (1H, d), 8. 68 (1H, s) |
| A-142 | 1.17(3H, d), 2. 64 (3H. s), 3.45(1H, m), 4. 32 (1H, dd), 4. 68 (1H, dd), 7.05 (1 H, d), 7. 27-7. 31 (2H, m), 7.55(1H, d), 8. 15(1H, s), 8.57 (1H, s) |
| A-143 | 1.05(3H, t), 1. 19 (3H, d), 3. 05(1H, m), 3. 37(1H, m), 3.52(1H, m), 4. 33 (1H, dd), 4. 78(1H, dd), 7.09(1H, d), 7. 10-7. 34 (2H, m), 7.33(1H, dd), 7.48(1H, s), 8.17(1H, s), 8. 27(1H, s) |

(continued)

| Compound No. | 1H-NMR [$CDCl_3$/TMS, 400 MHz] |
|---|---|
| A-144 | 1.23(3H, d), 2.08(1H, s), 3.53(1H, m), 3. 63(1H, d), 4.49(1H, dd), 4.77(1H , d), 4.79(1H. dd), 7.13(1H, d), 7.27(1H, m), 7.31(1H, s), 7.37(1H, d), 8.1 7(1H, s). 8.30(1H, s) |
| A-145 | 0.81(3H, d), 2.88(6H, br), 3.43(1H, m), 4.26 (1H, dd), 4.75(1H, dd), 7.19-7.23(2H, m), 7.39(1H, m), 7.42(1H, s), 7.92(1H, s), 7.18 (1H, s) |
| A-146 | 1.13 (3H, d), 3.41 (1H, m), 4.25(1H, dd), 4.60(1H, dd), 6.50(1H, d), 7.02(1 H,d), 7.25(1H, d), 7.36(1H, dd), 7.44(1H, s), 7.58(1H, d), 8.28(1H, d), 8. 68(1H, d) |
| A-147 | 1.15(3H, d), 2.63(3H, s), 3.45(1H, m), 4.24(1H, dd), 4.58(1H, dd), 6.49(1 H, d), 7.04(1H, d), 7.23-7.27(2H, m), 7.43(1H, s), 7.52(1H, d), 8.52(1H, s ) |
| A-148 | 0.82(3H, t), 1.07 (3H. d), 3.33 (1H, m), 3.50-3.70(2H, m), 4.26 (1H, m), 4.7 3(1H, dd), 6. 49(1H, d), 7.10(1H, m), 7.20-7. 32 (2H, m), 7.42-7.50(2H, m), 8.09(1H, s) |
| A-149 | 1.18(3H, d), 2.12(1H, s), 3.55(1H, m), 3.86(1H, d), 4.26(1H, m), 4.41(1H, d), 4. 65 (1H, m), 6.48 (1H, d), 7.15 (1H, d), 7.24(1H, d), 7.38(1H, s), 7.46-7.54(2H, m), 8.15(1H, s) |
| A-150 | 1.10(3H, d), 2. 88 (6H, br), 3. 40(1H, m), 4. 23(1H, dd), 4. 65(1H, dd), 6. 43( 1H, d), 7.18-7.19 (2H, m), 7. 37(1H, m), 7. 44(1H, d), 7. 55(1H, s), 7. 95(1H, s) |
| A-151 | 1.18(3H,d), 3.41(1H,m), 4.33(1H,dd), 4.72(1Hdd), 6.83(1H, dd), 7.25-7.34(2H, m), 8.19(1H, s), 8.31(1H, d), 8.70(1H, d) |
| A-161 | 1.17(3H, d), 3. 33-3. 44(1H, m), 4. 31 (1H, dd), 4. 71 (1H, dd), 6.95-7.12 (3H ,m), 8.17(1H, s), 8.19(1Hd), 8.71(1H, d) |
| A-171 | 1.18(3H, d), 3. 39(1H, m), 4.32(1H, dd), 4. 70(1H, dd), 6.83(1H, d), 7.18-7 .22(2H, m), 8.18(1H, s), 8.25(1H, d), 8.70(1H, d) |
| A-176 | 1.15(3H,d), 3. 40(1H, m), 4.25(1H, dd), 4. 59(1H, dd), 6. 49(1H, d), 6.79(1 H, br), 7.15-7.18(2H, m), 7.45(1H, s), 8.22(1H, d),8.70(1H, d) |
| A-171 | 1.17(3H,d), 2. 74(3H, s), 3. 42(1H, m), 4. 25(1H, dd) , 4.59(1H, dd), 6.50(1 H, d), 6.83(1H, br), 7.07(2H, m), 7.43(1H, s), 8.49(1H, s) |
| A-178 | 0.91 (3H, t), 1.19(3H, d), 3.32(1H, m), 3. 48(2H, m), 4.29 (1H, dd), 4. 61 (1H ,dd) , 6.49(1H, d), 6.85(1H, br), 6.99(1H, m), 7.07(1H, m), 7.44(1H, s),8. 06(1H, s) |
| A-179 | 1.19(3H, d), 2.09(1H, s), 3.52(1H, m), 4. 11 (1H, m), 4. 11 (1H, d), 4. 31 (1H, dd), 4.37(1H, d), 4.65(1H, dd), 6.48(1H, d), 6. 89(1H, br), 7.05 (2H, m), 7. 46(1H, s), 8. 23(1H, s) |
| A-181 | 1.16(3H, d), 3.35-3.44 (1H, m), 4.29(1H, dd), 4.68(1H, dd), 6.90(1H, dt), 7.02(1H,dt),7.33(1H,q),8.18(1H,s), 8.41(1H, d), 8.65(1H, d) |
| A-182 | 1.18 (3H, d), 2.25 (3H. s),3.37-3.46 (1H, m), 4.30(1H, dd), 4.69 (1H, dd), 6 .88-6.94(1H, m), 7.04(1H, ddd) , 7.23(1H, q), 8.16 (1H, s), 8.54(1H, s) |
| A-184 | 1.23(1.8H, d), 1.26(1.2H, d), 2.08(0.6H, t), 2.39(0.4H, t), 3.50-3.62(1 H, m), 3.68(0.6H, dd), 4.21-4. 40(1.8H, m), 4.56(0.6H, dd), 4.74-4.85(1H ,m). 6.95-7.04(1H, m), 7.09-7.27(2H, m), 7.66(0.6H, s),8.19(0.6H, s), 8.27(0.6H, s) , 8.30(0.4H, s) |
| A-185 | 1.19(1.5H, d) , 1.24(1.5H, d) , 3.45-3.57(1.5H, m) , 3.85-3.97(1H, m) , 4.2 4-4. 37 (1. 5H, m), 4.67-4.83(1.5H, m) , 5.00(0.5H, d) , 5.12-5.34(1.5H, m) , 5.53-5.63(0.5H, m) , 6.87-6.96(1H, m), 7.03-7.07(1H, m) , 7.15(0.5H,q) , 7.23(0.5H, q) , 7.73(0.5H, s) , 8.15(0.5H, s) , 8.27(0.5H, s), 8.39(0. 5H, s) |
| A-186 | 1.09(1.3H, d) , 1.13(1.7H, d) , 3.33-3.43 (1Hm), 4.25(1H, dt), 4.37-4.45( 1H, dd), 4.53(0.6H, d), 4.65 (0.45H, dd), 4.72 (0.57H, dd), 4.90 (0.45H, d) |
| A-187 | 1.10(1.43H,d) , 1.15(1.57H,d), 3.35-3.47(1H, m), 4.22-4.30(1H, m), 4.3 7(1H, t), 4.48(0.57H, d),4.65(0. 5H, dd), 4.73(0.5H, dd) , 4.80(0.48H, d) , 6.50-6.64(2H, m), 6.82 (0. 97H, d) , 6.88(1.04H, d), 7.01 (0.53H, q), 7.11 (0.47H, q), 7.25-7.28 (2H, m), 7.90(0.52H, s), 8.07(0.48H, s), 8.16 (0. 52 H, s), 8.24(0.48H, s) |
| A-194 | 1.17(3H, d), 3.38-3.43 (1H, m) , 4.30(1H, dd), 4.68 (1H, dd), 6.69-6.72(2H ,m) , 6.96(1H, tt), 8.18(1H,s), 8.32(1H, d), 8.67(1H, d) |

(continued)

| Compound No. | 1H-NMR [$CDCl_3$/TMS, 400 MHz] |
|---|---|
| A-195 | 1.19 (2.3H, d), 1. 25 (0.7H, d), 2. 69 (2.3H, s), 2.96(0.7H, s), 3.393.45(0. 7H, m), 3.49-3. 51 (0.3H, m), 4. 30(0.7H, dd), 4.37 (0.3H, dd), 4.69 (0.7H, d d), 4.79 (0.3H, dd), 6.70-6.73 (3H, m), 6.86-6.91 (1H, m), 7.65 (0. 3H, s), 8 .15(0.7H, s), 8.29(0.3H, s) , 8.56(0.7H, s). |
| A-196 | 0. 84 (1.2H, t), 1.09(1.8H, t), 1.22 (1. 2H, d), 1.25 (1. 8H, d), 3.09-3.16 (0 .4H,m), 3.42 (1.2H, q), 3.48-3.58 (1H, m), 3.65-3.74(0.4H, m) , 4.29-4.38 (1H, m) , 4.73 (1H, dd), 4.79(1H, dd), 6.72-6. 77(2H, m), 6.79-6.91 (1H, m), 7.67(0.6H, s), 8.16 (0.4H, s), 8.16 (0.4H, s), 8.26 (0.6H, s) . |
| A-197 | 1.25(3H,d), 2.10(0.6H, t)2.39(0.4H, t), 3.53-3.58(1H, m), 3.73(0.6H, d d), 4.23(0.8H, dd), 4.24-4.35(1H, m) , 4.55 (1H, dd), 4.74-4.80(1H, m) , 6. 81-6.84(2.4H, m) , 6.90(0.6H, tt) , 7.68(0. 4H, s) , 8.18(0. 6H, s), 8.27(0. 6H, s) , 8.29(0.4H, s). |
| A-234 | 1.25(3H, d), 3.38(1H, m) , 4.28 (1H, dd), 4.67 (1H, dd), 6.99(1H, d) , 7.25(1 H, s), 7.60 (1H, d) , 8.18(1H, s) , 8.66 (1H, d) |
| A-237 | 1.23(3H, d), 2.08(1H, s) , 3.53(1H, m), 3.67(1H, d), 4.32(1H, dd), 4.57(1H , d) , 4.77(1H,dd), 7.09(H,d), 7.35(1H, s) , 7.51(1H, d), 8.17(1H, s), 8.29 (1H, s) |
| A-238 | 1.18(3H, d), 2. 88(6H, br) , 3.42 (1H, m), 4.29 (1H, dd), 4.74(1H, dd), 7.17( 1H, d) , 7.39(1H, d), 7.55(1H, s), 7.93(1H, s), 8.17(1H,s) |
| A-244 | 1.17(3H, d), 3.37-3.42(1H, m), 4.30 (1H, dd), 4. 69 (1H, dd), 7.03(2H, d), 7 .50(1H,t), 8.18 (1H, s), 8.28 (1H, d), 8.67(1H,d). |
| A-254 | 1.19(3H, d) , 3.34-3.44(1H, m), 4.32(1H, dd) , 4.70(1H, dd) , 7.05-7.14(2H , m) , 8.18(1H,s),8.54-8.60(1H,m),8.66(1H,d). |
| A-264 | 0. 97 (3H, t), 1. 49 (1H, m), 1. 63 (1H, m), 3. 35 (1H, m), 4. 31 (1H, dd), 4.57(1H , dd), 6. 48 (1H, d), 7.02(1H, dd) , 7. 44-7. 46 (2H, m) , 8.38(1H, d), 8. 71 (1H, d) |
| A-268 | 0.90(3H, t) , 1.48(1H,m), 1.62(1H, m) , 2. 87 (6H, br) , 3. 34 (1H, m), 4. 33 (1H , dd) , 4. 66 (1H, dd), 6. 41 (1H, d), 7. 23-7. 28 (5H, m), 7. 43 (1H, d), 7.96(1H, s) |
| A-269 | 1.18 (6H, s), 4.52(2H, s) , 7.07 (2H, dd), 7. 46-7.54(3H, m) , 7.99(1H, d), 8. 21(1H, s) , 8.67(1H, d) |
| A-270 | 1.19(6H, s), 2.60(3H, s) , 4. 53 (2H, s), 7. 08-7. 14(2H, m), 7.32-7.42(3H, m ), 8.19(1H, s) , 8.53(1H, s) |
| A-271 | 1.16-1.20(9H, m) , 3.35(2H, q) , 4.66(2H, s), 7. 09-7. 12(2H, m), 7. 32-7. 41 (3H, m), 7. 63 (1H, s), 8. 65 (1H, s) |
| A-272 | 1.23(6H, s) , 2.17(1H, t) , 3.99(2H, d) , 4.62(2H,s) , 7.09-7.16(2H, m) , 7.3 0-7.42(3H, m), 8. 30(1H, s), 8. 32(1H, s) |
| A-273 | 1.14 (6H, s) , 2.76(6H, br), 4.63(2H, s), 6.92-6. 94(2H, m), 7.24-7.34(3H, m), 7.92 (1H, s), 8.38(1H, s) |
| A-274 | 1.15(6H, s), 4.43(2H, s), 6.53(1H, d), 7.00-7.09(2H, m), 7.44-7.52(4H, m ) , 7.99(1H, d) , 8.67(1H, d) |
| A-275 | 1.15(6H, s) , 2.59(3H, s) , 4.44 (2H, s), 6.53(1H, d), 7.13-7. 16(2H,m), 7. 3 2-7. 40 (3H, m), 7.46(1H, d), 8.49(1H, s) |
| A-276 | 0. 96 (1.5H, t), 1. 14-1. 22(7.5H, m), 3. 34 (2H, q), 4. 46 (1H, s) , 4. 58 (1H, s) , 6.53(0.5H, d), 6. 54(0. 5H, d), 7. 12-7. 17(2H, m), 7. 28-7. 40(3H,m), 7. 47 (0.5H,d), 7.63(0.5H,s) , 7.90(0. 5H, d) , 8. 15 (0. 5H, s) |
| A-271 | 1.19(6H, s) , 2.18(1H, t) , 4.04(2H,d) , 4.51(2H, s), 6.52(1H, d), 7. 14-7. 2 0(2H, m), 7.31-7.40(3H, m), 7. 54(1H, d), 8. 27(1H, s) |
| A-278 | 1.10(6H, s) , 2.76 (6H, br) , 4.59(2H, s) , 6.49 (1H, d) , 6.93-6. 96 (2H, m) , 7. 23-7.34(3H, m) , 7.61(1H,d), 7. 93 (1H, s) |
| A-285 | 1.17(6H,s), 4.48(2H, s), 7.12 (2H. dd), 7.23 (2H, t), 7.98(1H, d), 8.21(1H ,s), 8.66(1H, d) |

(continued)

| Compound No. | 1H-NMR [$CDCl_3$/TMS, 400 MHz] |
|---|---|
| A-286 | 1.18(6H, s) , 2.61(3H, s), 4.50(2H, s) , 7.02-7.18(4H, m) , 8.18(1H, s),8.5 1(1H, s) |
| A-287 | 1.12-1.26(9H, m) , 3.39(2H, q) , 4.63(2H, s), 7.02(2H, t), 7.13 (2H, dd), 7. 61(1H, s) , 8.55(1H, s) |
| A-288 | 1.23(6H,s),2.21(0.5H,t),2.43(0.5H,t),4.03(1H,d), 4.16(1H, d) , 4.59 (1H, s), 4. 68(1H, s), 7.02(0.5H, t) , 7.10-7.20(1. 5H, m), 7. 67(0. 5H, s) , 8 .27(0.5H, s) , 8.30(0.5H, s), 8.65(0. 5H, s) |
| A-289 | 1.13(6H, s) , 2.77(6H, br), 4.62(2H, s), 6.91 (2H,dd), 7.01 (2H, t), 7.91 (1 0H,s) , 8.36(1H, s) |
| A-295 | 1.17(6H, s),4.48(2H, s), 7.07(2H, dt), 7. 52 (2H, dt), 7.96(1H, d), 8.20(1 H, s), 8.66(1H, d) |
| A-296 | 1.18(6H, s), 2.63(3H, s) , 4.50(2H, s), 7.12(2H, dt) , 7.40(2H, dt), 8.18(1 H, s), 8. 51 (1H, s) |
| A-297 | 1. 12-1. 22 (9H, m), 3.38(2H, q), 4. 62 (2H, s) , 7.07(2H, dt), 7. 29 (2H, dt) , 7 .61(1H, s) , 8. 53(1H, s) |
| A-298 | 1.22(6H, s) , 2.21(0.5H, t) , 2.43(0.5H, t), 4.05(1H, d) , 4.17(1H, d) , 4.58 (1H, s), 4.67(1H, s), 7.08(0. 5H, d) , 7.14(0.5H, d), 7.30(0.5H, d), 7. 40 (0 .5H, d), 7. 66(0.5H, s), 8.27(0.5H, s) , 8.29(0.5H, s), 8.63(0.5H, s) |
| A-299 | 1.13(6H, s), 2.78(6H, br) , 4.62(2H, s) , 6.88(2H, dt), 7. 29 (2H, dt), 7. 91 ( 1H, s) , 8.36(1H, s) |
| A-300 | 1.14(6H, s), 4.37(2H, s), 6.53 (1H, d), 7.10 (2H, dt), 7.46 (1H, d), 7.49 (2H , dt) , 7.94(1H, d) , 8.64 (1H, d) |
| A-301 | 1.15(6H , s), 2.62 (3H, s), 4.38(2H, s), 6.53(1H, d), 7.17(2H, dt) , 7.38 (2H , dt) , 7.43(1H, d), 8.46(1H, s) |
| A-302 | 1.11-1.22(9H, m) , 3.38(2H, q) , 4.52(2H, s), 6.53(1H, d), 7.15 (2H, dt), 7. 29 (2H, dt), 7.60(1H, s), 7.77(1H, d) |
| A-303 | 1.18(6H, s) , 2.18(1H, t) , 4.09(2H, d), 4.46(2H, s), 6.52(1H, d) , 7.22(2H, dt), 7.38(2H, dt), 7.51 (1H, d) , 8.22(1H, s) |
| A-304 | 1.09(6H, s) , 2.78(6H, br) , 4.56(2H, s) , 6.49(1H, d) , 6.88(2H, dt) , 7.29(2 H, dt), 7.58(1H, d), 7.92(1H, s) |
| A-305 | 1.17(6H, s), 4. 48(2H,s), 7.01(2H, dt), 7.68 (2H, dt), 7.95(1H, d), 8.20(1 H, s) , 8.66(1H, d) |
| A-307 | 1.14-1.22(9H, m), 3.38(2H, q) , 4.62(2H, s), 7. 02 (2H, dt), 7.46(2H, dt) , 7 .61(1H, s), 8.52(1H, s) |
| A-308 | 1.25(6H, s), 2.21(0.5H, t), 2.43(0.5H, t), 4.05(1H, d), 4.17(1H, d), 4.58 (1H, s), 4.67(1H, s), 7.02(0.5H, d), 7.07(0.5H, d), 7.4 (0.5H, d), 7.46(0 .5H, d), 7.66(0.5H, s), 8.26(0.5H, s), 8.29(0.5H, s), 8.62(0.5H, s) |
| A-309 | 1.12(6H, s), 2.78(6H, br), 4.61(2H, s), 6.91(2H, dt), 7.46(2H, dt), 7.91( 1H, s), 8.35(1H, s) |
| A-341 | 1.18(6H, s), 4.53(2H, s), 7.09(1H, dd), 7.22(1H, dd), 7.31(1H, dd), 7.52( 1H, m), 8.00(1H, d), 8.19(1H, s), 8.69(1H, d) |
| A-342 | 1.18(6H, s),2.69(3H,s),4.55(2H,s),7.10-7.22(3H,m),7.43(1H,m),8.1 7(1H,s),8.53(1H,s) |
| A-343 | 0.97-1.01(9H,m),3.44(2H,m),4.70(2H,q),7.10-7.7.20(3H,m),7.45(1H ,m),7.64(1H,s),8.60(1H,s) |
| A-344 | 1.24(6H,s),2.22(1H,s),4.08(1H,d),4.18(1H,d),4.58(1H,d),4.68(1H, d),7.11-7.22(3H,m),7.44(1H,m),8.24(1H,s),8.32(1H,s) |
| A-345 | 0.88(3H,s),0.96(3H,s),2.76(6H,br),4.63(2H,s),6.92(1H,dd),7.00(1 H,dd),7.05(1H,dd),7.28(1H,m),8.21(1H,s),8.35(1H,s) |
| A-346 | 1.13(3H,s),1.18(3H,s),4.44(2H,s),6.53(1H,d),7.20(1H,dd),7.23(1H ,dd),7.28(1H,m),7.47(2H,-m),8.01(1H,d),8.71(1H,d) |
| A-347 | 1.12(3H,s),1.22(3H,s),2.69(3H,s),4.45(2H,s),6.52(1H,d),7.07-7.1 9(3H,m),7.39(1H,m),7.45(1H,d),8.47(1H,s) |
| A-348 | 0.99(3H,t),1.13(3H,s),1.28(3H,s),3.45(2H,m),4.48(2H,d),6.53(1H, d),7.09-7.20(3H,m),7.35(1H,m),7.65(1H,d),8.08(1H,s) |

(continued)

| Compound No. | 1H-NMR [$CDCl_3$/TMS, 400 MHz] |
|---|---|
| A-349 | 1.16(3H,s),1.21(3H,s),2.16(1H,s),4.10(1H,d),4.18(1H,d),4.51(1H, d),4.56(1H,d),6.52(1H,d),7.07-7.22(3H,m),7.41(1H,m),7.54(1H,d), 8.21(1H,s) |
| A-350 | 1.14(1H,d),2.76(6H,br),4.58(2H,d),6.48(1H,dd),6.91(1H,dd),7.02( 1H,dd),7.09(1H,dd),7.27(1-H,m),7.60(1H,d),7.97(1H,s) |
| A-351 | 1.96(3H,s),1.26(3H,s),4.50(1H,d),4.66(1H,d),7.97(1H,dd),7.41-7. 46(2H,m),7.51(1H,1H,dd),7.95(1H,d),8.21(1H,s),8.73(1H,d) |
| A-371 | 1.17(6H,s),4.49(2H,d),6.87-6.90(2H,m),7.20(1H,m),7.52(1H,m),7.9 6(1H,d),8.20(1H,s),8.67(1H,d) |
| A-372 | 1.23(6H,s),2.64(3H,s),4.50(2H,s),6.95(2H,m),7.12(1H,m),7.31(1H, m),8.17(1H,s),8.52(1H,s) |
| A-373 | 1.19(9H,m),3.37(2H,q),4.63(2H,s),6.89(1H,m),7.07(1H,m),7.28(1H, m),7.63(1H,s),8.54(1H,s) |
| A-374 | 1.24(6H,s),2.22(1H,s),4.06(2H,s),4.58(2H,s),6.95(1H,m),7.13(1H, m),7.41(1H,dd),8.68(1H,s),8.30(1H,s) |
| A-375 | 1.14(6H,s),2.77(6H,br),4.62(2H,s),6.67-6.71(2H,m),6.97(1H,m),7 .28(1H,m),7.91(1H,-s),8.36(1H,s) |
| A-376 | 1.15(6H,s),4.38(2H,s),6.53(1H,d),6.90-6.92(2H,m),7.18(1H,m),7.4 5-7.52(1H,m),7.96(1H,d),8.64(1H,d) |
| A-377 | 1.16(6H,s),2.95(3H,s),4.39(2H,s),6.54(1H,d),6.96-6.99(2H,m),7.1 0(1H,m),7.29(1H,m),7.44(1H,d),8.48(1H,s) |
| A-378 | 1.20-1.26(9H,m),3.37(2H,q),4.54(2H,s),6.54(1H,m),6.94-6.97(2H,m ),7.08(1H,m),7.30(1H,-m),7.62(1H,s),8.13(1H,s) |
| A-379 | 1.26(6H,s),2.19(1H,s),4.11(2H,s),4.47(2H,s),6.52(1H,d),6.92-7.1 2(3H,m),7.38(1H,m),7.51(1H,s),8.23(1H,s) |
| A-380 | 1.10(6H,s),2.78(6H,br),4.57(2H,s),6.49(1H,d),6.67-6.72(2H,m),6 .94(1H,m),7.26(1H,-m),7.58(1H,d),7.92(1H,s) |
| A-381 | 1.(6H,s),4.44(2H,s),6.70(1H,m),7.13(1H,d),7.47-7.48(2H,m),7.9 7(1H,d),8.20(1H,s),8.65(1H,d) |
| A-382 | 1.18(6H,s),2.64(3H,s),7.05(1H,dd),7.17(1H,s),7.35-7.40(2H,m),8. 17(1H,s),8.53(1H,s) |
| A-383 | 1.18-1.26(9H,m),3.38(2H,q),4.63(2H,s),7.02(1H,dd),7.13(1H,d),7. 26(1H,m),7.34(1H.m),7.62(1H,s),8.52(1H,s) |
| A-384 | 1.23(6H,s),2.23(1H,s),4.05(2H,s),4.67(2H,s),7.08(1H,dd),7.26(1H ,m),7.37(1H,m),8.25(1H,-s),8.30(1H,s) |
| A-385 | 1.14(6H,s),2.77(6H,br),4.62(2H,s),6.80(1H,m),6.95(1H,d),7.24-7. 26(2H,m),7.91(1H,s).8.35(1H,s) |
| A-386 | 1.14(6H,s),4.38(2H,s),6.53(1H,d),7.02(1H,m),7.13(1H,s),7.44-7.4 6(3H,m),7.95(1H,d),8.64(1H,d) |
| A-387 | 1.16(6H,s),2.63(3H,s),4.39(2H,s),6.53(1H,d),7.09(1H,dd),7.20(1H ,d),7.31-7.39(2H,m),7.45(1-H,s),8.48(1H,s) |
| A-388 | 1.18(6H,s),1.21(3H,t),3.37(2H,q),4.41(2H,s),6.52(1H,d),7.08(1H, m),7.17(1H,m),7.23(1H,m),7.30(1H,m),7.62(1H,s),8.13(1H,s) |
| A-389 | 1.18(6H,s),2.40(1H,s),4.11(1H,s),4.46(1H,s),6.53(1H,d),7.15(1H, m),7.23(1H,m),7.30-7.39(2H,m),7.50(1H,s),8.23(1H,s) |
| A-390 | 1.09(6H,s),2.78(6H,br),6.49(1H,d),6.82(1H,m),6.95(1H,s),7.23-7. 26(2H,m).7.58(1H,d),7.92(1H,s) |
| A-391 | 1.17(6H,d),4.44(2H,d),7.05(1H,d),7.28(1H,s),7.41(1H,dd),7.64(1H ,d),7.96(1H,d),8.20(1H,-s),8.66(1H,d) |

(continued)

| Compound No. | 1H-NMR [$CDCl_3$/TMS, 400 MHz] |
|---|---|
| A-392 | 1.18(6H,s),2.97(3H,s),4.62(2H,s),7.10(1H,d),7.27(1H,dd),7.31(1H ,s),7.60(1H,d),8.18(1H,-s),8.53(1H,s) |
| A-393 | 1.20-1.22(9H,m),3.36(2H,q),4.62(2H,s),7.09(1H,d),7.18(1H,dd),7. 28(1H,s),7.47(1H,d),7.62(1H,s),8.52(1H,s) |
| A-394 | 1.24(6H,s),2.45(1H,s),4.05(2H.s),4.58(2H,s),7.10(1H,d),7.28(1H, dd),7.35(1H,d),7.67(1H,s),8.26(1H,s),8.03(1H,s) |
| A-395 | 1.14(6H,s),2.78(6H,br),4.62(2H,s),6.85(1H,d),7.10(1H,s),7.18(1H ,dd),7.40(1H,d),7.91(1H,-s),8.35(1H,s) |
| A-396 | 1.15(6H,s),4.38(2H,s),6.53(1H,d),7.09(1H,d),7.28(1H,d),7.38(1H, dd),7.40(1H,s),7.60(1H,d),8.65(1H,d) |
| A-397 | 1.16(6H,s),2.97(3H,s),4.39(2H,s),6.53(1H,d),7.15(1H,d),7.26(1H, dd),7.34(1H,d),7.44(1H,s),7.52(1H,d),8.50(1H,s) |
| A-398 | 1.18-1.23(9H,m),3.39(2H,q),4.53(2H,s),6.53(1H,d),7.15-7.19(2H,m ),7.33(1H,d),7.45(1H,-d),7.61(1H,s),7.77(1H,s) |
| A-399 | 1.19(6H,s),2.22(1H,s),4.09(2H.s),4.46(1H,s),6.53(1H,d),7.20-7.2 7(2H,m),7.38(1H,s),7.50-7.53(2H,m),8.23(1H,s) |
| A-400 | 1.09(6H,s),2.78(6H,br),4.56(2H,s),6.88(1H,d),7.11(1H,d),7.19(1H ,dd),7.38(1H,d),7.57(1H,-d),7.92(1H,s) |
| A-411 | 1.18(6H,s),4.51(2H,d),6.99(1H,dt),7.06(1H,dt),7.16(1H,m),735(1H ,dt),8.04(1H,d),8.18(1H,-s),8.69(1H,d) |
| A-412 | 1.17(2H,s),1.21(3H,s),1.26(1H,s),2.71(2.1H,s),3.10(0.9H,s),4.52 (1.5H,s),4.53(0.5H,s),6.88-6.99(2H,m),7.10-7.20(1H,m),7.59(0.3H ,s),8.17(0.7H,s),8.52(0.7H,-s),8.60(0.3H,s) |
| A-414 | 1.19(1.4H,s),1.22(1.7H,s),1.25(1.7H,s),1.2H,s),2.20((0.6H,t),2 .45(0.4H,s),4.04-4.16(1H,-m),4.27-4.29(1H,m),4.54(0.6H,d),4.64-4 .78(1.4H,m),6.75(2H,m),7.15-7.23(1H,m),7.73(0.4H,-s),8.25(0.5H,s ),8.28(0.5H,s),8.63(0.4H,s) |
| A-421 | 1.20-1.20(6H,d),4.51(2H,d),7.05(1H,d),6.90-6.95(1H,m),7.19-7.24 (2H,m),8.06(1H,d),8.19(1H,s),8.68(1H,d)) |
| A-431 | 1.19(6H,s),4.45(2H,d),7.05(1H,d),6.90-6.95(1H,m),7.04-7.10(1H,m ),7.35(1H,q),8.03(1H,-d),8.20(1H,d),8.63(1H,d) |
| A-441 | 1.20(3H,s),4.45(2H,s),6.73-6.78(2H,m),6.97(1H,tt),7.99(1H,d ),8.19(1H,s),8.63(1H,d) |
| A-442 | 1.91(4.1H,s),1.24(2.1H,s),2.70(2.1H,s),3.01(1.1H,s),4.47(1. 4H,s),4.59(0.7H,s),6.68-6.95(3H,m),7.61(0.3H,s),8.17(0.7H,s ),8.50(0.7H,s),8.53(0.3H,s) |
| A-502 | 0.99(3H,t,),1.55(1H,m),1.68(1H,m),3.36(1H,m),4.35(1H,dd),4.69(1 H,dd),7.05-7.07(2H,m),7.47-7.49(3H,m),8.16(1H,s),8.42(1H,d),8.7 0(1H,d). |
| A-503 | 0.99(3H,t),1.55(1H,m),1.70(1H,m),2.59(3H,s),3.41(1H,m),4.38(1H, dd),4.69(1H,dd),7.06-7.09(2H,m),7.37-7.40(3H,m),8.13(1H,s),8.54 (1H,s) |
| A-504 | 0.79(0.5H,t),0.94-0.1.06(3H,m),1.07(1.5H,t),1.62(1H,m),1.74(1H, m),3.04(0.5H,m),3.27(1H,m),3.53(1H,m),3.65(0.5H,m),4.38(1H,m),4 .74(0.5H,dd),4.84(0.5H,-dd),7.13-7.15(2H,m),7.37(3H,m),7.70(0.5H ,s),8.12(0.5H,s),8.15(0.5H,s),8.31(0.5H,s). |
| A-505 | 0.99-1.07(3H,m),1.58(1H,m),1.75(1H,m),2.05(0.7H,d),2.33(0.3H,s) ,3.54(1H,m),3.64(0.7H,dd),4.00-4.20(0.6H,m),4.36(1H,m),4.52(0.7 H,m),4.78-4.82(1H,m),7.16-7.19(2H,m),7.34-7.41(3H,m),7.70(0.3H, s),8.17(0.7H,s),8.25(0.7H,s),8.34(0.3H,s). |
| A-506 | 0.93(3H,t),1.50(1H,m),1.67(1H,m),2.86(6H,s),3.38(1H,m),4.33(1H, dd),4.79(1H,dd),7.26-7.31(5H,m),7.94(1H,s),8.16(1H,s). |

(continued)

| Compound No. | 1H-NMR [$CDCl_3$/TMS, 400 MHz] |
|---|---|
| A-507 | 0.96(3H,d),1.09(3H,d),2.01(1H,m),3.36(1H,m),4.34(1H,dd),4.79(1H ,dd),6.99(2H,-d),7.44-7.49(3H,m),8.15(1H,s),8.44(1H,d),8.71(1H,d ). |
| A-508 | 1.19(3H,d),3.44(1H,m),4.32(1H,dd),4.72(1H,dd),7.34(1H,d),7.40(1 H,s).7.67(1H,dd),7.77(1H,d),8.20(1H,s),8.31(1H,d),8.68(1H,d) |
| A-509 | 1.20(3H,d),2.59(3H.s),3.48(1H,m),4.33(1H,dd),4.72(1H,dd),7.35(1 H,m),7.41(1H,m),7.57(1H,m),7.67(1H,d),8.17(1H,s),8.58(1H,s). |
| A-510 | 0.79(1H,t),1.08(2H,t),1.23(1H,d),1.27(2H,d),3.03(0.3H,m),3.40(1 .3H,q),3.56-3.59(1.4H,-m),4.35-4.40(1H,m),4.79-4.84(1H,m),7.39-7 .43(1.6H,m),7.49-7.61(1H,m),7.61-7.63(2H,-m),8.16(0.7H,s),8.27(0 .7H,s) |
| A-511 | 1.24-1.26(3H,m),2.07(0.6H,s),2.38(0.4H,s),3.58-3.63(1.8H,m),4.2 2(0.6H,m),4.31-4.35(1H,m),4.52(0.6H,d),4.76-4.82(1H,m),7.40-7.5 9(3.8H,m),7.70(0.6H,d),8.19(0.6H,s),8.29(1H,d) |
| A-512 | 1.15(3H,d),2.65(3H,s),3.42(1H,q),4.22(1H,dd),4.55(1H,dd),7.01(1 H,dt),7.13(1H,t),7.31-7.41(3H,m),7.68(1H,s),7.73(1H,s),8.54(1H, s). |
| A-513 | 0.84(1.5H,t),1.05(1.5H,t),1.18(1.5H,d),1.22(1.5H,d),3.06-3.17(0 .5H,mz),3.32-3.40(1H,-m),3.48-3.58(1H,m),3.60-3.70(0.5H,m),4.26( 1H,dtz),4.59(0.5H,dd),4.68(0.5H,dd),7.02-7.09(1H,m),7.15(1H,d), 7.25-7.40(2H,m),7.68(1H,s),7.73(1H,d),7.82(0.5H,s),8.15(0.5H,s) |
| A-514 | 1.20(2.1H,d),1.22(0.9H,d),2.14(0.7H,t),2.39(0.3H,t),3.46-3.58(1 H,m),3.85(0.7H,dd),4.17-4.34(1.7H,m),4.45(0.7H,dd),4.58-4.72(1H m),7.08-7.14(1H,m),7.20-7.24(1H,m),7.24-7.41(2H,m),7.68(0.3H,s ),7.70-7.76(1.7H,-m),7.85(0.3H,s),8.20(0.7H,s) |
| A-515 | 1.19(3H,d),2.69(3H,s),3.08(1H,q),4.07(1H,dd),4.45(1H,dd),6.89(1 H,dt),7.04-7.08(1H,m),7.27(1H,s),7.30-7.43(3H,m),7.53(1H,s),8.6 3(1H,s). |
| A-516 | 0.91(1.2H,t),1.16-1.24(4.8,m),3.12-3.29(1.4H,m),3.48(1H,q),3.57 -3.68(0.4H,m),4.06-4.18(1.8H,m),4.44-4.57(1H,m),6.92(0.4H,dt),7 .00(0.60H,dt),7.07(0.4H,-t),7.11(0.6H,t),7.26-7.44(3H,m),7.54(0. 4H,s),7.59(0.60H,s),7.64(0.6H,s),8.21(0.4H,s) |
| A-517 | 1.23(3H,d),2.18(0.60H,t),2.46(0.35H,t,J=2.18Hz),3.12-3.27(1H,m) ,3.85(0.65H,dd),4.06-4.20(1H,m),4.29(0.65H,t),4.43(0.65H,dd),4. 47-4.56(1.0H,m),7.01(0.65H,d),7.10(0.35H),7.16(0.60H,s),7.24(0. 35H,s),7.28-7.44(3H,m),7.56(0.65H,s),7.60(0.35H,s),7.65(0.35H,s ),8.33(0.60H,s). |
| A-518 | 1.21(3H,d),2.34(1H,s),2.37(3H,s),3.551-3.61(1H,m),3.65(1H,dd),4 .30(1H,dd),4.52(1H,dd),4.79(1H,dd),7.09(2H,d),7.20(2H,d),8.19(1 H,s),8.28(1H,s). |
| A-519 | 1.15(3H,d),2.38(3H,s),3.41(1H,m),4.29(1H,dd),4.70(1H,dd),6.82-6 .89(2H,m),7.28(1H,-d),7.38(1H,dd),8.17(1H,s),8.39(1H,d),8.68(1H, d) |
| A-520 | 1.17(2.4H,t),1.24(0.6H,t),2.34(0.6H,s),2.35(2.4H,s),2.62(2.4H,s ),2.78(0.6H,s),3.42-3.48(1H,-m),4.29(0.8H,dd),4.37(0.2H,dd),4.70 (0.8H,dd),4.84(0.2H,dd),6.88-6.91(2H,m),7.21-7.30(2H,m),7.71(0. 2H,s),8.14(0.8H,s),8.38(0.2H,s),8.55(0.8H,s) |
| A-521 | 0.81(1.8H,t),0.99(1.2H,t),1.19(1.8H,d),1.24(1.2H,d),2.33(1.2H,s ),2.34(1.8H,s),3.07(0.6H,-m),3.27(0.8H,m),3.55(1H,m),3.65(0.6H,m ),4.28-4.32(1H,m),4.74(0.6H,dd),4.84(0.4H,dd),6.92-6.96(2H,m),7 .18-7.28(2H,m),7.72(0.4H,s),8.14(0.6H,s),8.17(0.6H,s),8.32(0.4H ,s) |
| A-522 | 1.22(3H,d),2.06(1H,t),3.57(1H,m),3.64(1H,d),4.30(1H,dd),4.49(1H ,d),4.78(1H,dd),6.96-6.99(2H,m),7.20-7.30(2H,m),8.19(1H,s),8.29 (1H,s). |
| A-523 | 1.20(3H,d),3.40-3.51(1H,m),4.33(1H,dd),4.72(1H,dd),7.48(1H,dt), 7.75(1H,t),8.05(1H,t),8.21(1H,s),8.35(1H,ddd),8.54(1H,d) |

(continued)

| Compound No. | 1H-NMR [$CDCl_3$/TMS, 400 MHz] |
|---|---|
| A-524 | 1.15(1.8H,d),1.44(1.2H,d),3.45-3.57(0.6H,m),3.63(1.8H,s),3.95(1 .2H,s),4.04-4.10(0.4H,-m),4.31(0.6H,dd),4.53(0.4H,dd),4.80(0.6H, dd),4.98(0.4H,dd),7.21-7.23(1H,m),7.33-7.38(3H,m),7.46-7.48(1H, m),7.96(0.4H,s),8.12(0.6H,s),8.17(0.4H,s),8.26(0.6H,s). |
| A-525 | 1.15(1.2H, d) , 1.21(1.8H, t) , 1.42-1.46(3H, m) , 3. 38-3. 47(0.4H, m) , 3.4 8-3.54(0.4H, m) , 3. 55-3. 64(0.4H, m) , 3. 98-4. 06(1.2H, m) , 4.29-4.34(1H , m) , 4. 51 (0.6H, dd) , 4.80(0.4H, dd) , 4. 97 (0. 6H, dd) , 7.19-7.21 (1H, m) , 7 .33-7. 37(3H, m) , 7.45-7.47 (1H, m) , 7.96(0.6H, s) , 8. 11 (0.4H, s), 8.18(0 .4H, s) , 8. 25 (0.6H, s). |
| A-526 | 1.13(3H, d) , 3.38(1H, d) , 4. 20(1 H, q) , 4. 54(1H, q), 6.99-7.03(1H, m) , 7.1 1-7.14(1H, m), 7.43-7.47 (2H, m), 7.71 (2H, d), 8.32(1H, d), 8.68(1H, d). |
| A-527 | 1.16(3H, d) , 3.02-3.12(1H, m), 4.07(1H, dd) , 4. 45(1H, dd) , 6.98-6.93(1H , m), 7.04-7.07(1H, m) , 7.27-7.29(1H, m) , 7.42-7.50(2H, m), 7.54(1H, s), 8.40(1H, d) , 8.71(1H, d). |
| A-528 | 1.14(3H, d) , 3.59(1H, q) , 4.27(1H, dd), 4.72(1H, dd), 6.88(1H, s), 7.19(2 H, d) , 7.50(2H, d) , 8.27(1H, d) , 8.60(1H, d) . |
| A-529 | 1.14(3H, d) , 2.35(3H, s), 3. 45-3. 56(1H, m), 4. 06(1H, dd) , 4. 46(1H, dd) , 6 .25(1H, s), 7.09(2H,d), 7.26 (1H, s), 7. 47 (2H, d), 8. 26 (1H, d), 8.64 (1H, d ) . |
| A-530 | 1.20(3H, d) , 2. 05(1H, t) , 2. 33(3H, s), 3. 66-3. 77(2H, m), 4. 07(2H, dd), 4. 48-4. 59(2H, m), 6. 23(1H, s) , 7. 22-7. 27(3H, d), 7. 37 (2H, d). 8.05(1H, s). |
| A-531 | 1.17(3H, d) , 2.32(3H, s) , 2. 58 (3H, s) , 3. 53-3. 63 (1H, m) , 4.08 (1H, dd) , 4. 46 (1H, dd) , 6. 25(1H, s) , 7.15(2H, d) , 7. 37 (2H, d) , 8. 43 (1H, s). |
| A-532 | 1.10(3H, d) , 3. 26-3.37(1H, m) , 4. 13(1H, dd) , 4. 48 (1H, dd) , 7. 07(2H, d) , 7 . 41 (1H, s) , 7. 44-7. 50(3H, m) , 8.32(1H, d) , 8. 68 (1H, d). |
| A-533 | 1.10(3H, d) , 3. 27-3. 37(1H, m) , 4. 10(1H, dd), 4. 45(1H, dd), 7. 07(2H,d) , 7 . 39 (1H, s), 7. 43 (1H, s) , 7. 47 (2H, d), 8. 34(1H. d), 8. 67(1H, d) |
| A-534 | 1.13(3H, d) , 2. 65(3H, s), 3. 33-3. 43(1H, m), 4. 16(1H, dd), 4.49(1H, dd), 7 .08(2H, d) , 7.37(2H, d) , 7.40(1H, s) , 7.47(1H, s) , 8.51(1H,s). |
| A-535 | 1.17 (3H, d) , 2.17(1H, t) , 3. 43-3. 53(2H, m) , 3. 84(1H, dd), 4. 20(1H, dd) , 4 .48(1H, dd), 4.57(1H, dd), 7.17(2H,d) , 7.37(2H, d), 7.45(1H, s) , 7.47(1H , s) , 8.16(1H, s) |
| A-536 | 1.13(3H, d) , 2.65(3H, s), 3. 33-3.43(1H, m), 4. 13(1H, dd) , 4.47(1H, dd) , 7 .09(2H, d) , 7.35-7.40(3H, m) , 7.44(1H, s) , 8.51(1H, s) |
| A-537 | 1.17(3H, d) , 2.18(1H, t) , 3.42-3. 53 (1H, m) , 3. 84(1H, dd) , 4.19(1H, ddd), 4.48(1H, dd) , 4.54(1H, q, J=7.38Hz) , 7.18(2H, d) , 7. 37 (2H, d) , 7.43(1H, s ), 7. 43 (1H, s) , |

Table 7

| Compound No. | 1H-NMR [$CDCl_3$/TMS, 400 MHz] |
|---|---|
| C-1 | 1.30 (3H, d), 4.14-4.23 (1H, m), 4.29 (1H, dd), 4.76 (1H, dd), 7.48 (2H, t), 7.60 (1H, t), 7.91 (2H, d), 8.20 (1H, s) |
| C-2 | 1.30 (3H, d), 4.10-4.20 (1H, m), 4.28 (1H, dd), 4.75 (1H, dd), 7.12-7.18 (2H, m), 7.92-7.97 (2H, m), 8.20 (1H, s) |
| C-3 | 1.24 (3H, d), 4.15-4.29 (2H, m), 4.61 (1H, dd), 6.40 (1H, d), 7.12 (2H, t), 7.43 (1H, s), 7.90-7.96 (2H, m) |
| C-4 | 1.23 (3H, d), 4.15-4.29 (2H, m), 4.61 (1H, dd), 6.40 (1H, d), 7.41-7.44 (3H, m), 7.83 (1H, dt) |
| C-5 | 1.29 (3H, d), 4.09-4.18 (1H, m), 4.28 (1H, dd), 4.74 (1H, dd), 7.62 (2H, dt), 7.77 (2H, dt), 8.20 (1H, s) |
| C-6 | 1.23 (3H, d), 4.13-4.23 (1H, m), 4.25 (1H, dd), 4.61 (1H, dd), 6.40 (1H, d), 7.43 (1H, d), 7.59 (2H, dt), 7.75 (2H, dt) |

(continued)

| Compound No. | 1H-NMR [$CDCl_3$/TMS, 400 MHz] |
|---|---|
| C-7 | 1.31 (3H, t), 4.00-4.19 (1H, m), 4.26-4.33 (1H, m), 4.72-4.80 (1H, m), 7.14 (0.3H, dd), 7.25 (0.3H, t), 7.30 (0.7H, dt), 7.47 (0.7H, dt), 7.53-7.62 (1H, m), 7.69 (0.7H, d), 7.80 (0.3H, dt), 8.20 (0.7H, s), 8.24 (0.3H, s) |
| C-8 | 1.22 (3H, d), 4.05 (1H, m), 4.28 (1H, dd), 4.73 (1H, dd), 7.32 (2H, m), 7.41 (2H, m), 8.23 (1H, s) |
| C-10 | 1.31 (3H, d), 4.11-4.19 (1H, m), 4.29 (1H, dd), 4.75 (1H, dd), 5.65 (0.5H, t), 5.94-5.96 (0.5H, m), 7.20-7.33 (1H, m), 7.39-7.52 (1H, m), 7.59 (1H, ddd), 7.68 (1H, dt), 8.20 (1H, s) |
| C-11 | 1.29 (3H, d), 4.11 (1H, m), 4.26 (1H, dd), 4.73 (1H, dd), 7.40 (1H, dd), 7.44 (1H, d), 7.55 (1H, d), 7.76 (1H, s), 8.19 (1H, s) |
| C-12 | 1.23 (3H, d), 4.13 (1H, m), 4.25 (1H, dd), 4.62 (1H, dd), 6.40 (1H, d), 7.37 (1H, m), 7.52 (1H, d), 7.74 (1H, d), 7.86 (1H, s) |
| C-13 | 1.24 (3H, d), 4.12 (1H, m), 4.30 (1H, dd), 4.75 (1H, dd), 7.34 (1H, dd), 7.73 (1H, dd), 7.81 (1H, dd), 8.03 (1H, s), 8.20 (1H, s) |
| C-14 | 1.24 (3H, d), 4.14 (1H, m), 4.25 (1H, dd), 4.62 (1H, dd), 6.41 (1H, d), 7.31 (1H, dd), 7.43 (1H, s), 7.68 (1H, d), 7.70 (1H, d), 8.02 (1H, d) |
| C-15 | 1.28 (3H, d), 4.02-4.09 (1H, m), 4.27 (1H, dd), 4.75 (1H, dd), 7.19 (1H, m), 7.39 (1H, m), 7.53 (1H, m), 8.23 (1H, s) |
| C-16 | 1.28 (3H, dd), 3.98-4.03 (1H, m), 4.27 (1H, dd), 4.75 (1H, ddd), 6.89 (1H, ddd), 6.98 (1H, ddd), 7.88 (1H, dt) |
| C-17 | 1.24 (3H, d), 4.04-4.06 (1H, m), 4.24 (1H, dd), 4.64 (1H, dd), 6.44 (1H, s), 7.13 (1H, m), 7.23 (1H, m), 7.46 (1H, m), 7.48 (1H, s) |
| C-18 | 1.30(6H, dd) , 4.07-4.17(1H, m) , 4.28(1H, dd), 4.73(1H, dd) , 7.27(1H, dd) , 7.67-7.72(1H, m) , 7.76 (1H, dd), 8.20(1H, s) |
| C-19 | 1.28(3H, d) , 4.13(1H, m), 4.27(1H, dd), 4.74(1H, dd), 7.57(1H, d), 7.71 (1 H, d) , 8.00(1H, s) , 8.19(1H, s) |
| C-20 | 1.24(3H, d) , 4.14 (1H, m) , 4.25(1H, dd), 4. 60(1H, dd), 6.40(1H, d), 7.42(1 H, s), 7.53(1H, d) , 7.70(1H, d), 7.97(1H, d) |
| C-21 | 0.97(3H, t), 1.70(1H, m) , 1.85(1H, m), 4.11 (1H, m), 4.31 (1H, dd), 4.76(1H , dd) , 7.46(2H, dd) , 7.56(1H, dd), 7.87(2H, d) , 8.15(1H, s) |
| C-22 | 0.92 (3H, t), 1.63(1H, m) , 1.77(1H, m) , 4.19(1H, m), 4. 32(1H, dd), 4.60(1H , dd) , 6.34(1H, d) , 7.38(1H, d), 7.42(2H, dd) , 7.53(1H, dd) , 7.83 (2H, d) |
| C-23 | 1.46 (6H, s), 4.53 (2H, s) , 7.40-7.45 (2H, m) , 7.49-7.54 (1H, m) , 7.65-7.68 (2H, m) , 8.30(1H, s) |
| C-24 | 1.39 (6H, s), 4.50 (2H, s), 6.48 (1H, d), 7.38-7.42(2H, m), 7.46-7.50 (2H, m ) , 7.56-7. 58 (2H, m) |
| C-25 | 1.47(6H, s), 4.51(2H, s), 7.11(2H, t), 7.77 (2H, dd), 8.32(1H, s) |
| C-26 | 1.46(6H, s), 4.51(2H, s), 7.41(2H, dt), 7.64(2H, dt), 8. 30(1H, s) |
| C-21 | 1.38(6H, s), 4.48(2H, s), 6.48(1H, d), 7.33 (2H, dt), 7.48(1H, d), 7.55 (2H , dt) |
| C-28 | 1.45(6H, s), 4.51(2H, s), 7.56(4H, d), 8.30(1H, s) |
| C-30 | 1,31(6H, s), 4.52(2H, s) , 7.09(1H, t), 7.15-7.18(2H, m), 7.42(1H, m) , 8.2 1(1H, s) |
| C-31 | 1. 26 (6H, s) , 4.47 (2H, s) , 6.49 (1H, d) , 7.05 (1H, dd) , 7.07 (1H, dd) , 7. 38 (1 H, m), 7.46 (1H, d) |
| C-32 | 1.34(6H, s) , 4.54(2H, s) , 7.05(1H, d), 7.27-7.38(3H, m), 8.29(1H, s) |
| C-33 | 1.45 (6H, s) , 4.51(2H, s) , 7.22(1H, m) , 7.36(1H, d), 7.42-7.43(2H, m), 8.3 0(1H, s) |
| C-34 | 1.37(6H, s) , 4.48(2H, s),6.48(1H, d), 7.18(1H, m), 7.27(1H, m), 7.34-7.7 .39(2H, m), 7. 48(1H, d) |

(continued)

| Compound No. | 1H-NMR [$CDCl_3$/TMS, 400 MHz] |
|---|---|
| C-35 | 1.44(6H, s) , 4.51(2H, s) , 7.36(1H, dd), 7.50-7.52 (2H, m), 7.61(1H, dd) , 8 .29(1H, s) |
| C-36 | 1.37(6H, s) , 4.48(2H, s), 6.49(1H, d), 7.31 (1H, dd), 7. 41-7.47 (3H, m), 7. 52(1H, m) |
| C-37 | 1.44(6H, s) , 4.50 (2H. s) , 7.30(1H, dd) , 7.55(1H, d) , 7.63(1H, d) , 7.76(1H , s) , 8. 29(1H, s) |
| C-38 | 1.37(6H, s) , 4.47(2H, s) , 6.49(1H, d) , 7.27(1H, s) , 7.46-7.48(2H, m) , 7.6 2(1H, dd) , 7.67(1H, d) |
| C-39 | 1.30(6H, d) , 4.51 (2H, s) , 6.84(1H, tt), 6.92 (1H, dt) , 7.23 (1H, q) , 8.20 (1 H, s) |
| C-40 | 1.31 (6H, d) , 4.51(2H, s) , 6.92-6.96(1H, m), 7.03-7.15(2H, m), 8.20(1H, s) |
| C-41 | 1.44(6H, s) , 4.51 (2H,s), 6.97(1H, tt), 7.14-7.19(2H, m), 8.29(1H, s) |
| C-42 | 0.96(3H, d) , 1.11(3H, d) , 2.21(1H, m) , 4.08(1H, m) , 4.36(1H, dd) , 4.81(1H , dd) , 7.44(2H, dd) , 7.56(1H, dd) , 7.82(2H, d) , 8.11(1H, s) |
| C-43 | 1.31(3H, d) , 4.04-4.15(1H, m) , 4.29(1H, dd) , 4.74(1H, dd) , 7.06(1H, tt) , 7.39-7.45(2H, m), 8.20(1H, s). |
| C-44 | 1.25(3H, d) , 4. 09-4.26(2H, m) , 4.60(1H, dd) , 7.41(1H, t) , 7.55(1H, ddd) , 7.67(1H, s) , 7.70(1H, s), 7.78(1H, dt), 7.87(1H, t). |
| C-45 | 1.29(3H, d), 3.75-3087(1H, q) , 4.06(1H, dd) , 4.52(1H, dd) , 7.28(1H, t) , 7 .43(1H, t) , 7.54(1H, s) , 7.58(1H, ddd) , 7.75 (1H, dt) , 7.87(1H, t). |
| C-46 | 1.32(3H, d), 4.21(1H, m) , 4.32(1H, dd), 4.77(1H, dd) , 7. 64(1H, dd) , 7.86( 1H, d), 8.09(1H, d), 8.16(1H, s), 8.22(1H, s)<br>ask-398-3 |
| C-47 | 1.27 (3H, d), 4.03(1H, m), 4.28(1H, dd), 4.75(1H, dd), 7.14(1H, m), 7.24(1 H, m) , 7.50(1H, m), 8.24(1H, s) |
| C-48 | 1.31 (3H, dd), 3.96-4.07(1H, m) , 4.29(1H, dd), 4.75(1H, ddd), 7.12-7.20( 1H, m), 7.26-7.32(1H, m), 8.24(1H, s). |
| C-49 | 1.30(3H, d) , 4.06-4.13 (1H, m) , 4.29(1H, dd) , 4.73(1H, dd) , 7.58(1H, t) , 7 .75(2H, d) , 8.20(1H, s). |
| C-50 | 1.29(3H, d) , 2.41(3H, s), 4.09-4. 20(1H, m), 4.28(1H, dd) , 4.75(1H, dd) , 7 .27(2H, d), 7.80 (2H, d), 8.20(1H, s). |
| C-51 | 1.29(3H, d) , 2.41(3H, s) , 4.16(1H, m) , 4.28 (1H, dd) , 4.75(1H, dd) , 7.33-7 .39(2H, m) , 7.69 (1H, d) , 7.70(1H, s) , 8.20(1H, s). |
| C-52 | 1.22(3H, d) , 2.41 (3H, s) , 4.01-10(1H, m) , 4.26 (1H, dd) , 4.74(1H, dd) , 7.2 3-7.28(2H, m), 7.39(1H, d), 7.50(1H, dd), 8.22 (1H, s). |
| C-53 | 1.35(3H, d) , 4.19-4.30(1H, m), 4.34(1H, dd) , 4.78(1H, dd) , 7.71 (1H, t) , 8 .22-8.26(2H, m) , 8.46(1H, ddd), 8.74(1H, t). |
| C-54 | 1.29(3H, d) , 3.87 (3H, s) , 4.12(2H, m) , 4.27 (1H, dd) , 4.75 (1H, dd), 6.93 (2 H, d), 7.89 (2H, d) , 8.19(1H, s) |
| C-55 | 1.31 (3H, d), 4.15-4.24(1H, m) , 4.32(1H, dd) , 4.76(1H, dd) , 7.79(2H, d) , 8 .00(2H, d), 8.23(1H, s). |

| | |
|---|---|
| C-56 | 1.31 (3H, d), 4.11-4.19(1H, m), 4.29(1H, dd) , 4.75(1H, dd) , 7.30(2H, d) , 7 .97(2H, q) , 8.20(1H, s). |
| C-57 | 1.24(3H,d),4. 30-4. 41 (2H, m), 4.69(1H, q), 6.84(1H, s), 7.46(2H, d), 7.9 0(2H, d). |
| C-58 | 1.23(3H, d) , 2.30(3H, s) , 4.10(1H, dd) , 4.28-4.38(1H, m) , 4.45(1H, dd) , 6 .15(1H, s), 7.41 (2H, d), 7.83 (2H, d) |
| C-59 | 1.21 (3H, d) , 4.08-4. 20 (2H, m) , 4.55 (1H, dd) , 7.41-7.45(4H, m) , 7.85(2H, d). |
| C-60 | 1.21 (3H, d), 4.18-4. 18(2H, m), 4.53(1H, q) , 7.38(2H, s) , 7.43(2H, d) , 7.8 5(2H, d). |

Table 8

| Compound No. | 1H-NMR [$CDCl_3$/TMS, 400 MHz] |
|---|---|
| D-1 | 1.25 (3H, d), 2.31 (1H, t), 3.63-3.72 (1H, m), 3.79-3.85 (1H, m), 3.90-3.97 (1H, m), 7.46-7.52 (2H, m), 7.59 (1H, tt), 7.96-7.98 (2H, m) |
| D-5 | 1.22 (3H, dd), 2.21 (1H, t), 3.51-3.61 (1H, m), 3.79-3.85 (1H, m), 3.87-3.95 (1H, m), 7.15 (1H, ddd), 7.25 (1H, dt), 7.51-7.57 (1H, m), 7.82 (1H, dt) |
| D-6 | 1.17 (3H, d), 2.16 (1H, br), 3.50 (1H, m), 3.81 (1H, m), 3.88 (1H, m), 7.33-7.43 (4H, m) |
| D-8 | 1.24 (3H, d), 2.22 (1H, t), 3.58-3.66 (1H, m), 3.78-3.84 (1H, m), 3.92-3.98 (1H, m), 7.29 (1H, ddt), 7.47 (1H, dt), 7.65 (1H, ddd), 7.74-7.77 (1H, m) |
| D-9 | 1.24 (3H, d), 2.19 (1H, br), 3.62 (1H, m), 3.80 (1H, m), 3.93 (1H, m), 7.43 (1H, dd), 7.54 (1H, d), 7.82 (1H, dd), 7.93 (1H, s) |
| D-10 | 1.23 (3H, d), 2.24 (1H, br), 3.61 (1H, m), 3.80 (1H, dd), 3.92 (1H, dd), 7.35 (1H, dd), 7.70 (1H, d), 7.87 (1H, d), 8.09 (1H, s) |
| D-11 | 1.24 (3H, d), 2.20 (1H, br), 3.68 (1H, m), 3.81 (1H, dd), 3.96 (1H, dd), 7.62 (1H, dd), 7.84 (1H, d), 8.15 (1H, d), 8.22 (1H, s) |
| D-12 | 1.21 (1H, d), 2.12 (1H, br), 3.52 (1H, m), 3.82 (1H, m), 3.90 (1H, m), 7.19 (1H, m), 7.35 (1H, m), 7.56 (1H, m) |
| D-13 | 1.21 (6H, dd), 2.20 (1H, t), 3.48-3.56 (1H, m), 3.77-3.84 (1H, m), 3.86-3.96 (1H, m), 6.89 (1H, dd), 6.98 (1H, dd), 7.90 (1H, dt) |
| D-14 | 1.22 (3H, d), 2.10 (1H, br), 3.54 (1H, m), 3.82 (1H, m), 3.89 (1H, m), 7.11 (1H, m), 7.15 (1H, m), 7.51 (1H, m) |
| D-15 | 1.22 (3H, d), 2.21 (1H, t), 3.55-3.65 (1H, m), 3.75-3.83 (1H, m), 3.89-3.98 (1H, m), 7.26 (1H, dd), 7.73-7.78 (1H, m), 7.81 (1H, dd) |
| D-16 | 1.21 (3H, d), 2.17 (1H, br), 3.60 (1H, m), 3.80 (1H, m), 3.91 (1H, m), 7.58 (1H, d), 7.79 (1H, d), 8.04 (1H, s) |
| D-17 | 1.40 (6H, s), 2.56 (1H, t), 4.12 (2H, d), 7.41-7.43 (2H, m), 7.48-7.53 (1H, m), 7.75-7.78 (2H, m) |
| D-18 | 1.40 (6H, s), 3.68 (2H, s), 7.11 (2H, t), 7.86 (2H, dd) |
| D-19 | 1.38 (6H, s), 2.48 (1H, t), 3.68 (2H, d), 7.40 (2H, dt), 7.74 (2H, dt) |
| D-20 | 1.37 (6H, s), 2.46 (1H, t), 3.68 (2H, d), 7.57 (2H, dt), 7.65 (2H, dt) |
| D-21 | 1.24(6H, s) , 3.70(2H, s) , 7.11(1H, m) , 7.16(1H, m) , 7.28(1H, m) , 7.41(1H, m ) |
| D-22 | 1.26(6H, s) , 3.73(2H, s) , 7. 22(1H, dd), 7. 29-7. 7.40(2H, m) , 7.42(1H, d) |
| D-24 | 1. 37(6H, s), 2.40(1H, br) , 3.68(2H, d) , 7.19(1H, m) , 7. 37-7.45(2H, m) , 7. 5 4(1H, d) |
| D-25 | 3.41 (6H, s), 4.11 (2H,s), 7.31-7.63 (3H, m), 7.70(1H, s) |
| D-26 | 1.36(6H, s) , 2.40(1H, br), 3.67(2H, s), 7.30(1H, m), 7.62-7.67 (2H, m), 7.8 5(1H, s) |
| D-27 | 1.24(6H, d) , 3.69(2H, s), 6.86(1H, dt), 6.92(1H, ddt), 7.31 (1H, dt) |
| D-28 | 1.24 (6H, d), 2.20(1H, t), 3.70(2H, d), 6.97-7.10 (3H, m) |
| D-29 | 1.39 (6H, d) , 3.69(2H, s) , 7.22(1H, dd) , 7.58-7.64(1H, m) , 7.67(1H, ddd) |
| D-30 | 0.97(3H, t), 1.66-1.78(2H, m), 2.28(1H, t), 3.54(1H, m), 3.86(1H, m), 3.98 (1H, m) , 7.49(2H, dd) , 7.59 (1H, dd), 7.96 (2H, d) |
| D-31 | 1.23(3H, d) , 2.18(1H, t) , 3.53-3.61 (1H, m), 3.77-3.82(1H, m) , 3.91-3.97( 1H, m) , 7.04(1H, tt), 7.44-7.50(2H, m) . |
| D-32 | 1.22(3H, dd), 2.11 (1H, t), 3.47-3.56(1H, m), 3.78-3.85(1H, m) , 3.88-3.97 (1H, m) , 7.09-7.18(1H, m) , 7.27-7.33(1H, m) . |
| D-33 | 1.22(3H, d) , 2.08(1H, t) , 3.54-3. 62(1H, m) , 3.77-3. 82 (1H, m) , 3.91-4.13( 1H, m) , 7.57(1H, t) , 7.81 (2H, d). |

(continued)

| Compound No. | 1H-NMR [$CDCl_3$/TMS, 400 MHz] |
|---|---|
| D-34 | 1.23(3H, d) , 2.42(3H, s), 2. 43(1H, t), 3. 59-3.70(1H, m) , 3.77-3.84(1H, m) , 3.87-3.96(1H, m) , 7.28(2H, d), 7.87 (2H, d). |
| D-35 | 1.24(3H, d) , 2.34(1H, t) , 2.42(3H, s) , 3.66(1H, m) , 3.81(1H,m) , 3.93(1H, m ), 7.34-7.39 (2H, m) , 7.74 (1H, d), 7.77 (1H, s) |
| D-36 | 1.15(3H, d) , 2.34(1H, t) , 2.46(3H, s) , 3.45-3.55 (1H, m) , 3.74-3.82(1H, m) , 3.87-3.56 (1H, m) , 7.23-7.30(2H, m) , 7.38(1H, dt) , 7.59(1H, dd) . |
| D-37 | 1.24(3H, d) , 1.62(1H, s) , 3. 62(1H, m) , 3. 81 (1H, dd) , 3. 88 (3H, s) , 3. 91(1H, m), 6. 96(2H, d), 7.96(2H, d) |
| D-38 | 1.24(3H, d) , 2.23(1H, t), 3.60-3.69(1H, m) , 3.78-3.83(1H, m) , 3.91-3.98( 1H, m), 7.31 (2H, d), 8. 03(2H, d). |
| D-39 | 1.26(3H, d) , 3. 68-3. 77(1H, m) , 3.84(1H, dd) , 3.98(1H, dd) , 7.71 (1H, t) , 8. 30(1H, dt) , 8.44 (1H, ddd), 8.80(1H, t). |
| D-40 | 1.23(3H, d) , 2. 2(1H, t) , 3. 62-3. 70(1H, m), 3.79-3.84(1H, m) , 3.93-3. 99 ( 1H, m) , 7.79(2H, d) , 8. 05 (2H, d) |
| D-41 | 0.95(3H, d) , 0.97(3H, d) , 1.61(1H, s) , 2. 20 (2H, m), 3.47(1H, m), 3.83(1H, m ), 4.08(1H, m) , 7.48(2H, t), 7.59(1H, t), 7.96 (2H, t) |

Table 9

| Compound No. | 1H-NMR [CDCb/TMS, 400 MHz] |
|---|---|
| E-8 | 1.32 (3H, d), 3.59 (1H, dd), 3.75-3.83 (1H, m), 3.93 (1H, dd), 7.30 (1H, ddt), 7.48 (1H, dt), 7.63-7.67 (1H, m), 7.75 (1H, d) |
| E-13 | 1.36 (6H, s), 2.45 (3H, s), 4.15 (2H, s), 7.33 (2H, d), 7.36-7.41 (2H, m), 7.43-7.51 (1H, m), 7.57-7.60 (2H, m), 7.74 (2H, d) |
| E-14 | 1.37 (6H, s), 2.45 (3H, s), 4.14 (2H, s), 7.05 (2H, t), 7.32 (2H, d), 7.66 (2H, dd), 7.73 (2H, d) |
| E-15 | 1.36 (6H, s), 2.46 (3H, s), 4.13 (2H, s), 7.31-7.35 (4H, m), 7.54 (2H, dt), 7.71 (2H, d) |
| E-16 | 1.37 (6H, s), 2.46 (3H, s), 4.12 (2H, s), 7.32 (2H, d), 7.46 (2H, dt), 7.50 (2H, dt), 7.72 (2H, d) |
| E-17 | 1.24 (6H, s), 2.45 (3H, s), 4.10 (2H, s), 7.07 (1H, m), 7.16 (1H, m), 7.34 (2H, d), 7.40 (1H, m), 7.76 (2H, d) |
| E-18 | 1.25 (6H, s), 2.46 (3H, s), 4.12 (2H, s), 7.14 (1H, dd), 7.27-7.37 (5H, m), 7.78 (2H, d) |
| E-20 | 1.35 (6H, s), 2.45 (3H, s), 4.13 (2H, s), 7.16 (1H, m), 7.24 (1H, m), 7.32-7.37 (4H, m), 7.75 (2H, d) |
| E-21 | 1.35 (6H, s), 2.45 (3H, s), 4.13 (2H, s), 7.31-7.33 (3H, m), 7.42-7.49 (3H, m), 7.74 (2H, d) |
| E-22 | 1.35 (6H, s), 2.45 (3H, s), 4.12 (2H, s), 7.25 (1H, dd), 7.32 (2H, d), 7.48 (1H, d), 7.58 (1H, d), 7.64 (1H, s), 7.74 (2H, d) |
| E-23 | 1.24 (6H, d), 4.09 (2H, s), 6.80 (1H, dd), 6.90 (1H, ddt), 7.21 (1H, dd), 7.35 (2H, d), 7.74 (2H, d) |
| E-24 | 1.25 (6H, d), 2.46 (3H, s), 4.10 (2H, s), 6.83 (1H, dt), 7.00-7.12 (2H, m), 7.36 (2H, d), 7.76 (2H, d) |
| E-25 | 1.37 (6H, s), 2.46 (3H, s), 4.13 (2H, s), 7.16 (1H, ddd), 7.32 (2H, d), 7.40-7.48 (2H, m), 7.71 (2H, d) |

Hereinafter, formulations containing the compound of the present invention will be specifically described with reference to Formulation Examples, but the scope of the present invention is not limited in any way by the preparation methods of these formulations. In the Formulation Examples, all parts are parts by mass.

Formulation Example 1 Emulsifiable concentrate

The compound of the present invention (10 parts), xylene (60 parts), N-methyl-2-pyrrolidone (20 parts), and Sorpol 3005X (a mixture of a nonionic surfactant and an anionic surfactant, Toho Chemical Industry Co., Ltd., trade name) (10 parts) were uniformly mixed and dissolved to obtain an emulsifiable concentrate.

Formulation Example 2 Wettable powder

The compound of the present invention (20 parts), Tokusil GU-N (white carbon, Oriental Silicas Corporation) (20 parts), Sorpol 5096 (polyoxyethylene styrylphenyl ether sulfate, Toho Chemical Industry Co., Ltd.) (10 parts), and SS clay (clay, Showa KDE Co., Ltd.) (50 parts) were mixed and then pulverized using a pulverizer to obtain a wettable powder.

Formulation Example 3 Water-soluble powder

The compound of the present invention (5 parts), Runox P-65L (sodium alkylbenzenesulfonate, Toho Chemical Industry Co., Ltd.) (3 parts), and sodium bicarbonate (reagent) (92 parts) were uniformly mixed and then pulverized using a pulverizer to obtain a water-soluble powder.

Formulation Example 4 Water-dispersible granules

The compound of the present invention (20 parts), Demol N (naphthalenesulfonate formalin condensate, Kao Corporation, trade name) (5 parts), Aerosol CT-1L (dioctyl sulfosuccinate salt, Toho Chemical Industry Co., Ltd., trade name) (1 part), Celogen 701A (carboxymethyl cellulose, Dai-ichi Kogyo Seiyaku Co., Ltd.) (1 part), and kaolin clay (kaolinite, Takehara Chemical Industrial Co., Ltd., trade name) (73 parts) were uniformly mixed and pulverized in an air mill. Water was added to this mixture and kneaded well, then extruded and granulated, and dried and sized to obtain water-dispersible granules.

Formulation Example 5 Flowable agent

The compound of the present invention (20 parts), Sorpol 7948 (sodium polyoxyalkylene tristyrylphenyl ether sulfate, Toho Chemical Industry Co., Ltd.) (5 parts), propylene glycol (reagent) (10 parts), and water (40 parts) were mixed in advance and wet-milled with a bead mill to obtain a slurry. Next, KELZAN (xanthan gum, Sansho Co., Ltd.) (0.2 parts) was mixed and dispersed well in water (24.8 parts) to prepare a gel-like substance, which was thoroughly mixed with the milled slurry to obtain a flowable agent.

Formulation Example 6 EW-1

The compound of the present invention (20 parts) and Newkalgen D-230 (polyoxyethylene castor oil, Takemoto Oil & Fat Co., Ltd.) (15 parts) were mixed and homogenized; then, while stirring, water (59.8 parts) was gradually added to obtain a dispersion. To this dispersion, KELZAN (xanthan gum, Sansho Co., Ltd.) (0.2 parts) dispersed in propylene glycol (reagent) (5.0 parts) was added to obtain an emulsion formulation.

Formulation Example 7 EW-2

The compound of the present invention (10 parts) was dissolved in xylene (10 parts) and mixed with Rheodol 430V (polyoxyethylene sorbitan tetraoleate, Kao Corporation) (24 parts) to obtain a dispersion. This dispersion and water (50.8 parts) were dispersed with a homogenizer, and KELZAN (xanthan gum, Sansho Co., Ltd.) (0.2 parts) dispersed in propylene glycol (reagent) (5.0 parts) was added to obtain an emulsion formulation.

Formulation Example 8 Microemulsion

The compound of the present invention (10 parts), xylene (reagent) (20 parts), Newkalgen D-230 (polyoxyethylene castor oil, Takemoto Oil & Fat Co., Ltd.) (20 parts), Aerosol CT-1L (sodium dioctyl sulfosuccinate, Toho Chemical Industry Co., Ltd.) (2.0 parts), and water (48 parts) were uniformly mixed to obtain a microemulsion.

Formulation Example 9 Solution

The compound of the present invention (20 parts), N-methyl-2-pyrrolidone (reagent) (70 parts), and Newkalgen

D-230 (polyoxyethylene castor oil, Takemoto Oil & Fat Co., Ltd.) (10 parts) were uniformly mixed and dissolved to obtain a solution.

Formulation Example 10 Granules-1

The compound of the present invention (5 parts), Aerosol CT-1L (sodium dioctyl sulfosuccinate, Toho Chemical Industry Co., Ltd.) (2 parts), Bentonite Sado (bentonite, Kunimine Industries Co., Ltd.) (30 parts), and NK-300 (clay, Showa KDE Co., Ltd.) (63 parts) were uniformly mixed, water was added and kneaded well, then extruded and granulated, and dried and sized to obtain granules.

Formulation Example 11 Granules-2

The compound of the present invention (5 parts) was dissolved in methanol (reagent), adsorbed onto Ishikawa Light No. 2 (95 parts) using a tumbling granulator, and dried to obtain granules.

Formulation Example 12 Fine granules-1

The compound of the present invention (2 parts) was dissolved in methanol (reagent), adsorbed onto Ishikawa Light No. 4 (98 parts) using a tumbling granulator, and dried to obtain fine granules.

Formulation Example 13 Fine granules-2

The compound of the present invention (5 parts) and Tokusil GU-N (5 parts) were mixed and then pulverized using a fine pulverizer, and uniformly mixed with Iide silica sand (silica sand, JFE Mineral Company, Ltd.) (80 parts). To this mixture, a diluted aqueous solution of Toxanon GR-31A (polycarboxylic acid type surfactant, Sanyo Chemical Industries, Ltd.) (10 parts) was sprayed while further mixing to obtain a granular composition. This granular composition was dried to obtain fine granules.

Formulation Example 14 Dust

The compound of the present invention (5 parts), Tokusil GU-N (5 parts), and Taisei fine powder clay (clay, Taisei Sangyo Co., Ltd.) (90 parts) were uniformly mixed and then pulverized using a pulverizer to obtain a dust.

Formulation Example 15 DL Dust

The compound of the present invention (5 parts), propylene glycol (0.5 parts), and DL clay (94.5 parts) were uniformly mixed and then pulverized using a pulverizer to obtain a dust.

Formulation Example 16 Seed coating powder

The compound of the present invention (10 parts), Poval PVA-117 (polyvinyl alcohol, Kuraray Trading Co., Ltd.) (1 part), and Taisei fine powder clay (89 parts) were uniformly mixed and pulverized using a fine pulverizer to obtain a powder. This powder was mixed with pre-moistened seeds, and air-dried to obtain coated seeds.

Formulation Example 17 Seed coating flowable agent

The compound of the present invention (10 parts), Sorpol 7948 (5 parts), propylene glycol (reagent) (10 parts), and water (40 parts) were mixed in advance and wet-milled with a bead mill. Next, KELZAN (xanthan gum, Sansho Co., Ltd.) (0.2 parts) and water (29.8 parts) were mixed and dispersed well, and a gel-like substance mixed with AMECOAT HCA/83 (acrylic acid polymer, SOLVAY) (5 parts) was prepared. The milled slurry and the prepared gel-like substance were thoroughly mixed to obtain a flowable agent. This flowable agent was mixed with seeds and air-dried to obtain coated seeds.

Formulation Example 18 Oil-based suspension formulation

The compound of the present invention (20 parts), Newkalgen C-120 (POE tristyrylphenyl ether, Takemoto Oil & Fat Co., Ltd., trade name) (5 parts), Rheodol TWO120V (POE sorbitan monooleate, Kao Corporation, trade name) (10 parts), methyl oleate (Kanto Chemical Co., Inc., reagent) (62 parts), and Esben NZ (organobentonite, Hojun Co., Ltd.,

trade name) (3 parts) were thoroughly mixed and dispersed, and this slurry-like dispersion was wet-milled to obtain an oil-based suspension formulation.

Next, the effects and usefulness of the compound of the present invention will be explained with specific examples. The compound used for comparative control is indicated by the following compound symbol.

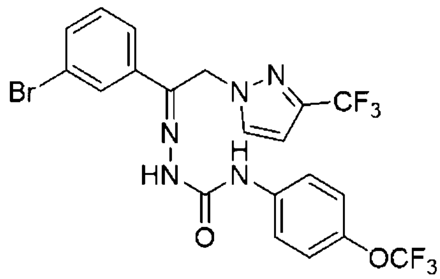

Comparative Agent A (No. 6 described in Japanese Unexamined Patent Application Publication No. Hei 08-073436)

Test Example 1: Efficacy test against the southern root-knot nematode (Meloidogyne incognita (Kofoid et White) Chitwood)

200 ml of test soil, with a nematode density adjusted to 200 nematodes/20 g of soil, was placed in a styrene cup (diameter 9 cm, height 6.5 cm). 50 ml of an aqueous dilution (400 ppm) of an emulsifiable concentrate prepared according to Formulation Example 1 was drenched into the soil, and then tomatoes were sown (13 seeds/pot). The plants were grown in a greenhouse after sowing. On the 17th day after sowing, the degree of root galling of each plant was investigated on a 5-point scale based on the following evaluation criteria, and the root-knot index and further the control value were calculated based on the following formula.

Degree of root galling (evaluation criteria): 0 (no root galls), 1 (1 to 2 root galls), 2 (3 or more root galls), 3 (large root galls or connected root galls, less than 50% of the entire root system), 4 (root galls on 50% or more of the entire root system)

Root-knot index=$\Sigma$ (degree of root galling$\times$number of plants with that degree)/(4$\times$number of plants investigated)

Control value (%)=[1-(root-knot index of treated plot/root-knot index of untreated plot)]$\times$100

As a result, the compounds of the present invention with compound numbers (A-1, A-5, A-17, A-21, A-22, A-27, A-28, A-30, A-33, A-42, A-47, A-51, A-52, A-71, A-75, A-83, A-87, A-91, A-95, A-121, A-125, A-131, A-132, A-133, A-134, A-135, A-136, A-137, A-138, A-139, A-140, A-141, A-142, A-143, A-144, A-146, A-147, A-148, A-149, A-151, A-161, A-171, A-176, A-177, A-178, A-179, A-181, A-182, A-184, A-185, A-186, A-194, A-195, A-196, A-197, A-234, A-237, A-238, A-244, A-254, A-264, A-269, A-270, A-271, A-272, A-274, A-277, A-287, A-288, A-295, A-296, A-297, A-298, A-300, A-303, A-307, A-308, A-341, A-342, A-343, A-344, A-345, A-346, A-349, A-351, A-371, A-372, A-373, A-374, A-375, A-376, A-377, A-378, A-379, A-381, A-382, A-383, A-384, A-386, A-389, A-391, A-393, A-394, A-396, A-411, A-412, A-414, A-421, A-431, A-441, A-442, A-502, A-507, A-508, A-509, A-510, A-511, A-515, A-516, A-517, A-519, A-523, A-524, A-525, A-526, A-527, C-37) showed a control value of 70% or more. On the other hand, Comparative Agent A had a control rate of 0%.

Test Example 2: Density suppression test against the tomato russet mite (Aculops lycopersici Massee)

A 430 ml capacity polyethylene cup filled with water was covered with a lid having a hole (approx. 5 mm in diameter) in the center. A circular filter paper with a diameter of 6.5 cm was cut with a slit about 5 mm wide, and a strip-like portion hanging downward was inserted through the hole in the lid so that it was immersed in the water inside the cup; absorbent cotton was placed on top of this filter paper. In this way, three leaf discs (1 cm x 1 cm) made from leaflets of tomato at the early stage of true leaf expansion were placed on the absorbent cotton, which was kept constantly supplied with water from the cup, with the back of the leaf facing up; small pieces of leaves infested with tomato rust mites were inoculated onto the discs. Twenty-four hours after inoculation, the small pieces were removed, and the cup was placed inside an acrylic cylinder 50 cm high and 10 cm in diameter; an aqueous dilution (500 ppm) of an emulsifiable concentrate prepared according to Formulation Example 1 was sprayed at a rate of 2.0 ml per cup using an airbrush. After spraying, the cups were kept in a constant temperature room at 25°C. Seven days after treatment, evaluation was made on a 4-point

scale of 100 (next-generation density suppression rate: 100%), 80 (ditto: 99-80%), 50 (ditto: 79-50%), and 0 (ditto: less than 50%), and the control rate was calculated based on the results using the following formula. The test was conducted with one cup per plot.

$$\text{Next-generation density suppression rate}=(A\times100+B\times80+C\times50)/(A+B+C+D)$$

A: number of discs with a score of 100, B: number of discs with a score of 80, C: number of discs with a score of 50, D: number of discs with a score of 0

As a result, the compounds of the present invention with compound numbers (A-1, A-5, A-17, A-21, A-22, A-26, A-27, A-30, A-33, A-42, A-46, A-47, A-51, A-52, A-56, A-75, A-95, A-121, A-125, A-131, A-132, A-133, A-134, A-135, A-136, A-137, A-138, A-139, A-140, A-141, A-142, A-143, A-144, A-145, A-146, A-147, A-148, A-149, A-161, A-171, A-176, A-177, A-178, A-179, A-181, A-182, A-184, A-185, A-186, A-187, A-197, A-234, A-237, A-238, A-254, A-264, A-269, A-270, A-271, A-272, A-274, A-275, A-276, A-277, A-285, A-286, A-287, A-288, A-289, A-295, A-296, A-297, A-298, A-299, A-300, A-301, A-302, A-303, A-304, A-305, A-307, A-308, A-309, A-341, A-342, A-343, A-344, A-346, A-347, A-348, A-349, A-351, A-350, A-371, A-372, A-373, A-374, A-375, A-376, A-377, A-378, A-379, A-381, A-382, A-383, A-384, A-385, A-387, A-388, A-389, A-391, A-392, A-393, A-394, A-396, A-398, A-399, A-411, A-412, A-414, A-421, A-431, A-502, A-503, A-504, A-505, A-509, A-515, A-516, A-517, A-525, A-526, A-527) showed a control rate of 90% or more. On the other hand, Comparative Agent A had a control rate of 0%.

## Claims

**1.** A hydrazone derivative represented by the following formula (1),

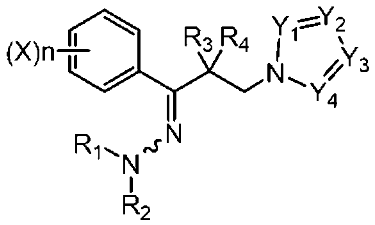

(1)

or a salt thereof,
wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_2$ represents a formyl group, or
$R_1$ and $R_2$ may, together, form a group represented by the following formula,

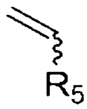

,

$R_5$ represents $OR_6$ or $NR_7R_8$,
$R_6$ represents a $C_{1-6}$ alkyl group,
$R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,

$Y_1$, $Y_2$, $Y_3$, and $Y_4$ each independently represent a nitrogen atom or a CR group, R represents a hydrogen atom, a halogen atom, a $C_{1-6}$ alkyl group, or a $C_{1-6}$ haloalkyl group, and when a plurality of Rs are present, they may be the same as or different from each other.

**2.** The hydrazone derivative or a salt thereof according to claim 1, wherein $Y_2$ represents CR, and R represents a $C_{1-6}$ haloalkyl group.

**3.** A hydrazone derivative represented by the following formula (2).

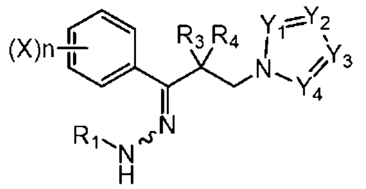
(2)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group, R represents a hydrogen atom, a halogen atom, a $C_{1-6}$ alkyl group, or a $C_{1-6}$ haloalkyl group, and when a plurality of Rs are present, they may be the same as or different from each other.

**4.** A ketone derivative represented by the following formula (3).

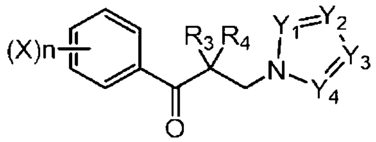
(3)

wherein in the formula, $R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other.

**5.** A method for producing a hydrazone derivative represented by the following formula (4),

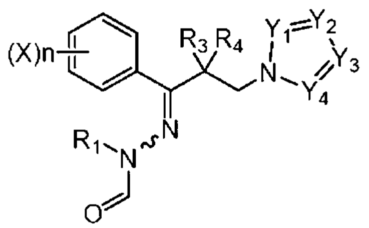
(4)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (2),

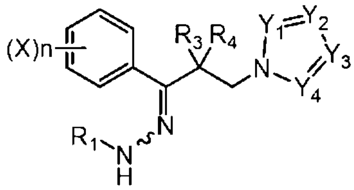
(2)

wherein in the formula, $R_1$, $R_3$, $R_4$, X, $Y_1$, $Y_2$, $Y_3$, $Y_4$, and n are as defined in the above formula (4),
with formic acid and/or a formate ester represented by the following formula (5), $HCOOR_9$ (5)
wherein in the formula, $R_9$ represents a $C_{1-6}$ alkyl group.

**6.** A method for producing a hydrazone derivative represented by the following formula (6),

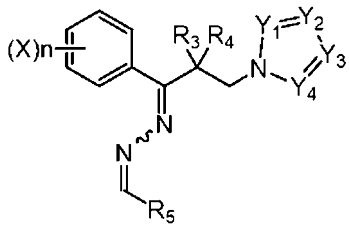
(6)

wherein in the formula, $R_5$ represents $OR_6$ or $NR_7R_8$,
$R_6$ represents a $C_{1-6}$ alkyl group,
$R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (7),

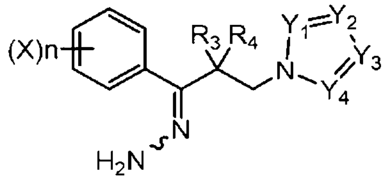

(7)

wherein in the formula, $R_3$, $R_4$, X, $Y_1$, $Y_2$, $Y_3$, $Y_4$, and n are as defined in the above formula (6), with an acetal derivative represented by the following formula (8),

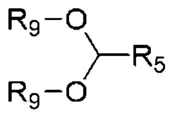

(8)

wherein in the formula, $R_5$ is as defined in the above formula (6), and $R_9$s represent a $C_{1-6}$ alkyl group and may be the same as or different from each other.

**7.** A method for producing a hydrazone derivative represented by the following formula (9),

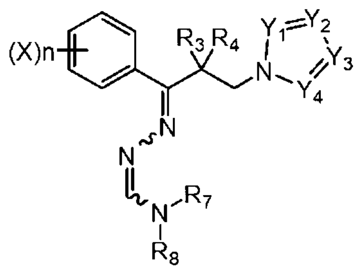

(9)

wherein in the formula, $R_7$ represents a hydrogen atom or a $C_{1-6}$ alkyl group,
$R_8$ represents a $C_{1-6}$ alkyl group, or
$R_7$ and $R_8$ may bond to each other and, together with the nitrogen atom to which $R_7$ and $R_8$ are bonded, form a saturated 4- to 7-membered ring,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group, R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (10),

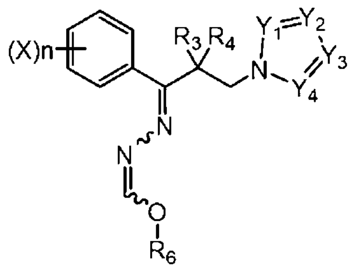

(10)

wherein in the formula, $R_3$, $R_4$, X, $Y_1$, $Y_2$, $Y_3$, $Y_4$, and n are as defined in the above formula (9), and $R_6$ represents a $C_{1-6}$ alkyl group,
with an amine derivative represented by the following formula (11),

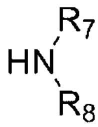 (11)

wherein in the formula, $R_7$ and $R_8$ are as defined in the above formula (9).

**8.** A method for producing a hydrazone derivative represented by the following formula (4),

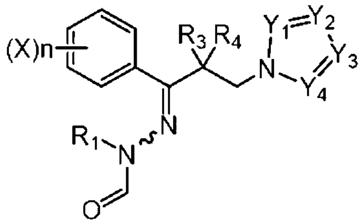 (4)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a hydrazone derivative represented by the following formula (12)

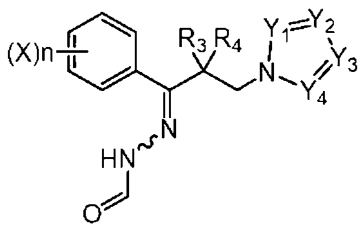 (12)

wherein in the formula, $R_3$, $R_4$, X, $Y_1$, $Y_2$, $Y_3$, $Y_4$, and n are as defined in the above formula (4),
with an alkylating agent represented by the following formula (13),

$Z$-$R_1$ (13)

wherein in the formula, $R_1$ is as defined in the above formula (4), and Z represents a chlorine atom, a bromine atom, an iodine atom, a methanesulfonyloxy group, a p-toluenesulfonyloxy group, or a trifluoromethanesulfonyloxy group.

**9.** A method for producing a hydrazone derivative represented by the following formula (2),

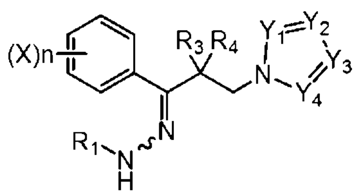 (2)

wherein in the formula, $R_1$ represents a hydrogen atom, a $C_{1-6}$ alkyl group, a $C_{2-6}$ alkenyl group, a $C_{2-6}$ alkynyl group, or a benzyl group,
$R_3$ represents a $C_{1-6}$ alkyl group,
$R_4$ represents a hydrogen atom or a $C_{1-6}$ alkyl group, or
$R_3$ and $R_4$ may, together, form a ($C_3$-$C_6$)-carbocycle,
X represents a hydrogen atom, a $C_{1-6}$ alkyl group, a $C_{1-6}$ haloalkyl group, a $C_{1-6}$ alkoxy group, a $C_{1-6}$ haloalkoxy group, a halogen atom, a cyano group, or a nitro group, and when a plurality of Xs are present, they may be the same as or different from each other,
n represents an integer of 1 to 5,
$Y_1$, $Y_2$, $Y_3$, and $Y_4$ each independently represent a nitrogen atom or a CR group,
R represents a hydrogen atom, a halogen atom, a $C_{1-6}$ alkyl group, or a $C_{1-6}$ haloalkyl group, and when a plurality of Rs are present, they may be the same as or different from each other,
the method comprising reacting a ketone derivative represented by the following formula (3),

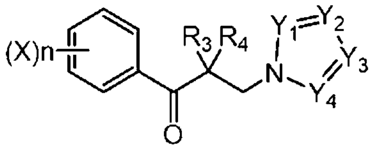

(3)

wherein in the formula, $R_3$, $R_4$, X, $Y_1$, $Y_2$, $Y_3$, $Y_4$, and n are as defined in the above formula (2),
with a hydrazine derivative represented by the following formula (14),

$NH_2NHR_1$ (14)

wherein in the formula, $R_1$ is as defined in the above formula (2).

**10.** An agricultural and horticultural chemical agent comprising the hydrazone derivative or a salt thereof according to claim 1 or 2.

**11.** An agricultural and horticultural nematode control agent comprising the hydrazone derivative or a salt thereof according to claim 1 or 2.

**12.** An agricultural and horticultural pest control agent comprising the hydrazone derivative or a salt thereof according to claim 1 or 2.

**13.** A method for controlling a plant pest, comprising a step of treating the pest and/or a habitat thereof and/or a seed and/or a plant propagation material with the agricultural and horticultural chemical agent according to claim 10.

**14.** A method for preventing a plant pest, comprising a step of treating a place where a useful crop is to be grown or is being grown, or a growing crop, with the agricultural and horticultural chemical agent according to claim 10.

**15.** A method for preparing an agrochemical composition, the method comprising a step of mixing the hydrazone derivative or a salt thereof according to claim 1 or 2 with any one of a carrier, an emulsifier, a wetting agent, a dispersing agent, and a disintegrant, or a combination of any of these.

**INTERNATIONAL SEARCH REPORT**

International application No. **PCT/JP2024/025816**

**A. CLASSIFICATION OF SUBJECT MATTER**

***C07D 231/12***(2006.01)i; ***A01N 43/48***(2006.01)i; ***A01N 43/50***(2006.01)i; ***A01N 43/653***(2006.01)i; ***A01P 5/00***(2006.01)i; ***A01P 7/00***(2006.01)i; ***C07D 233/64***(2006.01)i; ***C07D 249/08***(2006.01)i

FI: C07D231/12 E CSP; A01N43/48; A01N43/50 A; A01N43/653 A; A01P5/00; A01P7/00; C07D231/12 D; C07D233/64; C07D249/08 535

According to International Patent Classification (IPC) or to both national classification and IPC

**B. FIELDS SEARCHED**

Minimum documentation searched (classification system followed by classification symbols)

C07D231/12; A01N43/48; A01N43/50; A01N43/653; A01P5/00; A01P7/00; C07D233/64; C07D249/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | MENG, XIU-JIN et al., Electrochemical α-methoxymethylation and aminomethylation of propiophenones using methanol as a green C1 source, Organic Chemistry Frontiers, 2020, vol. 7, pp. 2399-2404, DOI 10.1039/d0qo00593b<br>chemical compounds 4d, 4h (table 3) | 4 |
| X | YANG, JINGYA et al., Cobalt-Catalyzed α-Methoxymethylation and Aminomethylation of Ketones with Methanol as a C1 Source, Organic Letters, 2018, vol. 20, pp. 6774-6779, DOI 10.1021/acs.orglett.8b02892<br>chemical compounds 5aa, 5ca, 5ja, 5ma, 5oa, 5pa (scheme 5) | 4 |
| X | SUN, KAI, Copper-Catalyzed N-Arylation of Azoles and Mannich-Type Coupling of Ketones and Azoles under Metal-Free Conditions, Journal of Organic Chemistry, 2016, vol. 81, pp. 1476-1483, DOI 10.1021/acs.joc.5b02593<br>chemical compounds 7s, 7t, 7u (table 4) | 4 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2024** | **08 October 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/025816**

**C. DOCUMENTS CONSIDERED TO BE RELEVANT**

| Category* | Citation of document, with indication, where appropriate, of the relevant passages | Relevant to claim No. |
|---|---|---|
| X | FU, NIANKAI et al., Chiral primary amine catalyzed asymmetric conjugate addition of azoles to α-substituted vinyl ketones, Organic Chemistry Frontiers, 2014, vol. 1, pp. 68-72, DOI 10.1039/C3QO00027C<br>chemical compounds 10a to 10d (table3), chemical compounds 13a, 13c, 13e (scheme 5) | 4 |
| X | SRINIVAS, NAGARAPU et al., Proline-catalyzed facile access to Mannich adducts using unsubstituted azoles, Tetrahedron Letters, 2008, vol. 49, pp. 7070-7073, DOI 10.1016/j.tetlet.2008.09.155<br>chemical compounds 8a, 9a, 10a, 10b (table 1) | 4 |
| X | JP 3-115267 A (MARUHO CO., LTD.) 16 May 1991 (1991-05-16)<br>examples 2, 3, 8-11, 13, 14, 16, 18, 26, 27 | 4 |
| X | US 4628104 A (G. D. SEARLE & CO.) 09 December 1986 (1986-12-09)<br>example 22 | 4 |
| X | DE 3020500 A1 (HOECHST AG.) 21 January 1982 (1982-01-21)<br>examples 1, 20-22 | 4 |
| X | RN 1071628-74-4, File REGISTRY (STN), [online], 07 November 2008, [retrieved on 17 September 2024]<br>entire text | 4 |
| A | JP 2022-65219 A (AGRO-KANESHO CO., LTD.) 27 April 2022 (2022-04-27)<br>claims | 1-15 |
| A | WO 2023/282110 A1 (ISHIHARA SANGYO KAISHA, LTD.) 12 January 2023 (2023-01-12)<br>claims | 1-15 |
| A | JP 8-73436 A (NISSAN CHEMICAL INDUSTRIES LTD.) 19 March 1996 (1996-03-19)<br>claims | 1-15 |
| A | JP 51-105064 A (ROHM AND HAAS COMPANY) 17 September 1976 (1976-09-17)<br>claims | 1-15 |

Form PCT/ISA/210 (second sheet) (July 2022)

**INTERNATIONAL SEARCH REPORT**
**Information on patent family members**

International application No.
**PCT/JP2024/025816**

| Patent document cited in search report | Publication date (day/month/year) | Patent family member(s) | Publication date (day/month/year) |
|---|---|---|---|
| JP 3-115267 A | 16 May 1991 | EP 423524 A2<br>examples 2, 3, 8-11, 13, 14, 16, 18, 26, 27 | |
| US 4628104 A | 09 December 1986 | (Family: none) | |
| DE 3020500 A1 | 21 January 1982 | (Family: none) | |
| JP 2022-65219 A | 27 April 2022 | WO 2020/179910 A1 | |
| WO 2023/282110 A1 | 12 January 2023 | EP 4368023 A1<br>claims<br>CN 117479838 A<br>KR 10-2024-0032841 A | |
| JP 8-73436 A | 19 March 1996 | (Family: none) | |
| JP 51-105064 A | 17 September 1976 | US 4085209 A<br>claims | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI08073436 A
- WO 2020179910 A
- WO 2023282110 A
- WO 0136381 A
- JP 2002155044 A
- WO 9319045 A

### Non-patent literature cited in the description

- *Journal of Organic Chemistry*, 2021, vol. 86, 6943
- *Organic Preparations and Procedures International*, 1989, vol. 21, 91